# EUROPEAN PATENT SPECIFICATION

(11) **EP 4 359 795 B1**
(45) Date of publication and mention of the grant of the patent: **14.01.2026**
(21) Application number: 22735870.2
(22) Date of filing: 21.06.2022
(51) Int. Cl.: G01N 33/569, G01N 33/574, G01N 33/68

(54) **METHODS FOR DIAGNOSING A CANCER- OR ANTIBIOTICS-INDUCED DYSBIOSIS AND THEIR USE FOR IMPROVING CANCER TREATMENT BY IMMUNOTHERAPY**
VERFAHREN ZUR DIAGNOSE EINER KREBS- ODER ANTIBIOTIKA-INDUZIERTEN DYSBIOSE UND IHRE VERWENDUNG ZUR VERBESSERUNG DER KREBSBEHANDLUNG DURCH IMMUNTHERAPIE
PROCÉDÉS DE DIAGNOSTIC D'UNE DYSBIOSE INDUITE PAR UN CANCER OU DES ANTIBIOTIQUES ET LEUR UTILISATION POUR AMÉLIORER LE TRAITEMENT DU CANCER PAR IMMUNOTHÉRAPIE

(30) Priority: 21.06.2021 EP 21305846; 16.12.2021 EP 21306817
(43) Date of publication of application: 01.05.2024
(73) Proprietor: Institut Gustave Roussy, 94800 Villejuif (FR); Everimmune, 94800 Villejuif (FR); Université Paris-Saclay, 91190 Gif-sur-Yvette (FR); Institut National de la Santé et de la Recherche Médicale (INSERM), 75013 Paris (FR)
(72) Inventor: FIDELLE, Marine, 75005 PARIS (FR); ZITVOGEL, Laurence, 75006 PARIS (FR); DAILLERE, Romain, 49100 ANGERS (FR); RAUBER Conrad, 69120 Heidelberg (DE)
(74) Representative: Casalonga
(86) International application number: PCT/EP2022/066929
(87) International publication number: WO 2022/268841

(56) References cited:
- WO-A1-2004/081049
- WO-A1-2017/165778
- WO-A1-2020/106983
- WO-A2-2004/045542
- WO-A2-2005/067620
- EUPHEMIA LEUNG ET AL: "Mucosal vascular addressin cell adhesion molecule-1 is expressed outside the endothelial lineage on fibroblasts and melanoma cells", IMMUNOLOGY AND CELL BIOLOGY, vol. 81, no. 4, 1 August 2003 (2003-08-01), pages 320 - 327, XP055145434, ISSN: 0818-9641, DOI: 10.1046/j.1440-1711.2003.t01-1-01175.x
- PORTER ROSS J ET AL: "Inflammatory Bowel Disease-Associated Colorectal Cancer: Translational Risks from Mechanisms to Medicines", vol. 15, no. 12, 10 June 2021 (2021-06-10), NL, pages 2131 - 2141, XP055922806, ISSN: 1873-9946, Retrieved from the Internet <URL:https://www.ncbi.nlm.nih.gov/pmc/articles/PMC8684457/pdf/jjab102.pdf> DOI: 10.1093/ecco-jcc/jjab102
- YUAN TIAN1 ET AL: "TRIM59 loss in M2 macrophages promotes melanoma migration and invasion by upregulating MMP-9 and Madcam1", AGING, 10 October 2019 (2019-10-10), pages 8623 - 8641, XP055924631, Retrieved from the Internet <URL:https://www.ncbi.nlm.nih.gov/pmc/articles/PMC6814609/pdf/aging-11-102351.pdf> [retrieved on 20220524]
- DOGAN AHMET ET AL: "Expression of Lymphocyte Homing Receptors and Vascular Addressins in Low-Grade Gastric B-Cell Lymphomas of Mucosa-Associated Lymphoid Tissue", AMERICAN JOURNAL OFPATHOLOGY, 1 November 1997 (1997-11-01), pages 1361 - 1369, XP055923310, Retrieved from the Internet <URL:https://www.ncbi.nlm.nih.gov/pmc/articles/PMC1858078/pdf/amjpathol00023-0182.pdf> [retrieved on 20220519]
- LEUNG EUPHEMIA ET AL: "Bioassay detects soluble MAdCAM-1 in body fluids", IMMUNOLOGY AND CELL BIOLOGY, CARLTON, AU, vol. 82, no. 4, 1 August 2004 (2004-08-01), pages 400 - 409, XP002417044, ISSN: 0818-9641, DOI: 10.1111/J.0818-9641.2004.01247.X
- BERTRAND ROUTY ET AL: "The Intestinal Microbiota Modulates the Anticancer Immune Effects of Cyclophosphamide", SCIENCE, vol. 359, no. 6371, 5 January 2018 (2018-01-05), US, pages 91 - 97, XP055554928, ISSN: 0036-8075, DOI: 10.1126/science.aan3706
- TRIVEDI PALAK J. ET AL: "Vascular adhesion protein-1 is elevated in primary sclerosing cholangitis, is predictive of clinical outcome and facilitates recruitment of gut-tropic lymphocytes to liver in a substrate-dependent manner", 20 April 2017 (2017-04-20), US, pages 1 - 11, XP055922843, ISSN: 0165-4608, Retrieved from the Internet <URL:https://gut.bmj.com/content/gutjnl/early/2017/04/20/gutjnl-2016-312354.full.pdf> DOI: 10.1016/S0165-4608(98)00200-3
- SHARPTON S R ET AL: "Current Concepts, Opportunities, and Challenges of Gut Microbiome-Based Personalized Medicine in Nonalcoholic Fatty Liver Disease", CELL METABOLISM, CELL PRESS, UNITED STATES, vol. 33, no. 1, 8 December 2020 (2020-12-08), pages 21 - 32, XP086436257, ISSN: 1550-4131, [retrieved on 20201208], DOI: 10.1016/J.CMET.2020.11.010
- JAIN TEJESHWAR ET AL: "New Insights Into the Cancer-Microbiome-Immune Axis: Decrypting a Decade of Discoveries", FRONTIERS IN IMMUNOLOGY, vol. 12, 23 February 2021 (2021-02-23), XP055922850, DOI: 10.3389/fimmu.2021.622064
- DEROSA LISA ET AL: "Microbiota-Centered Interventions: The Next Breakthrough in Immuno-Oncology?", vol. 11, no. 10, 1 October 2021 (2021-10-01), US, pages 2396 - 2412, XP055925491, ISSN: 2159-8274, Retrieved from the Internet <URL:https://aacrjournals.org/cancerdiscovery/article-pdf/11/10/2396/3082580/2396.pdf> DOI: 10.1158/2159-8290.CD-21-0236

## Description

### FIELD OF THE INVENTION

The present invention relates to the field of anticancer immunotherapy. In particular, the present invention concerns the role of the MAdCAM-1/α4β7 axis the gut microbiota in the efficacy of cancer treatments and provides methods for determining if a patient is likely to benefit from a cancer treatment, more precisely, an imuno-oncology (I-O) therapy, such as a treatment comprising administration of an antibody directed against immune checkpoint blockers PD1, PD-L1 or PD-L2 alone or together with CTLA4. The present invention also provides methods to improve the efficacy of such a treatment in patients in need thereof.

### BACKGROUND OF THE INVENTION

During the last decade, the gut microbiome has garnered much attention in the context of evolving immuno-oncology with compelling evidence of the role of intestinal dysbiosis in primary resistance to immune checkpoint blockade across a large range of advanced malignancies. This contention was supported by numerous epidemiological studies pointing out the deleterious impact of broad-spectrum antibiotics in the clinical outcome of anti-PD-1/anti-PD L-1 antibodies (Abs) in stage III and IV lung, kidney, bladder cancers and melanoma. Of note, decreased richness of the intestinal ecosystem was associated with a tumor microenvironment poor in T cell infiltrates.

The increased awareness of the potential contribution of gut dysbiosis in treatment failure led many investigators to describe metagenomics-based intestinal blueprints associated with resistance to immunotherapy. However, to which extent cancer-associated dysbiosis precedes the tumorigenic process and therefore is causatively linked to neoplasia development or is merely its direct consequence remains unknown.

β7 integrin-containing heterodimers play a central role in leukocyte homing to the gut and retention at epithelial surfaces. The β7 integrin subunit can form a heterodimer with the α4 (CD49d) integrin subunit resulting in the α4β7 integrin (also called "Lymphocyte Peyer's patch Adhesion Molecule-1" (LPAM-1)). Through interaction with its counter-receptor Mucosal Addressin Cellular Adhesion Molecule-1 (MAdCAM-1), α4β7 integrin mediates lymphocyte adhesion and diapedesis from the circulation across the vascular endothelial barrier into gut-associated secondary lymphoid tissue (GALT) or the intestinal lamina propria (LP). The transmembrane adhesion molecule MAdCAM-1 is constitutively expressed on LP venules and GALT high endothelial venules (HEV) and inducible by proinflammatory cytokines (Briskin et al., 1997; Dogan et al., 1997; Gorfu et al., 2009; Ogawa et al., 2005). Outside the endothelial cell lineage, MAdCAM-1 was shown to be expressed in fibroblasts (Leung et al., 2003) and in melanoma cells (Tian et al., 2019). Retinoic acid (RA) expressing CD103+ dendritic cells govern the expression of α4β7 integrin and CCR9 receptors in the GALT to enable binding to MAdCAM-1 and homing of lymphocytes to the gut mucosa through the intestinal CCR9 ligand, CCL25. By preventing the influx and extravasation of inflammatory β7+ T cells from the circulation to the gut, antibodies targeting α4β7 or MAdCAM-1 significantly reduced the severity of colitis in animal models and in patients suffering from inflammatory bowel disease (Hassan-Zahraee et al., 2018; Reinisch et al., 2021) . Soluble MAdCAM-1 was proposed as a surrogate marker for ongoing gastrointestinal inflammation, in particular in the context of Inflammatory Bowel Disease (WO 2004/081049 A1; Leung et al., 2004; WO 2005/067620 A2). Antibodies targeting MAdCAM have been investigated for IBD treatment but are no longer envisioned as a possible treatment for IBD because of safety issues (Porter et al., 2021). There is a growing awareness of the role played by intestinal microbiota in maintaining the regulatory functions of intestinal Treg and TH17 cells to ensure gut epithelial barrier integrity (Littman and Rudensky, 2010; Pandiyan et al., 2019).

Beyond their seminal homeostatic role in the intestines, TH17 also control extraintestinal inflammatory lesions (Krebs et al., 2016; Lee et al., 2011a; Magnuson et al., 2015; Morton et al., 2014; Wu et al., 2010). Importantly, TH17 and their lineage-related FoxP3⁺ regulatory cells blunt antitumor immunosurveillance by restoring tolerance during inflammation induced in the course of chronic carcinogenesis (Cochaud et al., 2013; Fridman et al., 2012; Guéry and Hugues, 2015; Langowski et al., 2006; Young, 2016). RORyt expression identified a phenotypically stable population of tumor-infiltrating Tregs in humans and mice that are induced by gut commensals (Sefik et al., 2015). Conditional RORyt knockout mice showed reduced tumor incidence or polyp formation, through an indirect mechanism involving dendritic cells (Blatner et al., 2012; Rizzo et al., 2018).

Immune checkpoint inhibitors (ICI) reinstate tumor immunosurveillance and have become the standard of care for the clinical management of several histological types of cancers (Brahmer et al., 2015; Robert et al., 2011). Primary resistance to ICI has been mainly attributed to low tumor mutational burden and poor intrinsic antigenicity of tumor cells (Riaz et al., 2016; Rizvi et al., 2015), defective antigen presentation (Spranger et al., 2015), intratumoral T cell exhaustion (Smyth et al., 2016), genomic defects in the Interferon-γ (IFNγ) signaling pathway (Gao et al., 2016), CSF1-dependent tumor associated macrophages (Neubert et al., 2018), and immunosuppressive metabolic cues (Smyth et al., 2016). In addition, several studies indicated that a deviated gut microbiome repertoire negatively impact ICI efficacy, suggesting the crucial importance of commensals in affecting the cancer-immune set point (Chen and Mellman, 2017). Retrospective and prospective studies analyzing the impact of antibiotics (ATB) on patients survival revealed their negative predictive impact on clinical outcome during immune checkpoint blockade (Elkrief et al., 2019b; Mohiuddin et al., 2021; Routy et al., 2017; Tinsley et al., 2019; Derosa et al. 2022). Meta-analyses revealed that ATB uptake is more harmful on clinical outcome when administered prior to the first systemic administration of (rather than during) anti-PD1/PDL-1 Abs, suggesting that recolonization post-ATB course rather than direct iatrogenic effects may be deleterious (Derosa et al., 2021). Indeed, ATB-treating patients tended to be colonized by distinct species (such as *Hungatella hathewayi* (Derosa et al. 2022). These epidemiological findings led to the hypothesis that ATB, by affecting the composition of the intestinal ecosystem, could tilt the equilibrium between intratumoral effector and regulatory T cells by affecting the gut/tumor trafficking of intestinal homeostatic regulatory T cell subsets (*38*), thereby aggravating cancer immunosuppression.

### SUMMARY OF THE INVENTION

Recently, the inventors conducted meta-analyses which revealed that ATB uptake is more harmful on clinical outcome when administered prior to (rather than during) the first systemic administration of anti-PD1/PDL-1 Abs, suggesting that recolonization post-ATB course rather than direct iatrogenic effects may be deleterious (Derosa et al., Cancer Discovery, 2021).

In the experimental part which follows, the inventors confirm this hypothesis and show the pathways through which the gut microbiota distantly affects the evolution of several types of cancers and the patient's response to immunotherapy.

Indeed, the inventors showed that ATB-induced dysbiosis (Routy et al., 2018; Vétizou et al., 2015) results in the disruption of the interaction between Mucosal Addressin Cell Adhesion Molecule-1 (*MAdCAM-1*) and its receptor α4β7 expressed by enterotropic T cells.

When the course of ATB is stopped, selected species from the *Enterocloster* gen.nov. of bacteria (such as *Enterocloster clostridioformis* comb.nov (Haas and Blanchard, 2020), previously classified in the Clostridia class of bacteria) take over and downregulate ileal Mucosal Addressin Cell Adhesion Molecule-1 (*madcam1*) gene and cell surface protein expression on ileal lamina propria and high endothelial venules. Loss of MAdCAM-1 provokes the exodus of the gut homing α4β7⁺ TH17 and RORγt⁺Treg CD4⁺T cells to the tumor beds, tolerizing the cancer microenvironment and culminating in resistance to PD-1 blockade.

The present invention is based on these results and first pertains to a method for *in vitro* assessing whether an individual having a cancer also has a cancer-associated dysbiosis or antibiotics-associated dysbiosis, based on down regulation of MAdCAM in serum or ileum and/or Gut OncoMicrobiome Signature (GOMS). This method can also be used to follow-up the effects of a microbiota-centered intervention (MCI) in a patient.

Diagnosing such a cancer- and/or ATB-related dysbiosis indicates that the subject needs a compensatory microbiota-centered intervention (MCI), especially before receiving a treatment with an immune checkpoint inhibitor (ICI) or with another immuno-oncology (I-O) therapy.

The use of such MCI, including oral vancomycin antibiotics, phages and rare-cutting endonucleases able to kill bacteria of the *Enterocloster* gen. nov. clade, *Akkermansia* spp and/or *Akkermansia muciniphila,* possibly mixed with other beneficial bacteria, retinoic acid, fecal microbial transplantation, and mixtures thereof, for restoring sensitivity to an I-O therapy, is also part of the present invention.

The invention also pertains to combined therapies for cancer patients having a low level of serum soluble MAdCAM, with (i) an anti-PD1 or an anti-PDL1 blocker antibody and (ii) an anti-IL-17A or an anti-IL-17R blocker antibody and/or a recombinant IL-7.

The present disclosure also relates to screening methods for identifying agents capable of normalizing MAdCAM-1 expression levels in endothelial cells of the lamina propria.

### BRIEF DESCRIPTION OF THE DRAWINGS

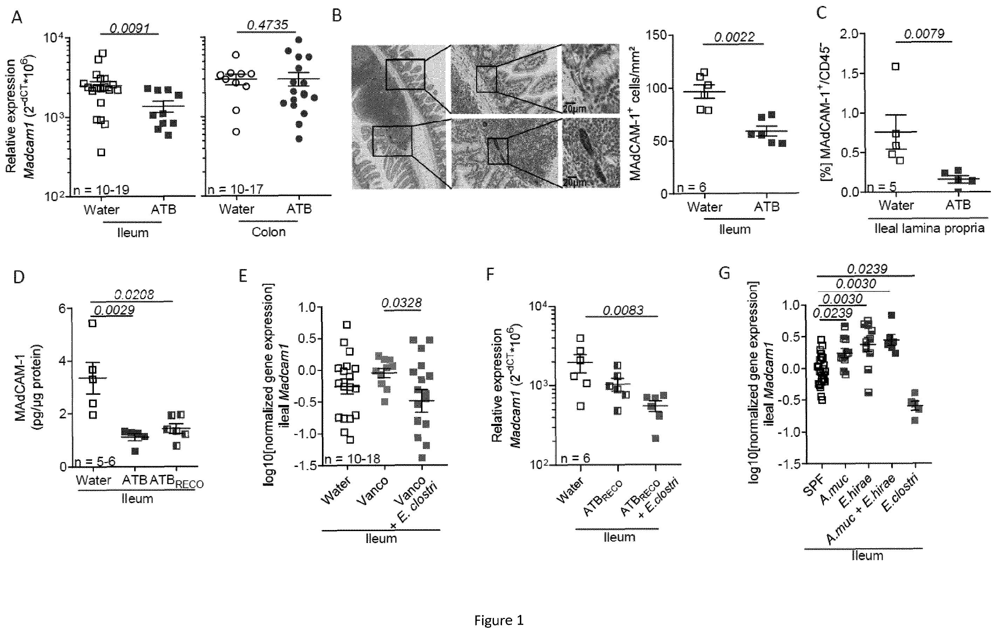

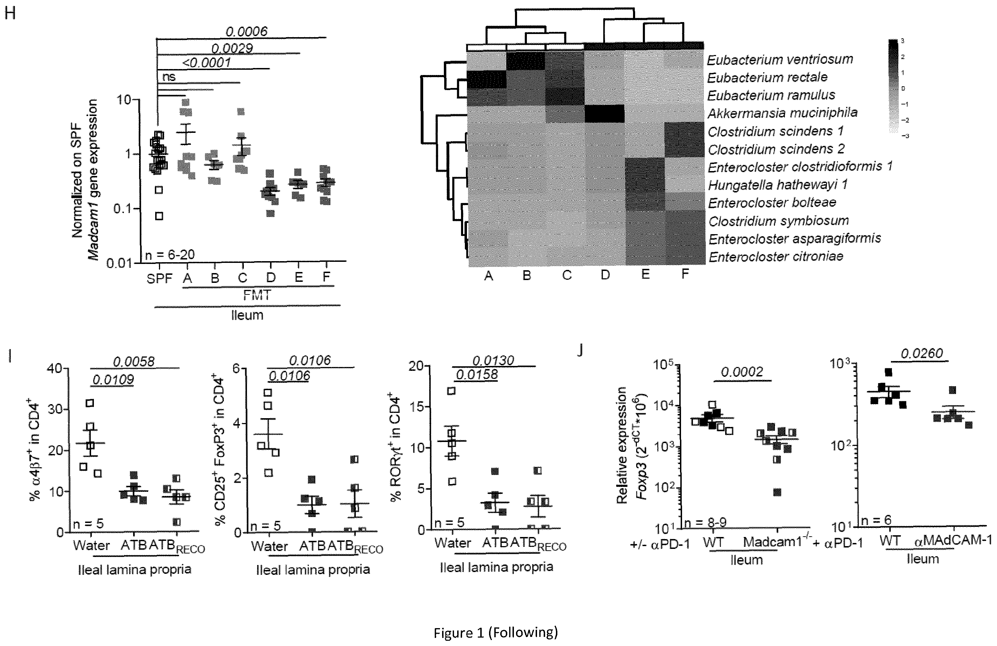

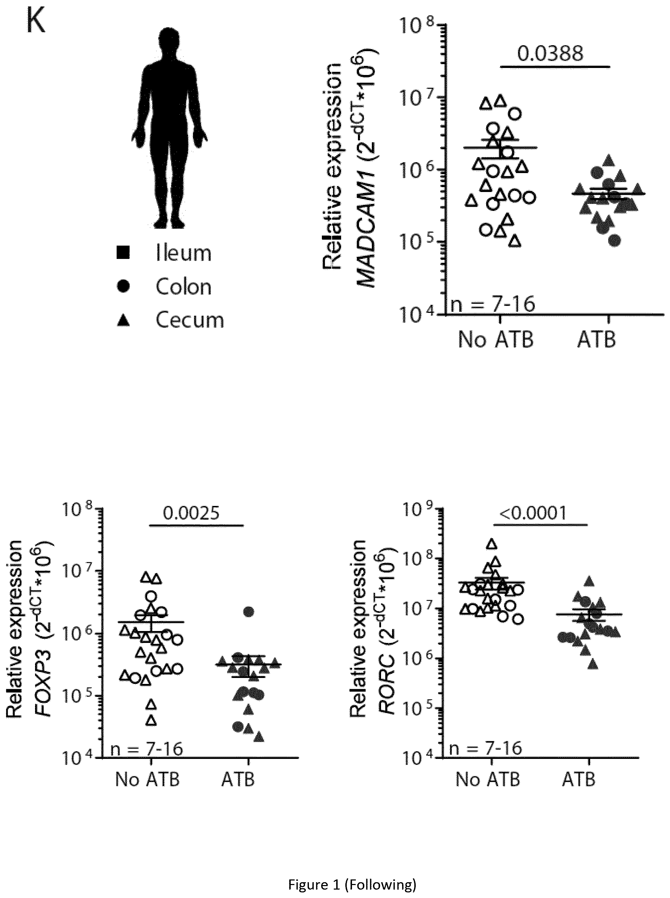

**Figure 1****. Broad-spectrum antibiotics (ATB) downregulate MADCAM-1 expression in the ileal vasculature.**

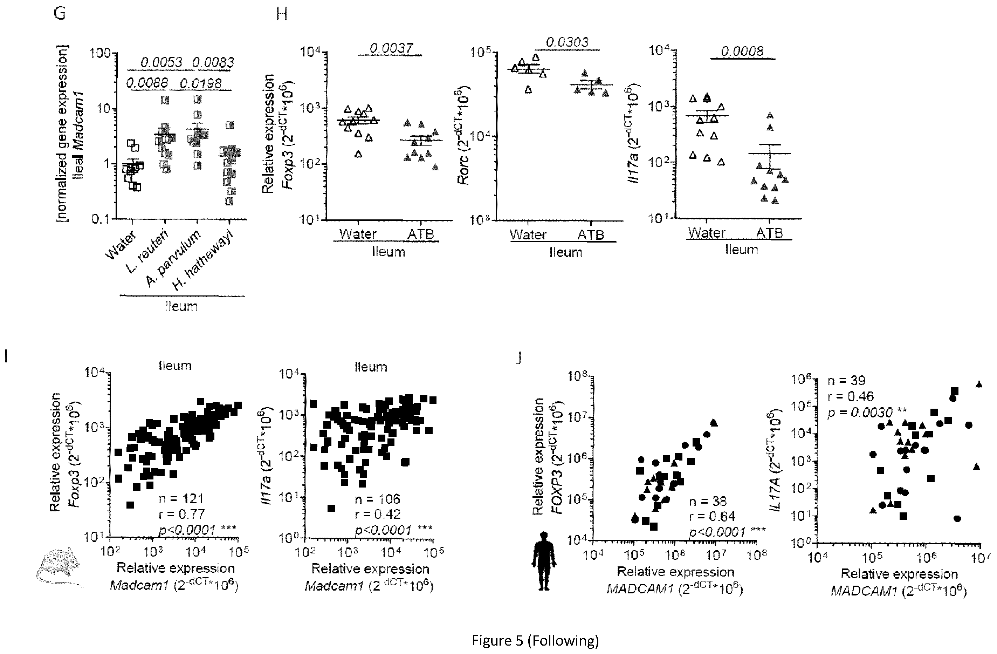

**A-D.** Relative transcription (**A**) and protein (**B-D**) levels of *Madcam1* gene product obtained with RT-PCR (**A**), immunohistochemistry staining of MAdCAM-1 in LP venules (upper micrograph pictures) and high endothelial venules (HEV, lower micrograph pictures) (**B**), flow cytometry gating on CD45⁻ cells of LP (**C**) or ELISA (**D**) of ileal (A-D) or colonic (A) tissues in C57BL/6J mice after continuous broad-spectrum antibiotics *(ATB: Ampicillin, Colistin, Streptomycin)* or after a 4 to 7 days spontaneous recolonization (ATB_{RECO}) following ATB stop. Each dot represents one ileum or colon. **E-F.** Quantitative RT-PCR of relative *madcam1* gene expression in ileal mucosae of mice treated with 8 days of oral vancomycine (E) or broad spectrum ATB followed by 4-7 days recolonization (ATB_{RECO}) (F), and then supplemented with gavage with *Enterocloster clostridioformis* once prior to sacrifice 7 days later. Each graph depicts a representative experiment (comprising 5-6 mice/group) out of 2-3 yielding similar results. Panel A or E left panel pooled the data of 2 experiments. **G.** Idem as in E. but oral gavage was performed in C57BL/6J mice reared in SPF conditions (no ATB conditioning) using bacteria spp. aligned in the x axis. **H.** Left panel: Impact of fecal microbiota transplantation (FMT) in 3 days- ATB treated recipient mice using feces from NSCLC patients at diagnosis (prior to PD-1 blockade) on ileal *Madcam1* gene expression normalized on the naïve specific-pathogen free (SPF) mice group *(log10 axis).* Each experiment used a different FMT donor and comprised 5-6 animals/group. Each dot represent on ileum. Right panel: non supervised hierarchical clustering of the taxonomic composition of donor feces (defined using shot gun MG sequencing), selecting bacteria of high prevalence >25% and clinically relevant . **I**. Same experiments as in A-D performing flow cytometric analysis of ileal lamina propria CD4⁺ T cell subsets (α4β7⁺ vs CD25⁺ FoxP3⁺ Treg vs RORγt⁺ CD4+ (T_{H}17) cells) during continuous ATB or at the ATB_{RECO} phase, each dot representing one ileum. A representative Facs analysis is depicted out of 2 yielding similar results. Also refer to Figure 5H-I for qPCR-related data on similar ileal mucosae tissues. **J.** Idem as in A. but in *Madcam-1* gene deficient mice (treated or not with anti-PD1 Abs that did not impact on ileal MADCAM-1 expression levels, not shown) without ATB conditioning. Each dot represents the qPCR data of one ileum. **K.** RT-PCR -based transcriptional levels of human *MADCAM-1, FOXP3,* RORC genes in intestinal biopsies collected during endoscopic intervention in 9 control (ATB free) patients and 7 ATB-treated patients (**Table 1**). Each dot represents one biopsy either from ileum, colon and caecum, a single patient being represented 1 to 3 times. ANOVA statistical analyses (Kruskal-wallis test): *p<0.05, **p<0.01, ***p<0.001.

**Table 1. Description of patients donating the FMT.**

| | | No | % |
|---|---|---|---|
| Age-yr | <65 | 3 | 50 |
| | ≥65 | 3 | 50 |
| Gender | Male | 6 | 100 |
| | Female | 0 | 0 |
| Smoking status | Smoker | 4 | 67 |
| | Former | 2 | 33 |
| | Never | 0 | 0 |
| ECOG performance status | 0-1 | 4 | 67 |
| | ≥2 | 2 | 33 |
| Antibiotics | No | 6 | 100 |
| | Yes | 0 | 0 |
| Overall-survival | <12 | 3 | 50 |
| | ≥12 | 3 | 50 |

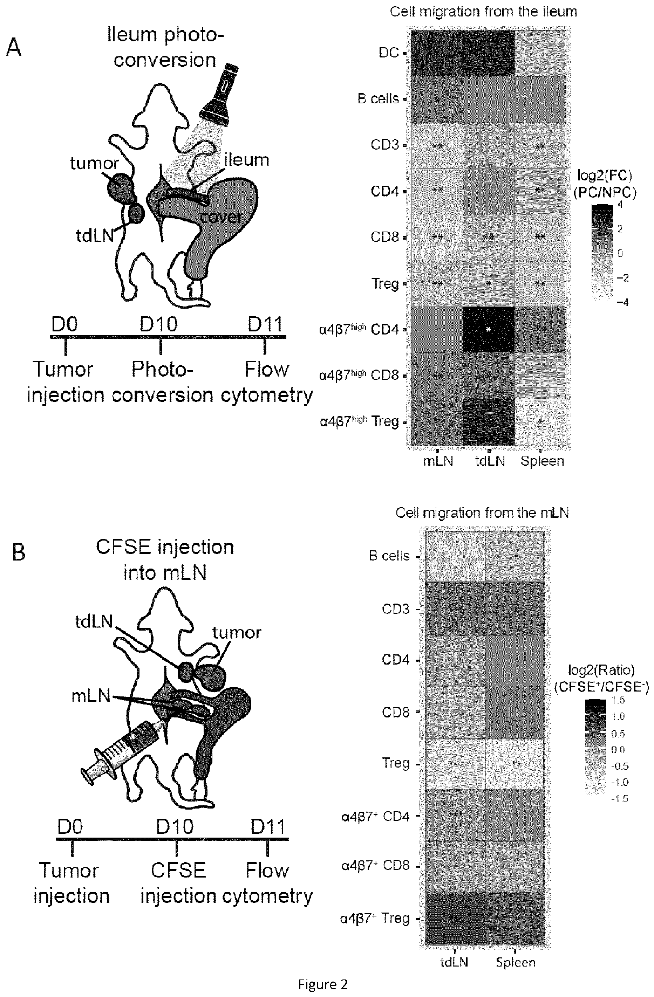

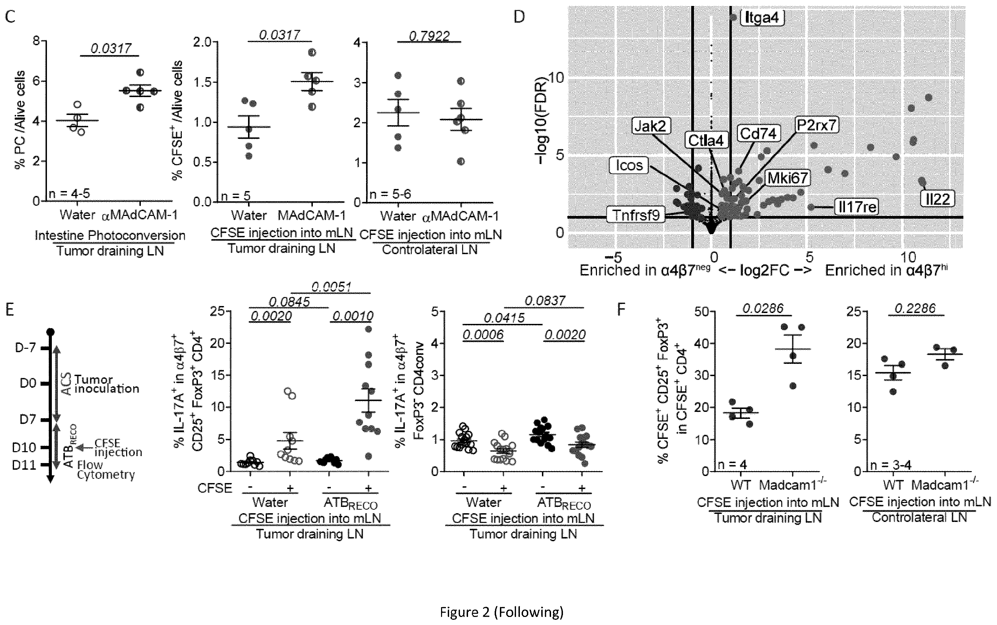

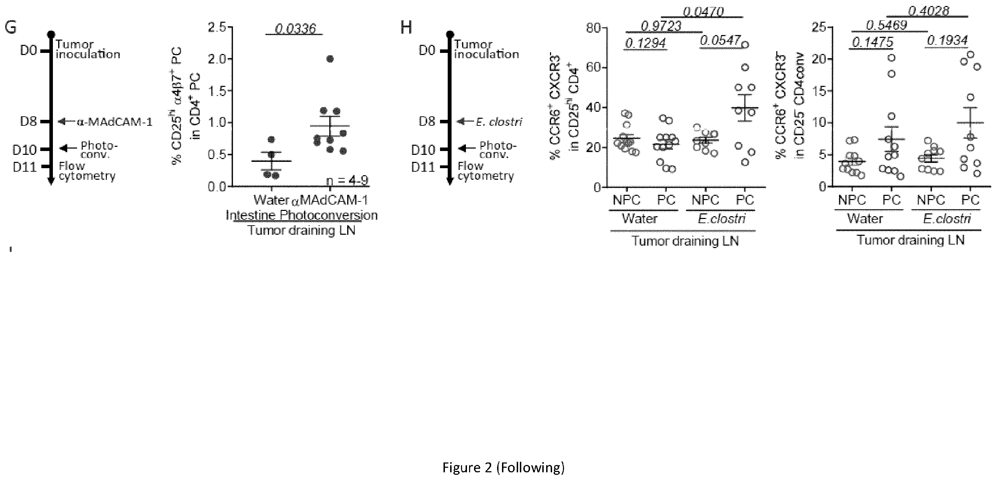

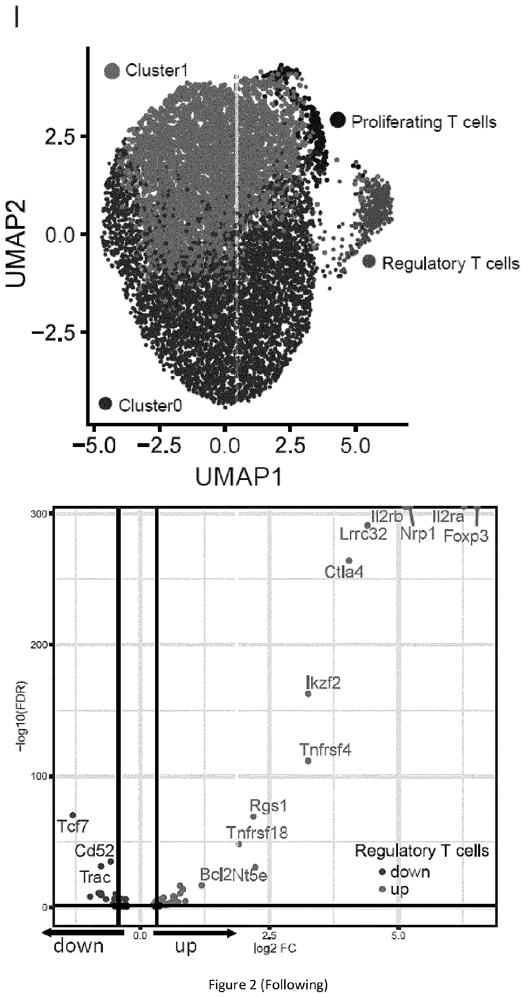

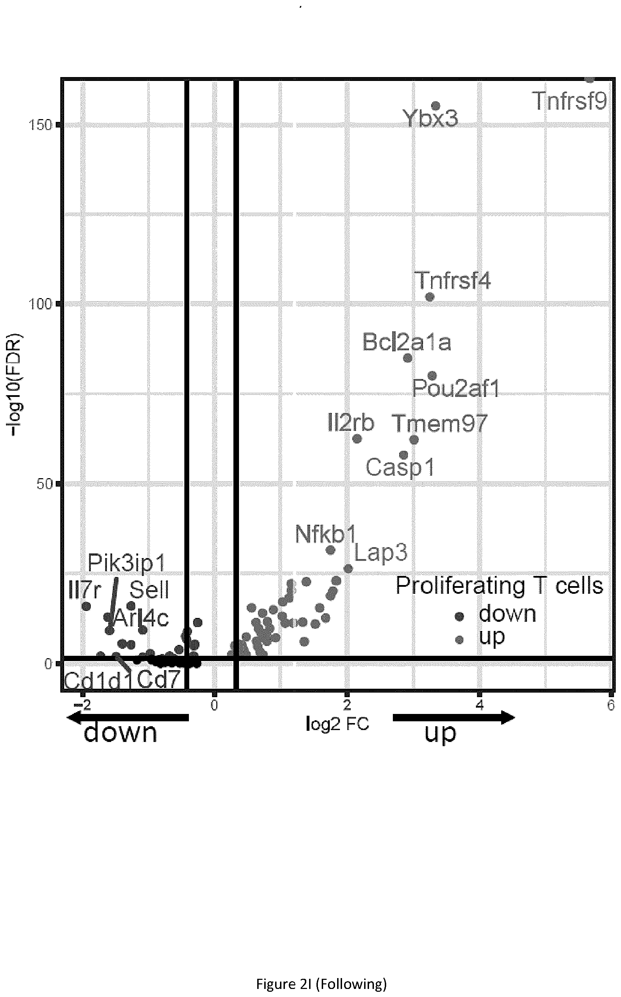

**Figure 2****. Emigration of enterotropic IL-17A and IL-22 secreting- α4β7⁺ CD4⁺ T and Treg cells towards tumor beds induced by bacterial recolonization post-ATB.**

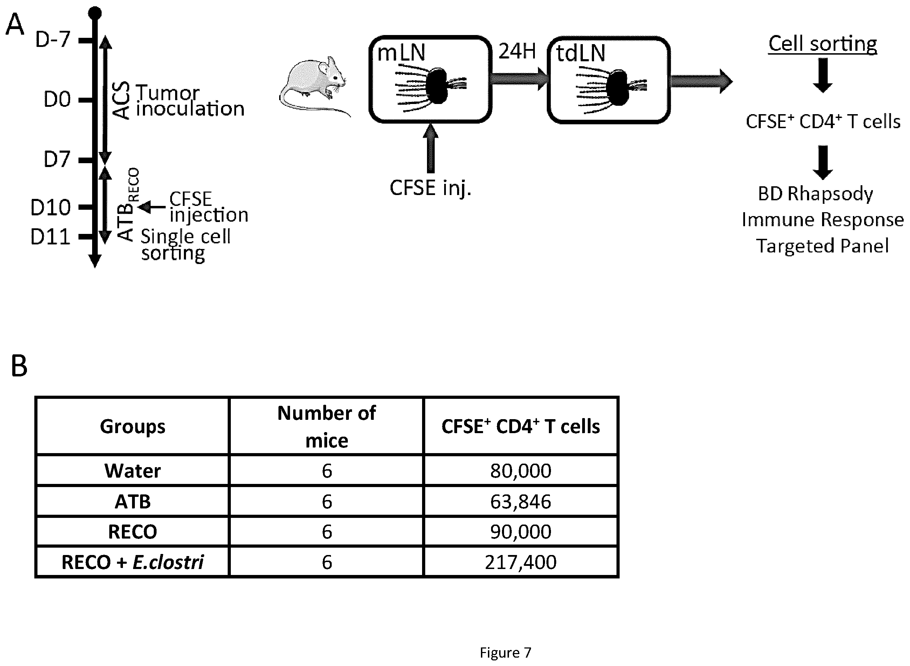

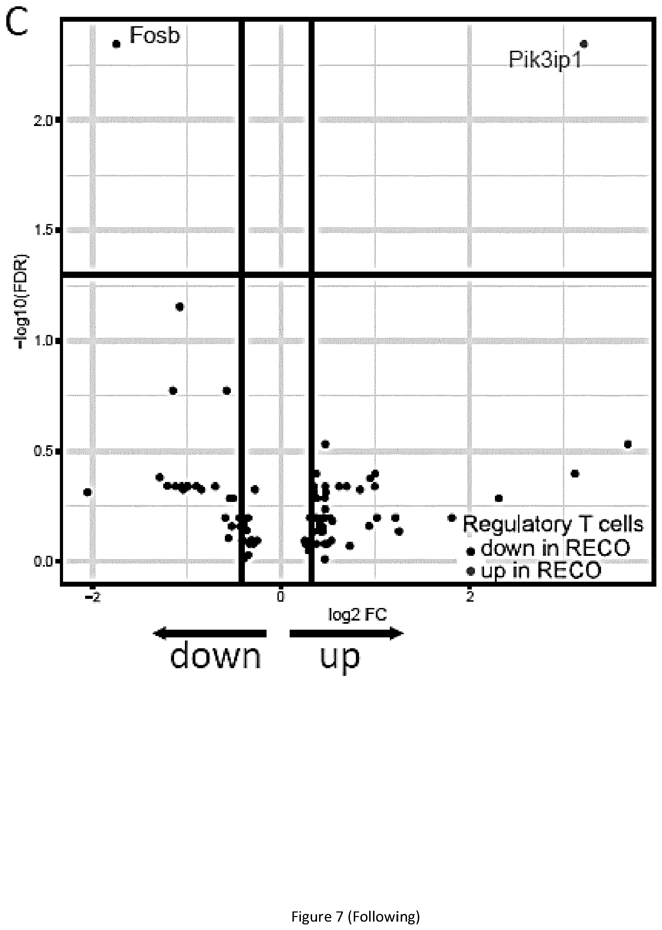

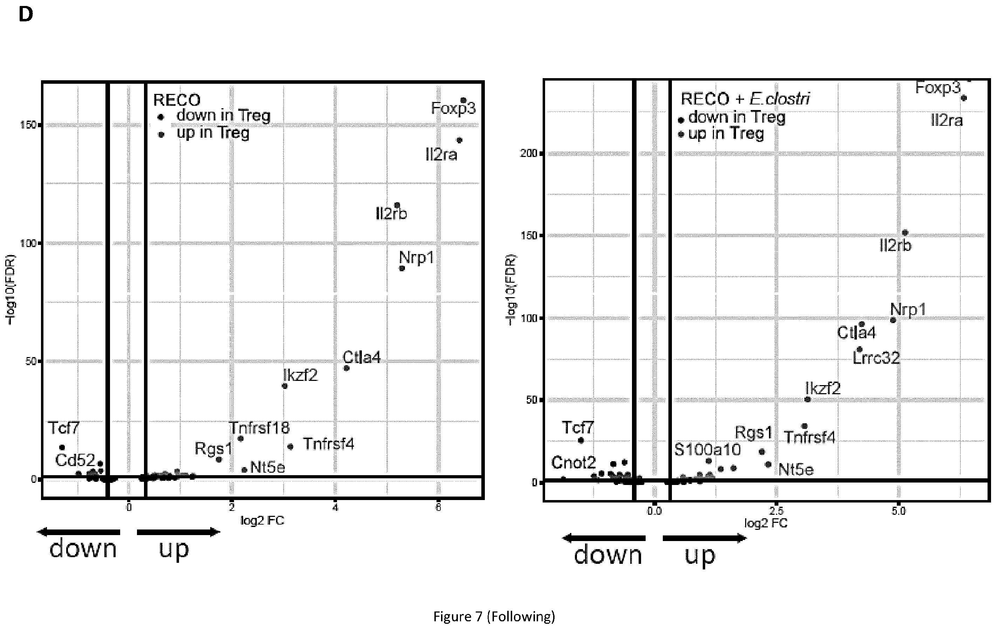

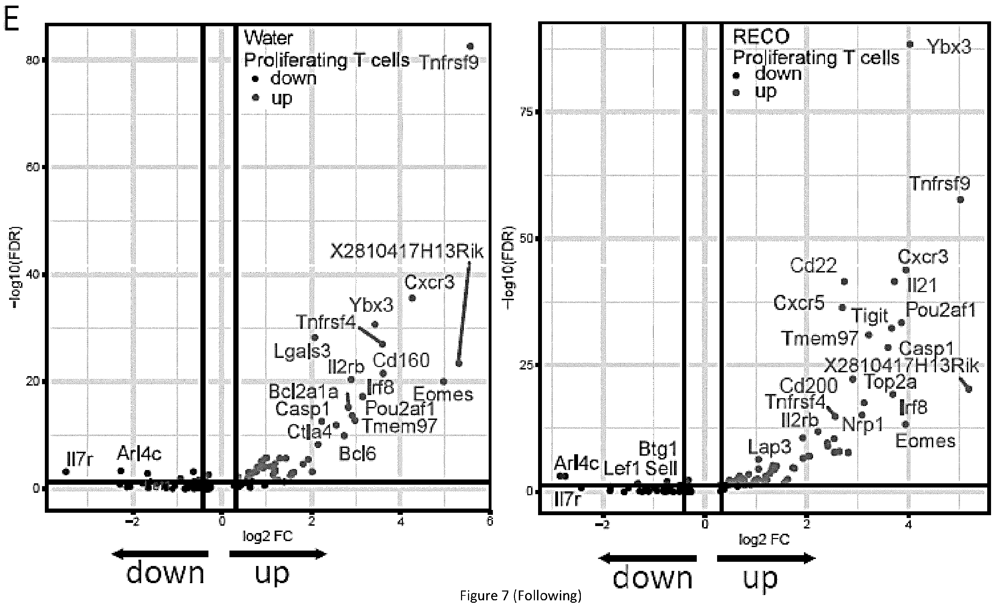

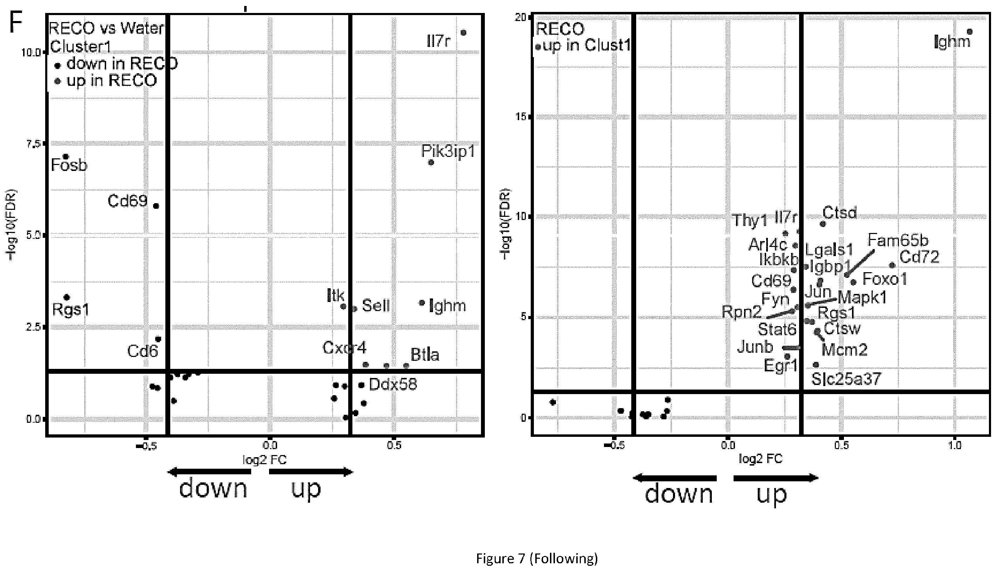

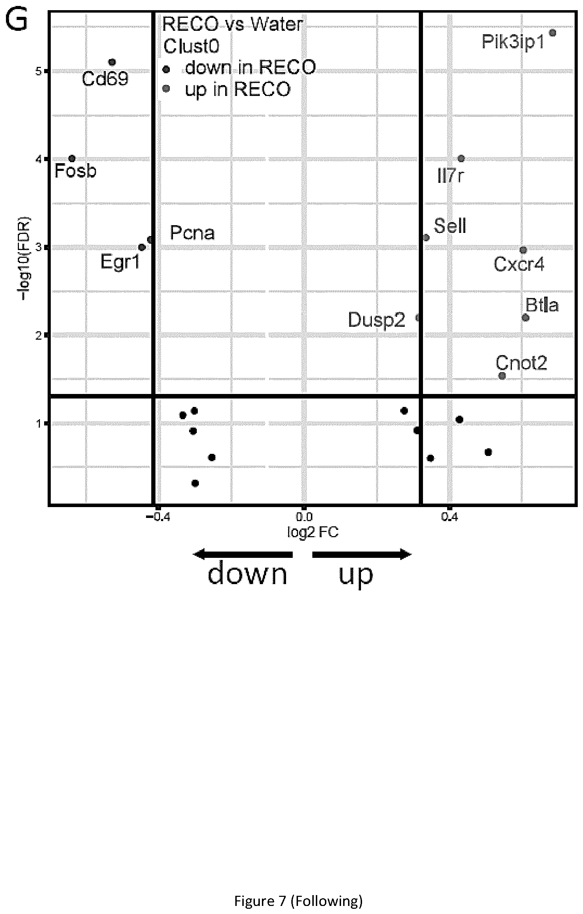

**A-B.** Flow cytometric determination of photoconverted (A) or CFSE labeled (B) gut-originating cells in secondary lymphoid organs (mesenteric LN, spleen, tdLN: tumor draining LN) assessed in Kaede mice after illumination according to the schema (**A, left panel**) or by CFSE injection into the mLN in wild-type mice (**B, left panel**). Left panels depict the graphical schema of the experimental setting and right panels show the relative accumulation (log2 Fold Change (FC) of photoconverted (PC) versus resident (non photoconverted (NPC) cells in each subset detailed in lines using a color gradient (A) or CFSE lalbeled vs resident (non labeled) cells (B) 24 hours after tissue UV-A illumination or CFSE injection in the mLN, in various secondary lymphoid organs. Statistics: test of Mann-Whitney with *p-value* adjusted by Benjamini-Hochberg method, *p<0.05, **p<0.01, ***p<0.001. C. Effect of the neutralization of MAdCAM-1 using αMAdCAM-1 mAb on the recirculation of gut-derived cells towards the tdLN in Kaede (**left**) or in mLN- CFSE injected WT mice (**middle panel**) or towards a controlatreal LN (**right panel**). Each dot represents one mouse. A representative experiment out of two is depicted, reaching similar conclusions. **D.** Volcano plot depicting the differential gene transcription in RNA sequencing of α4β7^{high} versus α4β7^{neg} CD4⁺ lymphocytes cell-sorted from mLN in 5 MCA205 tumor-bearing animals. Volcano plots were generated computing for each gene product expressed in each cell type for 5 mice i) the log2 of fold ratio (FR) among the mean relative abundances of transcripts after normalization in α4β7^{high} versus α4β7^{neg} CD4⁺ lymphocytes (x axis); ii) the co-log10 of *p-values* deriving from Mann-Whitney U test calculated on relative abundances in absolute values (y axis). Black and grey dots are considered significant (*p<0.05*) while back dots are not (*p>0.05*)*.* **E**. Experimental setting (left panel) and flow cytometric evaluation of the IL-17A⁺ secreting α4β7⁺ Treg (middle panel) or α4β7⁺ Tconv (**right panel**) CD4⁺ T cells within CFSE⁺ (originating from the mLN, grey dots) or CFSE⁻ cells (tdLN resident cells, black dots) reaching the tdLN during the ATB _{RECO} phase following ATB conditioning *(Ampicillin, Colistin, Streptomycin).* Each graph depicts two independent and pooled experiments (comprising 5-6 mice/group). **F.** Flow cytometric determination of CFSE⁺ Treg cell reaching the tdLN or the contralateral LN in MCA205 bearing WT versus *Madcam1*^{*-*/*-*} mice (not treated with ATB) after CFSE injection in mLN. Each dot represents one mouse, a representative experiment out of two yielding similar results being depicted. **G.** Id. As in E. but in Kaede mice treated or not with anti-MADCAM-1 Abs where flow cytometry could identify Treg using membrane stainings for CD25 and CD127. **H.** Experimental setting to assess the short term (4-7 days) effects of bacterial recolonization after stopping the ATB course onto recirculation of enterotropic CD4+ T cells, with or without enforced oral gavage with *Enterocloster spp.* (left panel). Flow cytometric phenotyping of TH17 cells expressing high levels of CD25 (Tr17-like, middle panel) or TH17 conv.(right panel) in Kaede mice within PC (originating from the mLN) or NPC cells (tdLN resident cells) reaching the tdLN during the ATB _{RECO} phase (**G**). Each dot represents one mouse and graphs depicts two pooled experiments. ANOVA statistical analyses (Kruskal-wallis test) or Wilcoxon matched-pairs signed rank test: raw p values are indicated. **I**. Single cell transcriptomics analyses of CFSE+ mLN -derived CD4+ T cells emigrating to tdLN in MCA205 tumor bearers treated with water (group 1), with ATB 7 days, ATB being continued (group 2), or ATB being stopped since 4 days (ATB_{RECO}) (group 3) or replaced by oral gavage with *E. clostridioformis* for 4 days (group 4), the experiment containing 5 animals/group. UMAP gene patterns in 4 subsets of emigrating CFSE+ CD4+ T cells in plate-based full-length single-cell RNA-seq data by unsupervised clustering, overlaying the four groups of mice (I, left panel). Volcano plots of differential gene expression patterns associated with each specific cell type according to Log2 p values and fold ratios for the proportion of each subtype compared with the 3 others (Treg (I, middle panel) and proliferating cells (I, right panel). Refer to Figure 7 for cell type gene patterns according to each of the 4 experimental groups.

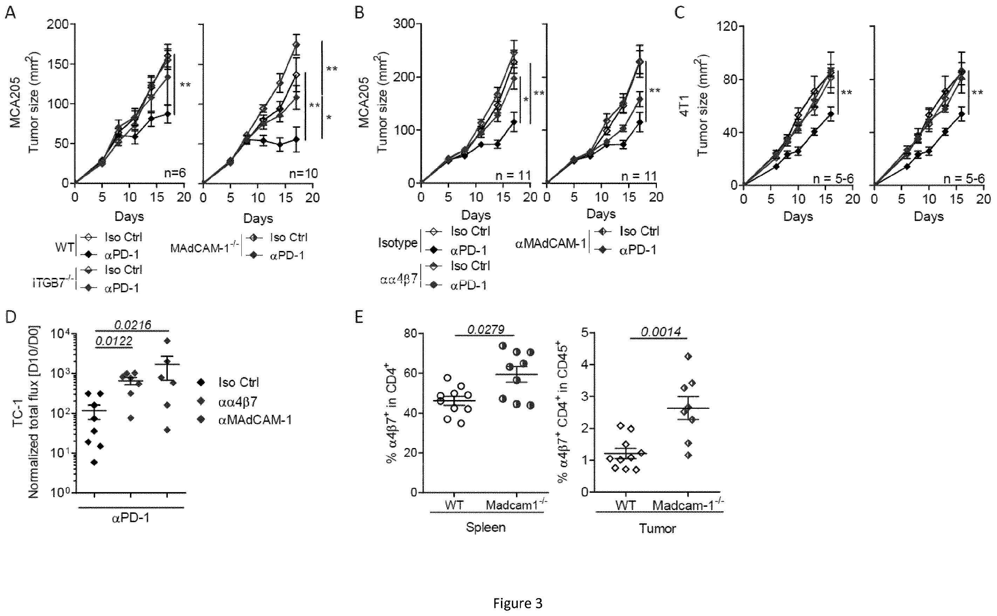

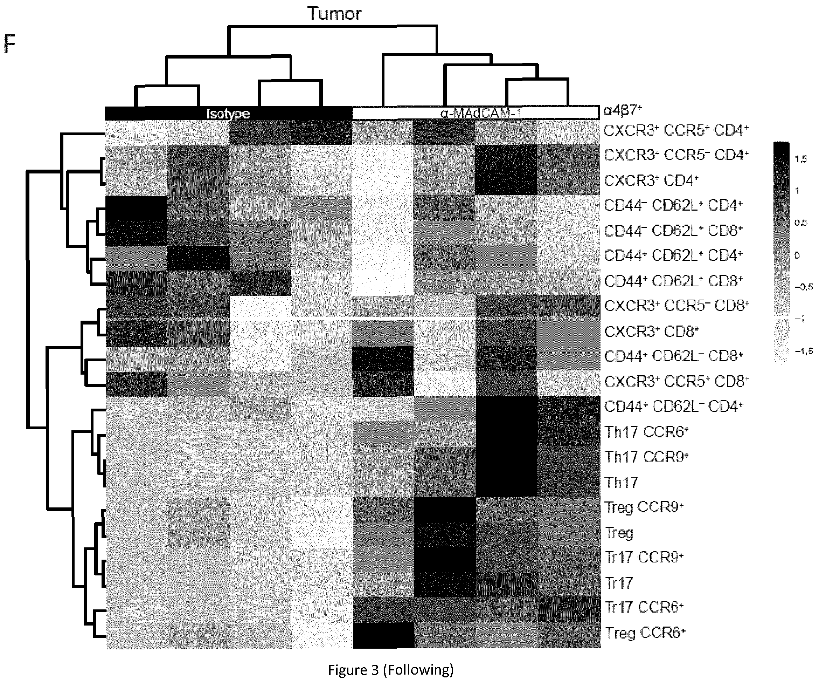

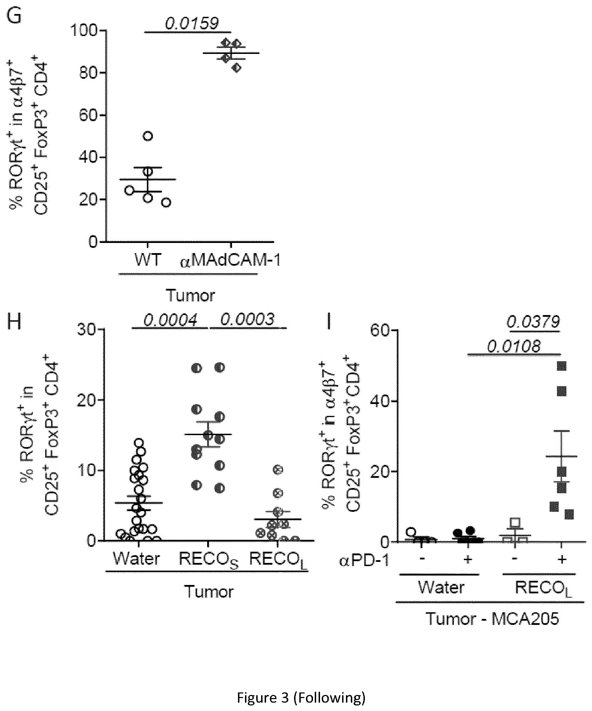

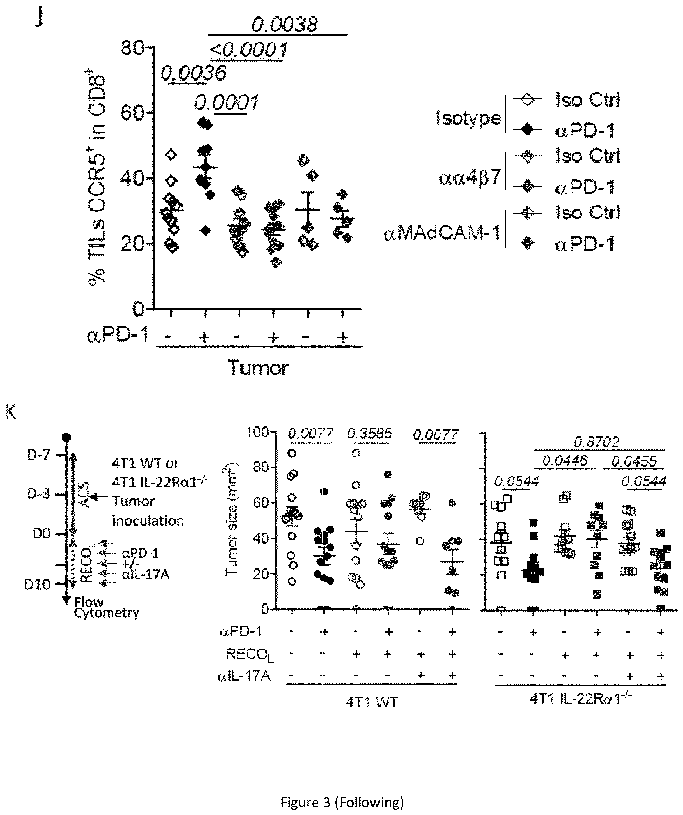

**Figure 3****. Disruption of the MAdCAM-1/α4β7 axis induced maladaptive responses to αPD-1-based immunotherapy in mice.**

**A.**Tumor growth kinetics of subcutaneous MCA205 (syngeneic of C57BL/6J) implanted in wild type (wt) versus *Itgb7* or *Madcam1* gene deficient mice. Means±SEM of tumor sizes among 5-6 mice/group overtime in 2 treatment groups (anti-PD1 versus isoCtl Ab). **B-D**. Tumor growth kinetics or tumor luminescence after implantation of subcutaneous MCA205 (B), mammary 4T1 (syngeneic of BALB/c, **C**) and lung-orthotopic TC1-luc (syngeneic of C57BL/6J, **D**) tumors in animals treated with isotype control, anti-α4β7 mAb or -αMAdCAM-1 mAb while receiving anti-PD1 therapeutic antibodies (or iso-Ctl Abs). For lung-orthotopic TC1 tumor, a whole-body luminescence using IVIS, Imaging System was used to quantify luciferase-expressing TC1 kinetics in lungs. Ratios between pre- and post-PD-1 blockade with isotype control mAb, αα4β7 mAb or αMAdCAM-1 mAb were calculated. **E**. Flow cytometric analysis of α4β7 expression on CD4+ T cell splenocytes and tumor infiltrating lymphocytes (TILs) in wild type (wt) versus *Madcam1* gene deficient mice. Each dot represents one mouse. The graph pooled 2 independent experiments of 5 mice/group. Student's t test. **F.** Non supervised hierarchical clustering represented in a heatmap showing the relative percentages of various immune α4β7⁺ CD4⁺ T cell subsets (determined by flow cytometric analyses of TILs dissociated from established s.c. MCA205) in mice treated with neutralizing αMAdCAM-1 mAb or isotype control mAb. **G-I.** Intracellular flow cytometric analysis of Roryt expression in α4β7⁺Treg TIL in wild type (wt) versus *Madcam1* gene deficient mice (G) or in wt MCA205 tumor bearing mice receiving ATB conditioning regimen ((4 days post-ATB stop (ATB_{RECO-S)} or 12 days post ATB stop (ATB_{RECO-L})) treated or not with anti-PD1 Abs (H,I). Each dot represents one mouse. A representative experiment out of 2 independent ones yielding similar results and comprising 5 mice/group is depicted (G, I). Student's t test (G). **J.** Flow cytometric analyses of tumor infiltrating cells (TILs) for CCR5⁺ CD8⁺ effector T cells in s.c. MCA205 tumor-bearing mice treated with isotype control mAb, αα4β7 mAb or αMAdCAM-1 mAb in the setting of PD-1 blockade. **K.** Schema of the experimental setting (left panel) and cross sectional study of tumor sizes (means±SEM) of s.c. 4T1 WT (**left**) or 4T1 IL-22Rα1 KO (**right**) tumor cell lines at sacrifice after a therapy with αPD-1 mAb during the ATB_{RECO} phase and systemic neutralization of IL-17A using αIL-17A mAb (ip) in parallel to the immunotherapy. In each experiment, four αPD-1 intraperitoneal (i.p.) injections were administered every three days during the ATB_{RECO} phase. Each experiment comprised 6 mice/group. A representative experiment out of 2-3 is depicted. ANOVA statistical analyses (Kruskal-wallis test): *raw p values are indicated.

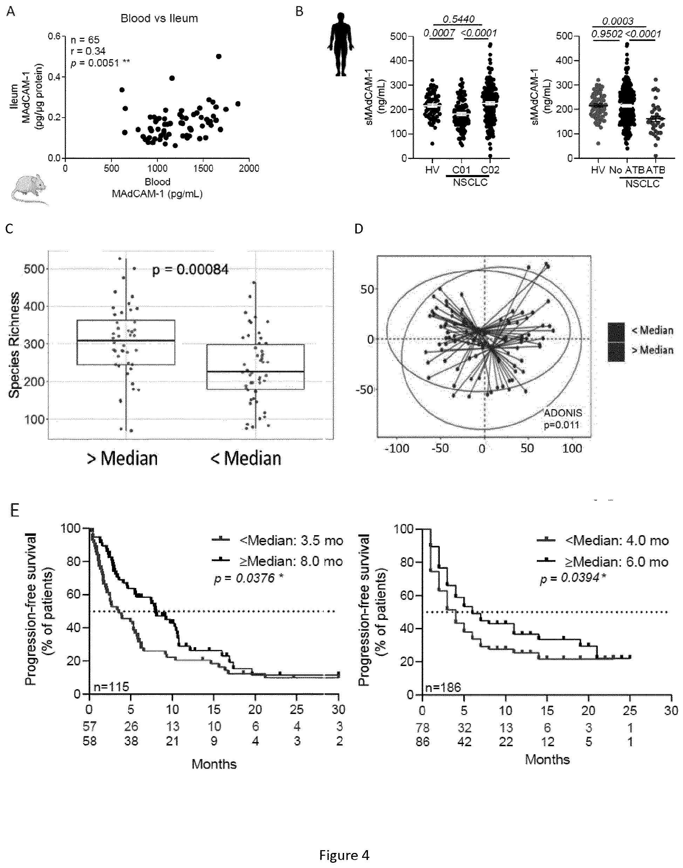

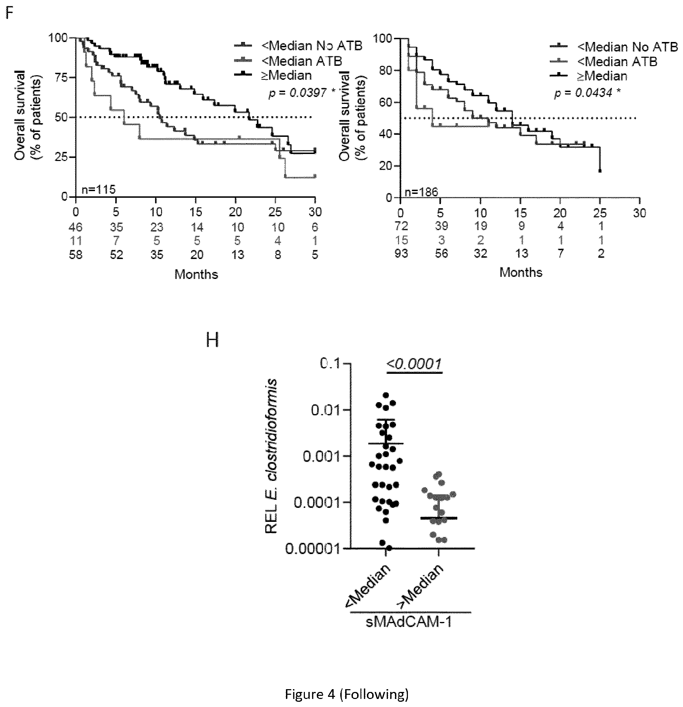

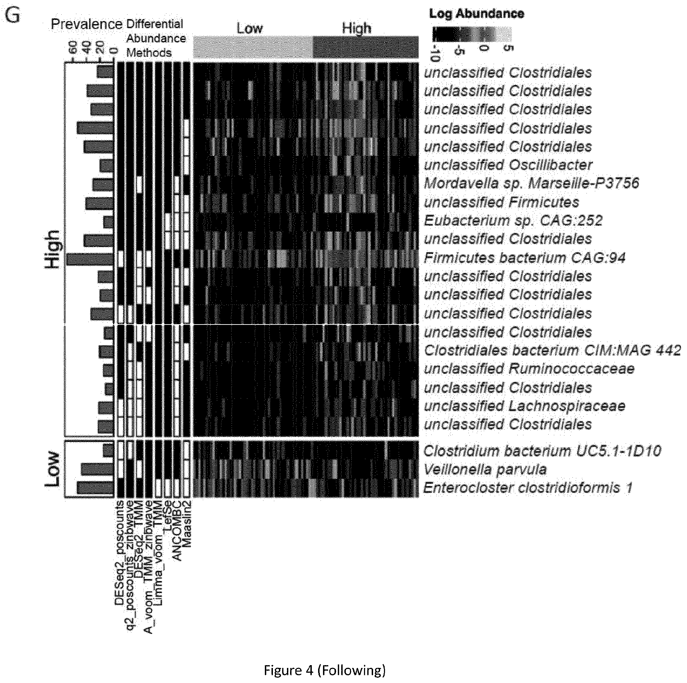

**Figure 4****. Serum soluble MADCAM-1 is a predictor of clinical benefit to PD1 blockade in NSCLC patients and a proxy for gut dysbiosis.**

**A**.Spearman correlation between RT-PCR -determined ileal *Madcam1* gene expression levels and serum soluble MADCAM-1 (determined by ELISA) in 65 MCA205 tumor bearing mice, each dot representing one animal. **B.** ELISA monitoring of serum levels of soluble MADCAM-1 in 301 NSCLC patients belonging to 2 independent cohorts (C01, C02, left panel) according to history of their recent ATB uptake (right panel) compared with serum levels in healthy volunteers (HV, n=70). Each dot represents one patient's serum. C-D. Alpha and beta diversity of the taxonomic content of the intestinal microbiota according to the serum sMADCAM-1 levels in NSCLC patients. MGS diversity-based richness evaluation in shot gun MG sequencing according to the median of sMADCAM-1 in the whole population composed of 95 NSCLC patients (C) and interindividual variability of the composition in both groups (D). **E-F.** Kaplan Meier survival curves and Cox regression linear analysis of the predictive value of serum levels of sMADCAM-1 split according to the median of each cohort in two independent (left and right columns) cohorts of stage IV NSCLC treated with αPD-1 mAb immunotherapy (refer to Tables 5-6 for patient description and multivariable analysis) for PFS (E) and OS (F). **G.** Supervised hierarchical clustering of MGS (with prevalence>10%) according to sMADCAM-1 serum levels (< (Low) or > (High) median), the color code featuring the relative abundance of each significant species, aligned in bar graph in the left column using various pipelines and algorithms. **H.** Relative abundance of *E. clostridioformis* in percentage among all MGS species in fecal materials of 95 patients, split in two groups according to their sMADCAM-1 serum levels (< (Low) or > (High) median). Each dot represents one feces/serum/patient. Student t' test.

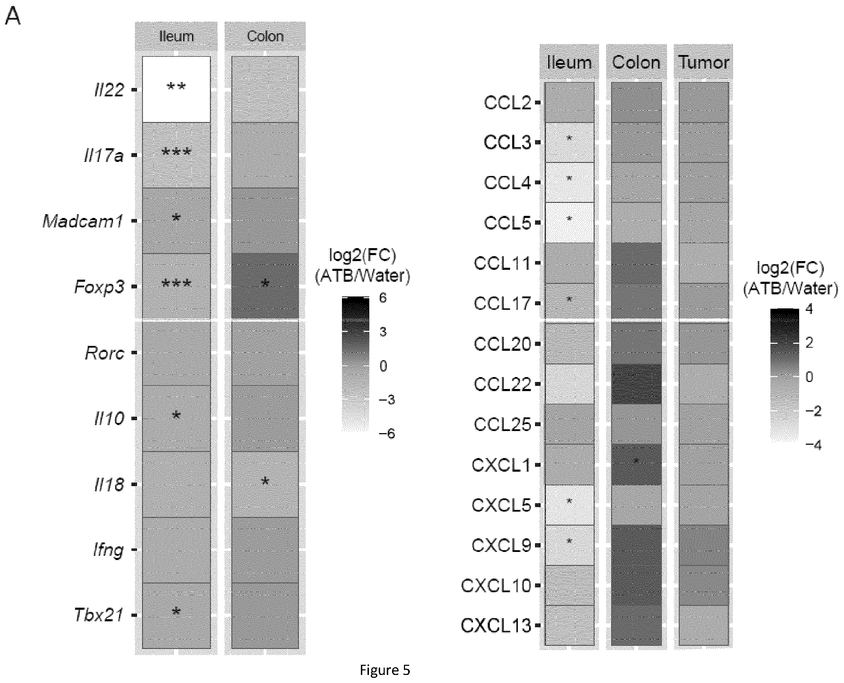

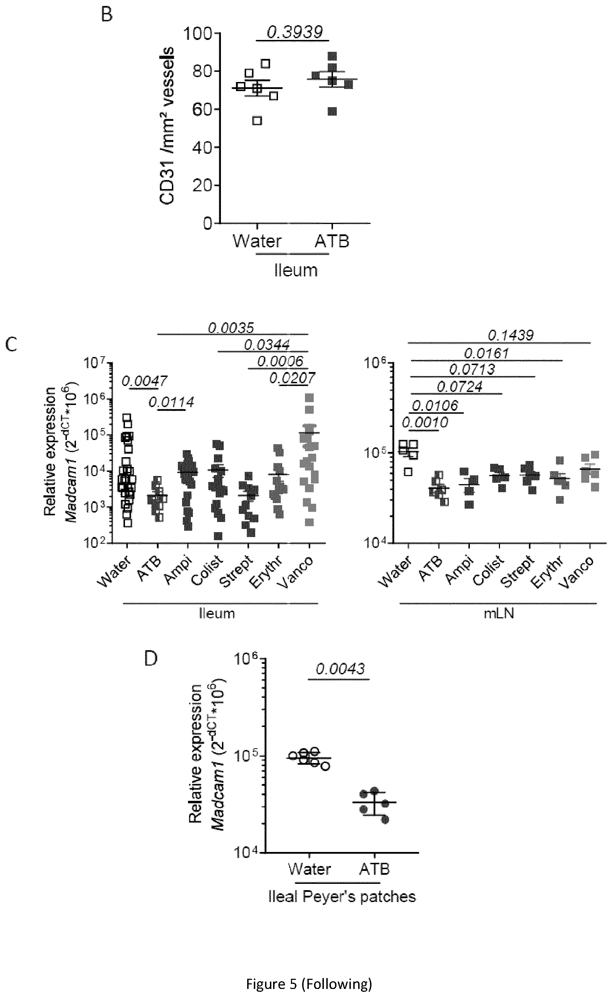

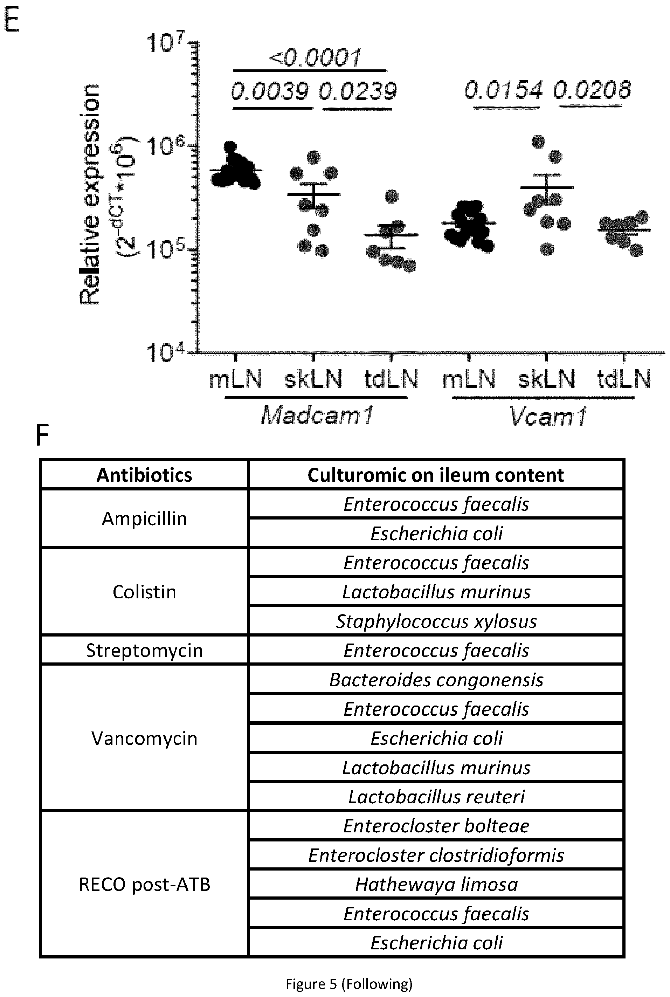

**Figure 5****. ATB-induced dysbiosis affects the transcriptional program of immune gene products in the intestinal lamina propria.**

**A**.Heatmap of log2 Fold Change ratios between ATB-treated and not treated (Water) ileum, colon, as well as tumor beds for chemokines (left panel) and cytokines/transcription factors (right panel) expression profiles in ELISA (left panel) and RT-PCR (right panel) of the tissue lysates. Statistics: test of Mann-Whitney with *p-value* adjusted by Benjamini-Hochberg method, *p<0.05, **p<0.01, ***p<0.001. **B.** Evaluation of CD31 expression on ileal HEV by IHC in ATB-treated mice. C. RT-PCR evaluation of the impact of 7 days conditioning with various antibiotic regimens or after ATB _{RECO} on relative transcription levels of *Madcam1* gene in ileal tissue (**left**) or in mesenteric lymph nodes (**right**). *Continuous (c.), Ampi_{C}: c. Ampicillin, Colist_{C}: c. colistin, Strepto_{C}: c. Streptomycin, Vanco_{C} : c. vancomycin, Erythr_{C} : c. Erythromycin.Each* dot represents one ileum. The graph gathered 2-3 experiments comprising 5 mice/group. **D-E.** RT-PCR assessment of the relative expression of *Madcam1(D, E)* and *VCAM1 (E)* genes in Peyer's patches (PP) (D) or various lymph nodes (LN) (mLN: mesenteric LN, sk: skin LN, td: tumor draining LN) (E). Each dot represents one ileum. The graph gathered 2 experiments comprising 5 mice/group. **F.** Culturomics, in aerobic and anaerobic conditions, of ileal material harvested from mice treated with various ATB regimen (refer to C) and sacrificed at day 4 after their stop. **G.** Qualitative identification by mass spectrometry of cultivated species in each condition. **H-I**. RT-PCR evaluation of murine *Foxp3, RORc* and *IL17a* genes in ileal tissue of mice treated or not with ATB (H) and Spearman correlations between ileal *Madcam1* and *Rorc* or *Foxp3* expression levels in mice (I). Each dot represents one animal. Concatenated data from 3 independent experiments. **J.** Spearman correlations between RT-PCR -based transcriptional levels of human *FOXP3 (**left**), and IL-17A (**right**) and Madcam-1* genes in intestinal biopsies collected during endoscopic intervention in 9 control (ATB free) patients and 7 ATB-treated patients (Table 1). Each dot represents one biopsy either from ileum, colon and cecum, a single patient being represented 1 to 3 times. ANOVA statistical analyses (Kruskal-wallis test): *p<0.05, **p<0.01, ***p<0.001.

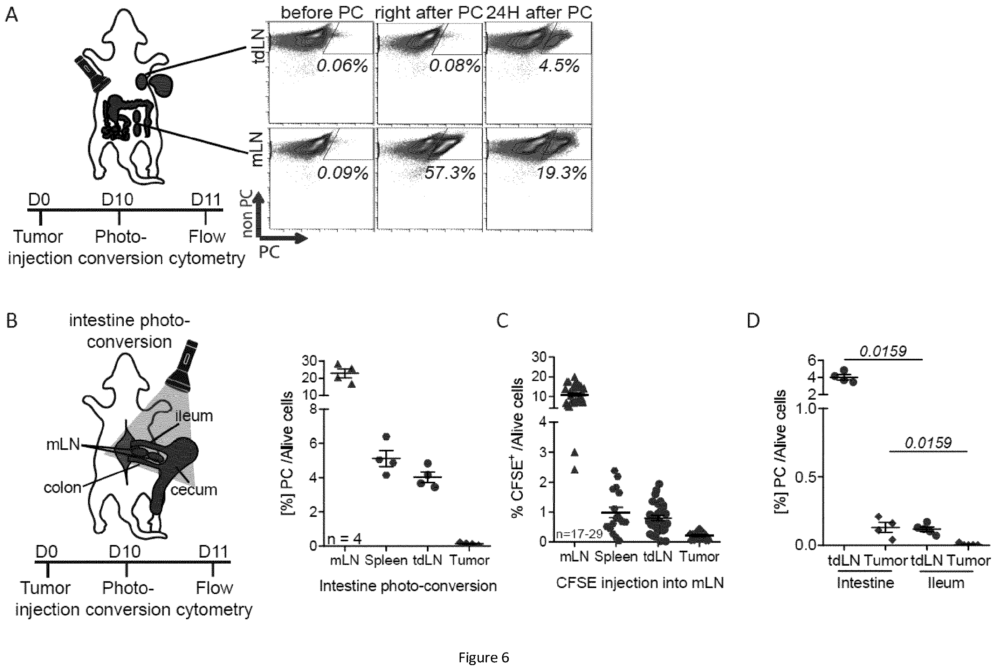

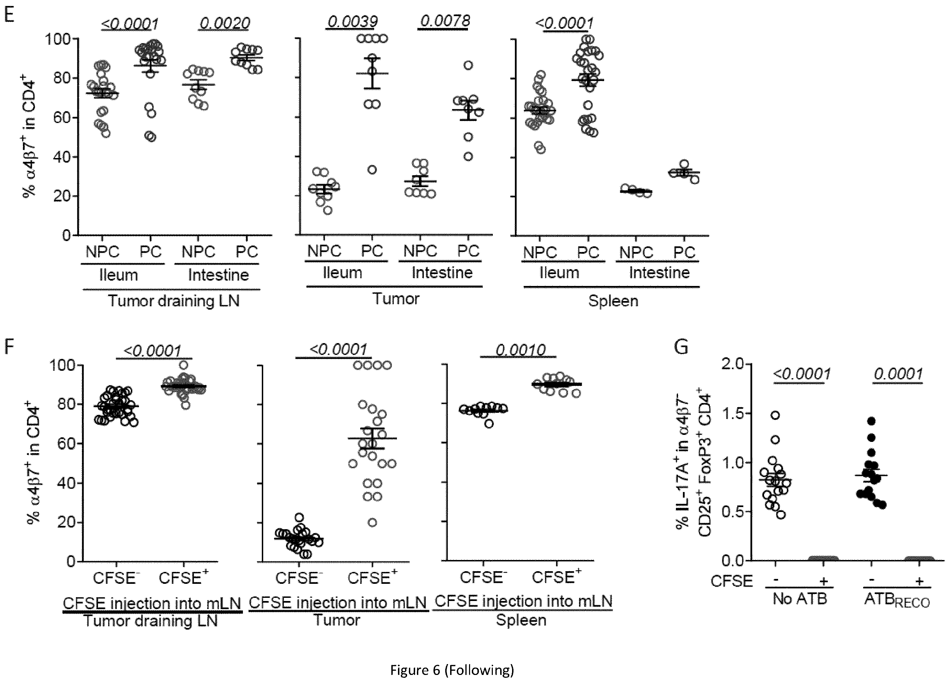

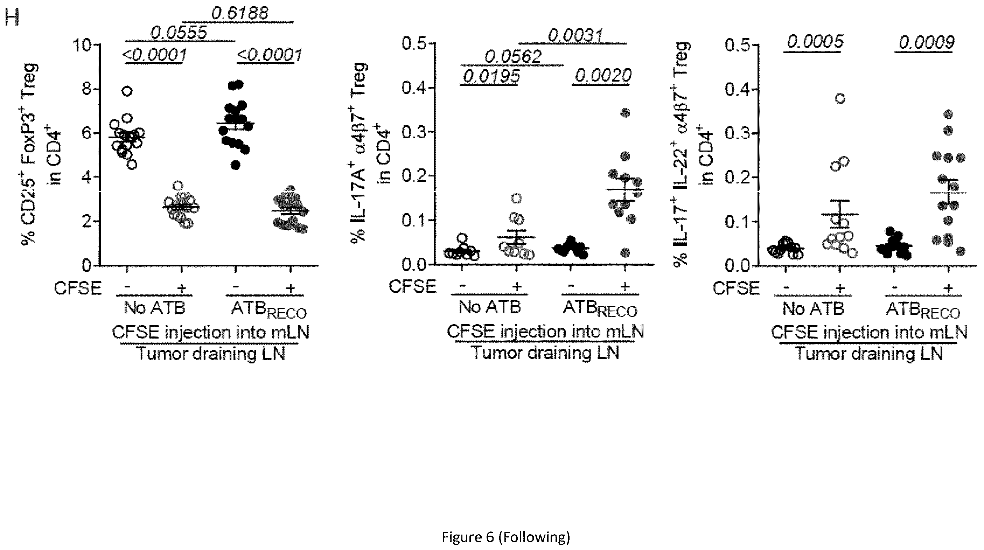

**Figure 6****. Profiling of gut-originating cells in various compartments.**

**A.** Schematic overview of the experimental setup of UV-A illumination of the intestine (ileum, caecum, mLN) in tumor-bearing Kaede mice and flow cytometric gating strategy of Photoconverted cells (PC) in tdLN (upper panels) and mLN (lower panels). PC frequencies in target organs before, 5 minutes and 24 hours after laparotomy and UV-A illumination of the intestine compartment are indicated. **B.** Id. As in A. with detailed percentages of PC cells in mLN, spleen, tdLN and tumor beds at 24 hours, each dot representing one mouse. A typical experiment out of 3 yielding similar results is depicted. **C.** Experimental setting (left panel) and flow cytometric determination of CFSE+ cells 24 hrs after CFSE injection in mLN in different tissues (x axis), each dot representing one mouse. Data from 3 independent experiments are pooled. **D.** Id. As in B. monitoring the mobilization of enterotropic cells after UV-A illumination of "intestine" (ileum+caecum+mLN) versus ileum only, towards tdLN or tumor beds, each dot representing one mouse. A typical experiment out of 2 yielding similar results is depicted. **E-F.** Flow cytometric evaluation of gut integrin α4β7 expression by resident (NPC) or recirculating (PC) CD4⁺ T cells in the spleen, tdLN or tumor bed of Kaede mice according to the area of UV-A illumination (ileum vs "intestine" (ileum+caecum+mLN) or in WT mice CFSE injected in mLN (**F**). **G-H.** Flow cytometric evaluation of different CD4+ Treg cell types (varying in the expression of α4β7, IL-17, IL-22) in tdLN traced by CFSE labeling in animals treated or not with ATB, and bearing subcutaneous MCA205 and subjected to CFSE injection in the mLN 24 hours before. ANOVA statistical analyses (Kruskal-Wallis test): *annotated raw p values.

**Figure 7****. Gene expression profiling induced by recolonization.**

**A**.Experimental design for- scRNA seq data collection. Single cell transcriptomics was performed on CFSE+ CD4+ T cells harvested from tdLN 24 hrs post-CFSE inoculation in mLN in 4 experimental groups of mice (water, continuous ATB, ATB RECO, E. clostridioformis) containing 5 MCA205 tumor-bearing animals. **B.** Absolute numbers of CFSE+ cells recovered in tdLN for each condition after quality control and sequenced. **C.** Volcano plot depicting the differential gene transcription in RNA sequencing of CFSE⁺ CD4+ T cells belonging to the Treg subset cells in the group ATB_{RECO} versus the water group (control). Volcano plots were generated computing for each gene product expressed in each cell type for 5 mice i) the log2 of fold ratio (FR) among the mean relative abundances of transcripts after normalization in group 3 versus group 1 (x axis); ii) the co-log10 of *p-values* deriving from Mann-Whitney U test calculated on relative abundances in absolute values (y axis). Blue (downregulated gene products) and red (upregulated gene products) dots are considered significant *(p<0.05)* while back dots are not (*p>0.05*)*.* **D.** Idem as C but comparing the gene transcripts in Treg versus the other T cell subsets within the ATB _{RECO} (group 3, left panel) or ATB *E. clostridioformis* (group 4). **E**. Idem as D but comparing the gene transcripts in proliferative T cell subset versus the other T cell subsets within the negative control group (water, group 1, left panel) and the ATB _{RECO} (group 3, right panel). **F.** Idem as E but comparing the gene transcripts in cells belonging to cluster 1 (in UMAP, Fig. 2I) in two groups of mice (water, group 1 versus ATB _{RECO} (group 3), in the left panel, and then, comparing the transcription profile of these cluster 1 cell subset to the other clusters in the ATB _{RECO} (group 3) (right panel). G. Idem as E but comparing the gene transcripts in cells belonging to cluster 0 (in UMAP, Fig. 2I) in two groups of mice (water, group 1 versus ATB _{RECO} (group 3).

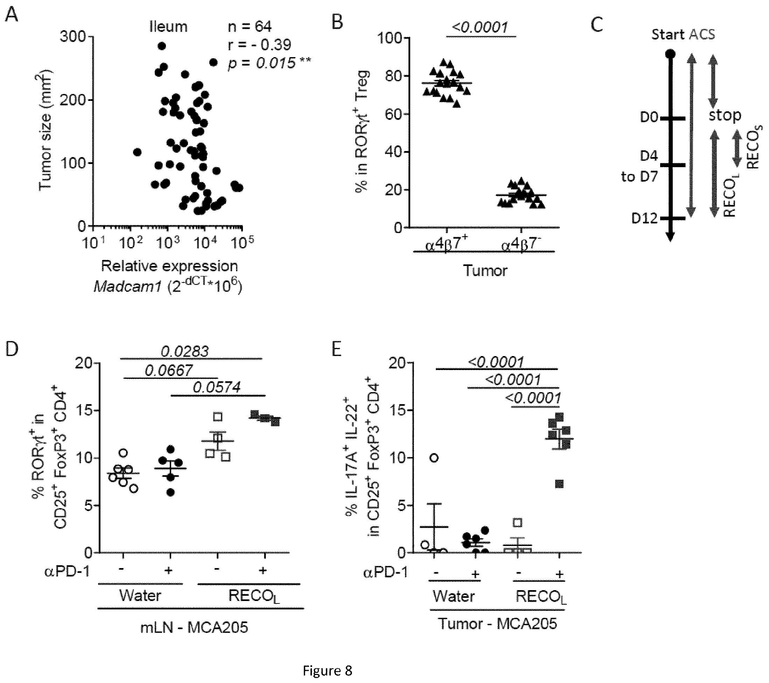

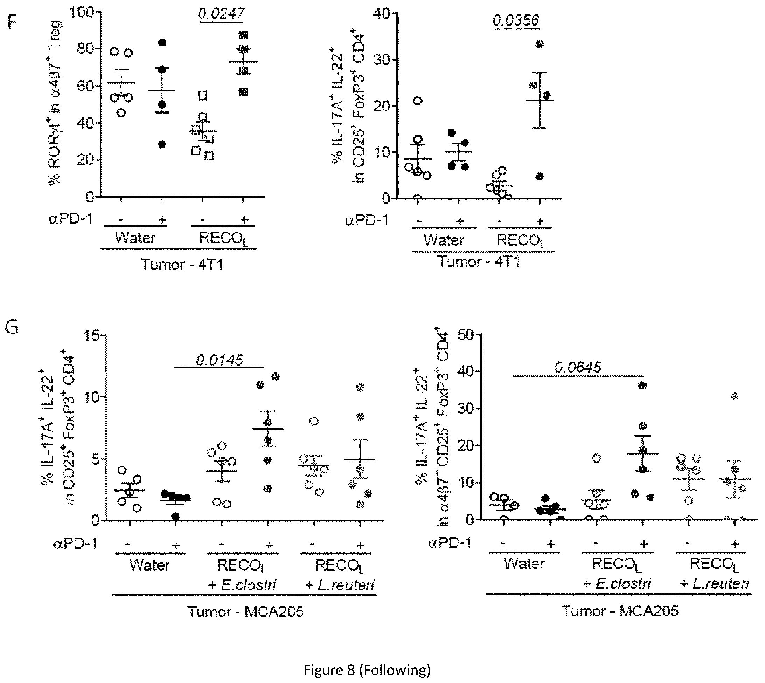

**Figure 8****. αPD-1 mAb aggravates the accumulation of enterotropic α4β7⁺RORγt⁺ T_{reg} or IL-17A⁺ IL-22⁺ T_{reg} towards tumor lesions caused by ATB-induced dysbiosis.**

**A**.Spearman correlations between ileal *Madcam1* and tumor size in MCA205 tumor bearing (anti-PD1 or isotype Ctl Ab -treated) mice. Each dot represents one mouse. ANOVA statistical analyses (Kruskal-wallis test): *annotated raw p values. **B.** Flow cytometric assessment of RORγt+ Treg (Tᵣ17) among α4β7⁺ or α4β7⁻ CD4⁺ TIL fractions in subcutaneous MCA205 tumors. Each dot represents one tumor and mouse. C. Experimental setting of PD1 blockade during ATB intervals of various durations in tumor bearers. **D-F.** Flow cytometric assessment of RORγt⁺ or IL-17A⁺ IL-22⁺ cells within Treg in the mLN (D) or in subcutaneous MCA205 (**E**) or 4T1 (**F**) tumors. **G.** Flow cytometric determination of IL-17A⁺ IL-22⁺ secreting Treg (left) and α4β7⁺ Treg cells (**right**) in subcutaneous (s.c.) MCA205 tumors treated by PD1 blockade after ATB stop and spontaneous ATB_{RECOL} +/- enforced gut colonization with *E. clostridioformis* or *L. reuteri* bacteria during αPD-1 -based therapy. Each dot represents the tumor size in each animal at sacrifice, in a typical experiment containing 6 mice/group out of two yielding similar results. Refer to Table 4 for detailed phenotypes. ANOVA statistical analyses (Kruskal-Wallis test): *annotated raw p values.

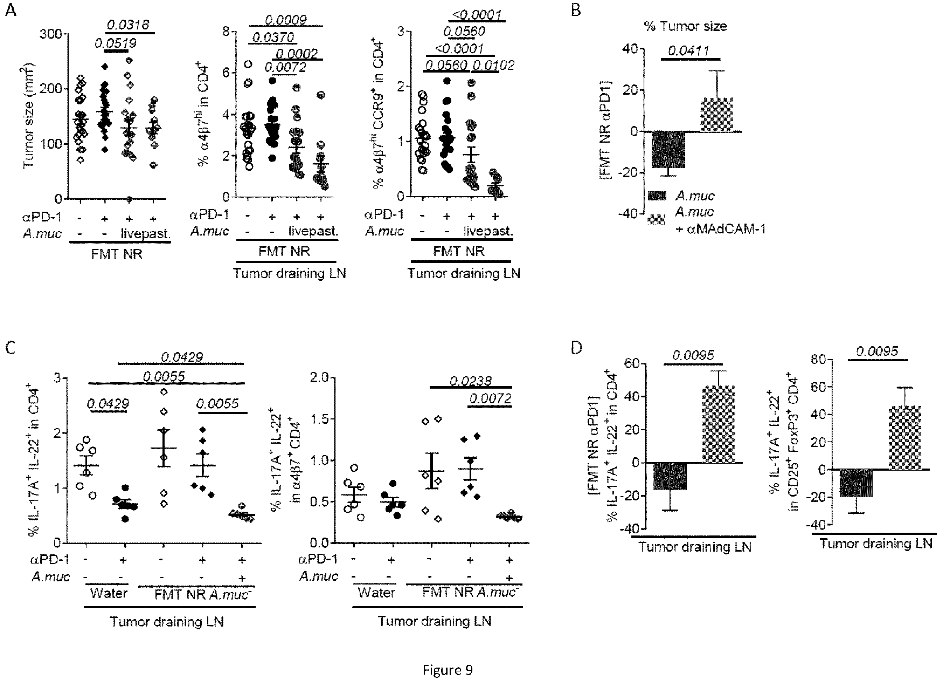

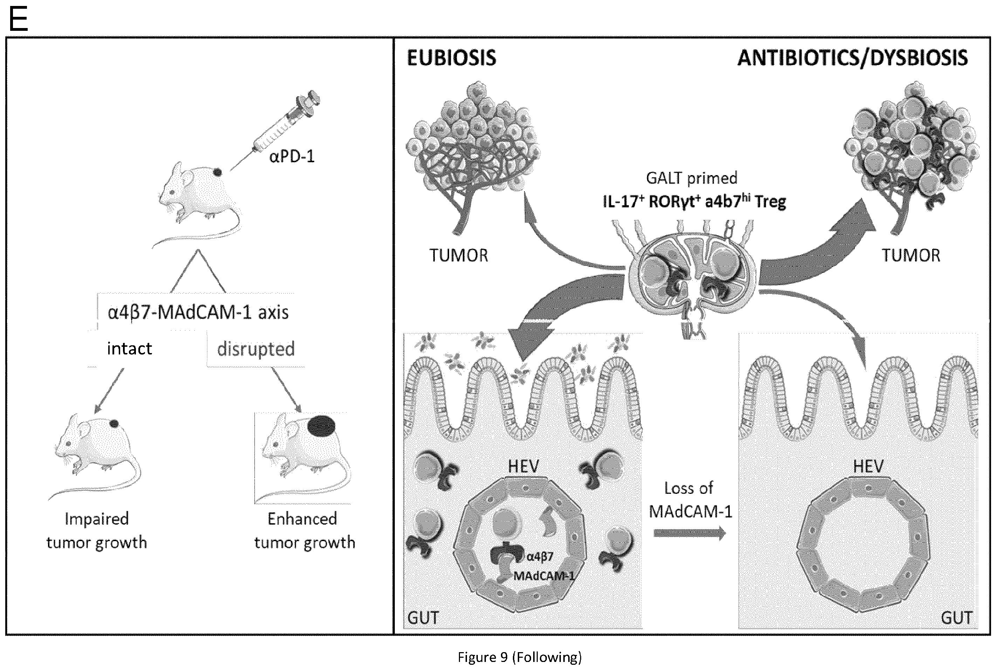

**Figure 9****. *Akkermansia muciniphila* upregulated ileal *Madcam-1* gene expression and prevented the recirculation of enterotropic T cells to tumor beds in a MADCAM-1-dependent manner.**

**A**.Tumor sizes at sacrifice in MCA205 tumor bearing mice treated with 3 days ATB and gut humanized using FMT from PD1 refractory NSCLC patients (FMT NR), supplemented or not with oral gavage of live or pasteurized A. *muciniphila SGB9228* (A. *muc, left panel).* Flow cytometric evaluation of α4β7 and/or CCR9 expression by CD4⁺ T cells in the tdLN draining s.c. MCA205 sarcoma (middle and right panels). **B.** Effects of anti-MADCAM-1 neutralizing antibodies on the compensatory effects of pasteurized A. *muciniphila SGB9228* for the response to PD1 blockade depicted as a ratio between tumor sizes in anti-PD1 -treated FMT NR avatar mice compensated with the bacterium +/- anti-MADCAM-1 **Abs.C-D.** Flow cytometric evaluation of IL-17A and IL-22 cytokine secretion by CD4⁺ T and Treg cells in the tdLN of mice colonized with FMT NR and supplemented by oral gavage with *Akkermansia muciniphila* (*A.muc*), receiving or not the αMAdCAM-1 mAb treatment and treated with αPD-1 mAb (C). Effects of the anti-MADCAM-1 neutralizing antibodies on the preventive effects of pasteurized A. *muciniphila SGB9228* to prevent the gut exodus of Tr17 depicted as a ratio between IL-17⁺IL-22⁺ Treg or total CD4⁺ T cells reaching the tdLN in anti-PD1 -treated FMT NR avatar mice compensated with the bacterium +/- anti-MADCAM-1 Abs (D). ANOVA statistical analyses (Kruskal-wallis test) or Wilcoxon matched-pairs signed rank test: *raw p values are annotated. **E**. Graphical abstract illustrating the recirculation toward the tumor microenvironment of gut-primed IL-17A⁺ IL-22⁺ RORγt⁺ α4β7⁺ Treg (Tᵣ17) cells occurring when there is a disruption of MADCAM-1 expression in ileal HEV in a context of ATB-induced dysbiosis.

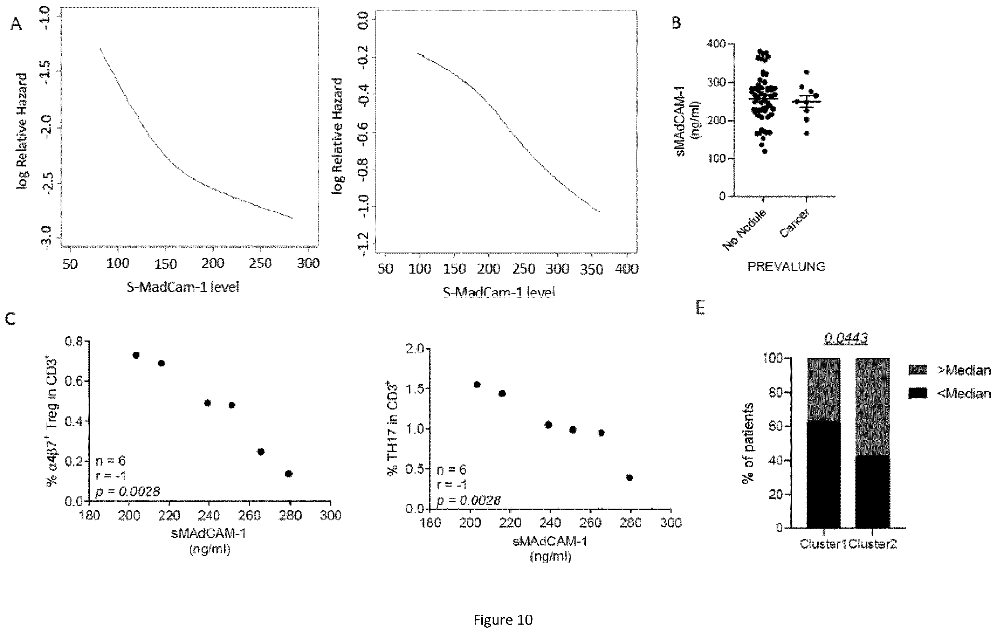

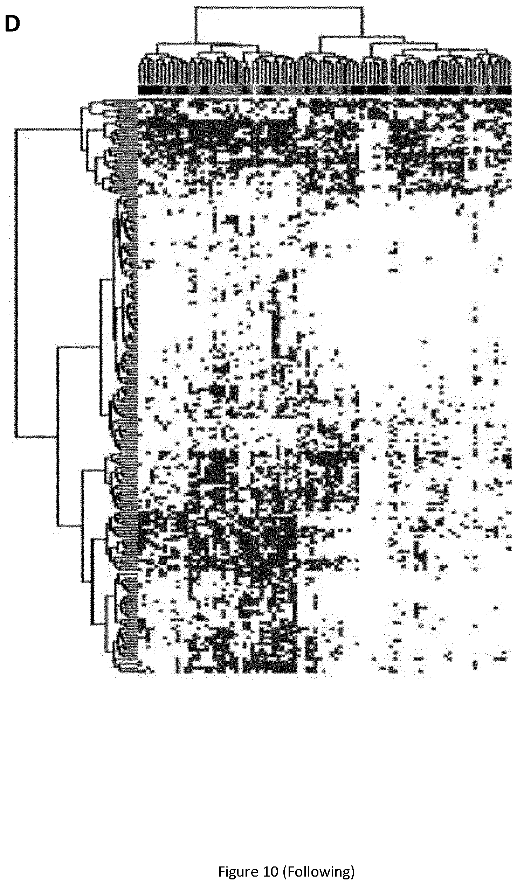

**Figure 10****. Soluble MADCAM-1 serum levels are robust predictors of clinical benefit to PD-1 blockade.**

**A.** Cox linear regression analysis evaluating the Hazard Ratio (HR) for the risk of progression or death during PD1 blockade and serum sMADCAM-1 (monitored in ELISA) in each cohort C01 and C02 described in Table 5, using restricted cubic splines (RCS) with 4 knots. The number of knots was chosen according to the Akaike Information Criterion. Left panel (C01): N=115 NSCLC patients, 62 deaths, median OS= 15 months (95%C.I: 11.2-25 mo), HR= 0.92 (95% C.I: 0.88-0.97, p=0.001) Right panel (C02): N=186 NSCLC patients, 83 deaths, median OS= 12 months (95%C.I: 9-17 mo), HR= 0.97 (95% C.I: 0.94-0.99, p=0.02). **B.** Prevalung study: Serum sMADCAM-1 levels in CVD smokers before lung cancer incidence in those patients who will be diagnosed with lung cancers (N=9) versus not (N=56 controls) within 2 years of follow up. **C.** Spearman correlation between serm sMADCAM-1 and circulating α4β7+ Roryt Treg. **D-E.** Non supervised hierarchical clustering of the taxonomic composition based on shot gun MG sequencing of feces in NSCLC patients according to sMADCAM-1 paired serum levels and ANOVA statistical analysis of the segregation between taxonomic MGS-based clustering and median of sMADCAM-1.

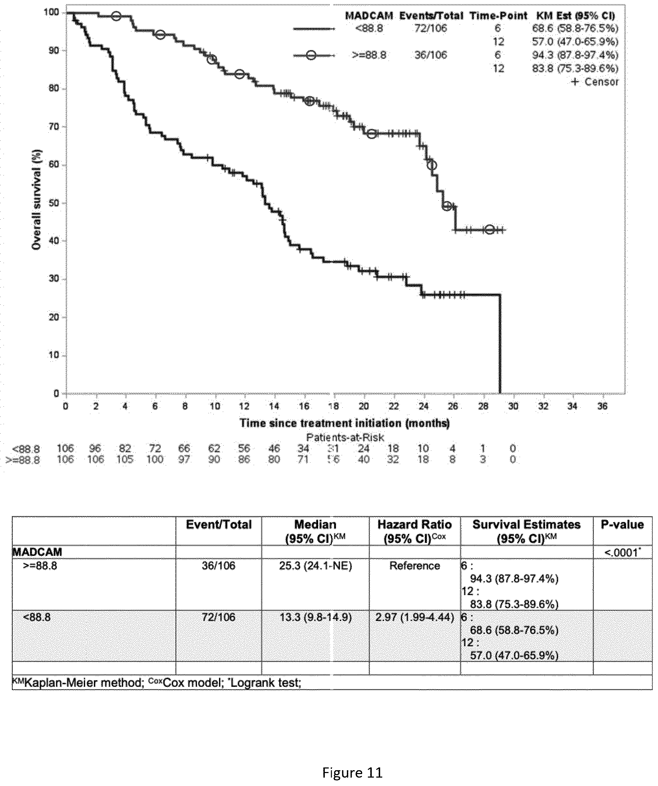

**Figure 11****. Kaplan Meier overall survival curves** and Cox regression univariate analysis using the Log rank statistical test of 212 RCC patients according to the median value of sMAdCAM-1 in the serum. RCC in second line immunotherapy based on anti-PD1 Ab after failure of tyrosine kinase inhibitors.

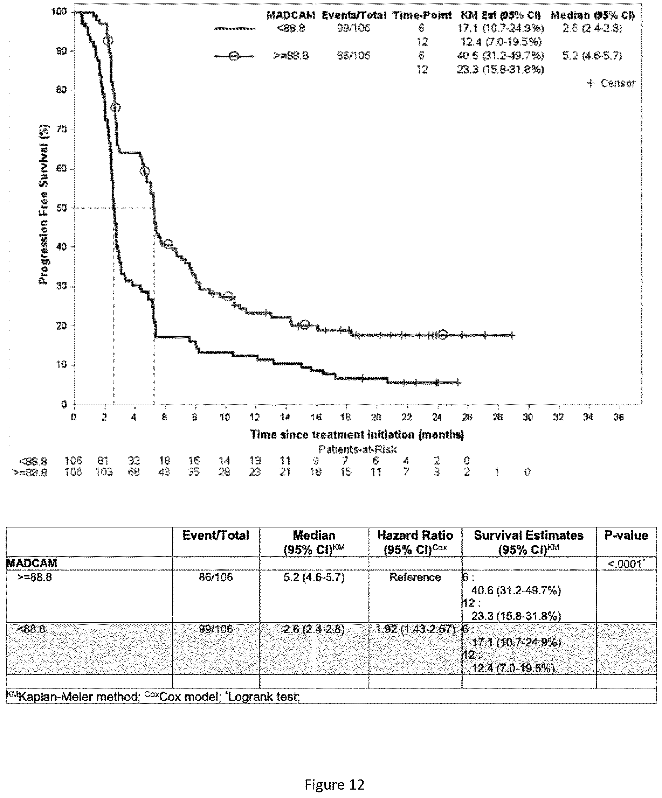

**Figure 12****. Kaplan Meir progression-free survival curves** and Cox regression univariate analysis using the Log rank statistical test of 212 RCC patients according to the median value of sMAdCAM-1 in the serum. RCC in second line immunotherapy based on anti-PD1 Ab after failure of tyrosine kinase inhibitors.

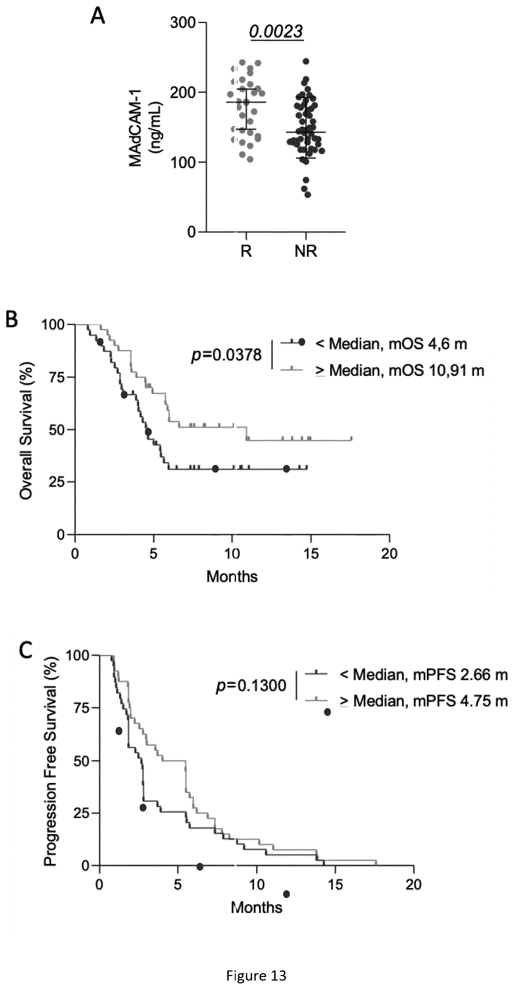

**Figure 13****. Efficacy of the biomarker sMAdCAM-1 to predict clinical benefit to anti-PDL-1 Ab Durvalumab in pre-treated second line metastatic bladder cancer patients.**

**A.** Concentrations of sMAdCAM_1 in serum of patients at baseline prior to Durvalumab and during therapy at 1 month and 4 months in responders (elite patients, PFS>5 months) and non responders (progressors OS>6 months), each dot representing one time point and patient, each patient being represented 3 times. **B-C**. Log rank univariate analysis of the predictive value of sMAdCAM_1 and Kaplan Meier curves of survival (OD, **B**) and PFS (C) in patients segregated according to the median value of MAdCAM_1 within the whole cohort.

### DETAILED DESCRIPTION

According to a first aspect, the present invention pertains to a method for *in vitro* diagnosing cancer-associated or antibiotics-associated intestinal dysbiosis in an individual having a cancer, comprising measuring soluble MAdCAM-1 in a serum sample from said individual (e.g., by ELISA), wherein a reduced level of serum soluble MAdCAM-1 indicates that the individual has a cancer-associated or antibiotics-associated intestinal dysbiosis.

Alternatively, the above method can be performed by assessing the expression of MAdCAM-1 in ileal lamina propria (LP) venules or in high endothelial venules (HEV) in an ileal biopsy from the patient, wherein a reduction of MAdCAM-1 expression in LP or HEV indicates that the individual has a cancer-associated ileopathy/dysbiosis. In such a case, the expression of MAdCAM-1 can be measured by RT-PCR or with an ELISA or flow cytometry or immunohistochemistry performed on the biopsy.

The present invention thus relates to the use of serum soluble MAdCAM-1 level, as a marker of cancer-associated dysbiosis or antibiotics-associated dysbiosis, wherein reduced level of serum soluble MAdCAM-1 is a marker of cancer-associated dysbiosis or antibiotics-associated dysbiosis.

As shown in the experimental part below, the inventors have demonstrated that the level of soluble MAdCAM-1 in the serum of an individual also is a marker of resistance or sensitivity to an immuno-oncology (I-O) therapy, wherein a reduced level of serum soluble MAdCAM-1 is a marker of resistance to the I-O therapy.

According to the present invention, the level of soluble MAdCAM-1 in the serum of an individual can thus be used to assess whether the individual is likely to resist to an immuno-oncology (I-O) therapy (low level). A normal (or elevated) level of serum soluble MAdCAM-1 is a marker of clinical benefit.

In the present text, the phrase "I-O therapy" includes immune checkpoint inhibitors (ICI), as well as CAR-T cells, adoptive TIL transfer and combinations thereof. In the context of the present invention, "I-O therapies" also include combined therapies including one of the above I-O agents and other antineoplastic treatments, such as chemotherapy, especially any combination of an immune checkpoint inhibitor (ICI) with taxanes, permetrexed, cis-platin and/or oxaliplatinum, or EGFR inhibitors.

In the context of the present invention, "ICI" include anti-PD1 antibodies (Ab), anti PDL-1 Ab, anti-CTLA4 Ab, anti-Lag3 Ab, anti-Tim3 Ab, anti-TIGIT Ab, anti-OX40 Ab, anti-41BB Ab, anti-VISTA Ab, bispecific antibodies targeting PD1 and Lag3, as well as other molecules exerting the same function(s), such as non-Ab molecules blocking any of the above immune checkpoints. According to a particular embodiment, the I-O therapy includes an anti-PD1/PDL-1 Ab, e.g. monoclonal Ab blocking PD1 or PDL-1.

In certain cases of treatment resistance, it has been shown that ICI drugs can not only be useless, but even have deleterious effects, leading to rapid tumor progression (i.e., hyperprogressive disease or HPD). Identifying a patient likely to resist to an I-O treatment is thus of major importance to decide not to administer the I-O treatment to this patient, at least not without a compensatory treatment or combined therapy to avoid HPD onset.

The present invention is particularly useful for assessing the resistance status of an individual who has taken broad spectrum antibiotics during a period ranging from 60 days before and 42 days after the first administration of an I-O therapy/ICI. Indeed, as shown in the experimental part below, broad spectrum antibiotics increase the risk of dysbiosis associated with I-O resistance.

When performing the above method, the level of serum soluble MAdCAM-1 is considered as reduced when it is inferior to a predetermined threshold. This threshold can be, for example, the median of the levels of soluble MAdCAM-1 in a representative cohort, such as a cohort of individuals suffering from cancer, preferably from the same cancer as the tested individual. Even more preferably, the representative cohort is a cohort of individuals who suffer from the same cancer as the tested individual and are receiving the I-O therapy/ICI in a same therapeutic scheme (especially, as a same line of therapy, at least differentiating 1L vs ≥ 2L therapy). The skilled person can refine the threshold by measuring the median of the levels of soluble MAdCAM-1 in a cohort of patients who share the same therapeutic history and received the same I-O therapy. In the experimental results disclosed below, the value of this median was respectively, 177.1 ng/ml and 233.3 ng/ml in a discovery and a validation cohort in advanced non small cell lung cancer (NSCLC) patients treated with anti-PD1/L-1 antibodies with or without chemotherapy (Example 1), while it was 88.8 ng/ml in a cohort of patients with kidney cancer treated with nivolumab in second line therapy (Example 2.1), and of 158.8 ng/ml in a cohort of cancer patients with pre-treated metastatic bladder treated with durvalumab (Example 2.2).

Alternatively, the predetermined threshold can be calculated from a cohort of individuals not suffering from cancer. For example, the level of serum soluble MAdCAM-1 can be considered as reduced if it is in the inferior tertile of a cohort of individuals not suffering from cancer.

The above method is particularly interesting for patients suffering from a cancer amenable to immunotherapy alone or combined with chemotherapy or tyrosine kinase inhibitors or hormonotherapy (androgen or oestrogen deprivation, or LHRH antagonists).

Examples of such cancers include breast cancer, Chronic Myelomonocytic Leukemia (CMML), colorectal cancer, kidney cancer, lung cancer (e.g. NSCLC), urothelial cancer, melanoma, ovary cancer, stomach and oesophageal cancers, mesothelioma, hepatocarcinoma, prostate cancer and any mismatch repair insufficiency (MSI) high tumor for which there is an agnostic histology with FDA-approval for anti-PD1 Abs. According to a particular embodiment, the individual suffers from a cancer selected from the group consisting of NSCLC, melanoma, breast cancer, kidney cancer, bladder cancer and colorectal cancer.

The present invention also relates to a theranostic method for determining if an individual having a cancer needs a compensatory microbiota-centered intervention (MCI) before administration of an immune-oncology (I-O) therapy, comprising assessing, by the above method, whether the individual has a cancer-associated dysbiosis or antibiotics-associated dysbiosis, wherein if the individual has a cancer-associated dysbiosis or antibiotics-associated dysbiosis, he/she needs a MCI before administration of the treatment with an I-O therapy.

In certain cases, such as lung cancers, the MCI can be combined to another treatment, for example to chemotherapy for avoiding HPD.

In the present text, the phrase "microbiota-centered intervention (MCI)" designates any treatment having a direct or indirect effect on the intestinal microbiota composition.

Examples of MCI according to the invention include:
- oral vancomycin antibiotics (e.g., same protocol as for treating C. *difficile* infection),
- phages killing bacteria of the *Enterocloster* gen. nov. clade,
- rare-cutting endonucleases such as Crispr Cas9 engineered to kill bacteria of the *Enterocloster* gen. nov. clade,
- *Akkermansia* spp and/or *Akkermansia muciniphila,* possibly mixed with other beneficial bacteria,
- retinoic acid
- fecal microbial transplantation (FMT), especially with a product proved to increase the MAdCAM-1 expression levels in endothelial cells of the lamina propria, for example by a method as described below, and
- mixtures of the above treatments.

In the above, phages and endonucleases are considered as "killing bacteria of the *Enterocloster* gen. nov. clade" if they are able to kill bacteria of at least one of the species of the *Enterocloster* list corresponding to clading taxonomy reported in Haas and Blanchard, Int J Syst Evol Microbiol 2020;70:23-34.

This list includes:
*Clostridium*/*Enterocloster asparagiformis*
*Clostridium*/*Enterocloster lavalense*
*Clostridium*/*Enterocloster boltae*
*Clostridium*/*Enterocloster clostridioforme*
*Clostridium*/*Enterocloster citroniae*
*Clostridium*/*Enterocloster aldenense*
*Clostridium*/*Enterocloster symbosium*
*Hungatella hathewayi*
*Hungatella effluvia*

The present invention also pertains to the use of an agent for MCI, as those listed above, for treating cancer in combination with an ICI in an individual having a reduced level of serum soluble MAdCAM-1.

Another object of the present invention is a method for following-up the success of a MCI, comprising measuring the level of serum soluble MAdCAM-1, wherein a normalized level of serum soluble MAdCAM-1 indicates that the MCI successfully restored gut eubiosis, or at least partially corrected the dysbiosis.

When performing this method, the level of serum soluble MAdCAM-1 can be considered as "normalized" if it is higher than a predetermined threshold (as described above). Alternatively, it can be considered as "normalized" or at least "partially normalized" if it is higher than before the MCI.

The present invention also relates to the use of serum soluble MAdCAM-1 level, as a biomarker predicting early NSCLC incidence in high risk heavy smokers experiencing a cardiovascular event (HRHSCV), wherein reduced level of serum soluble MAdCAM-1 is a marker of predicting the onset of NSCLC.

In the experimental part which follows, the inventors demonstrated that in the setting of intestinal dysbiosis, PD1 blockade facilitates the amplification and homing to tumors of TH17/Tr17 in the GALT. They also showed that in such a situation, blocking IL-17A bioactivity could circumvent the deleterious effects of dysbiosis.

Hence, combining IL-17A and PD1 blockades is clinically very interesting for patients who have been identified as likely to resist to a treatment with an anti-PD1 Ab by one of the methods described above.

Besides, IL-7 controls α4β7 integrin expression and imprints gut-homing specificity on T cells, acting on CD127 highly expressed on emigrating Tr17 in inflammatory bowel disease (Belarif et al. J. Cin Invest May 2019, vol 129, numero 5).

As a result, combining recombinant IL-7 with PD1 blockade is another option clinically very interesting for patients who have been identified as likely to resist to a treatment with an anti-PD1 Ab by one of the methods described above.

Another aspect of the present invention is thus the use of a combined therapy comprising (i) an anti-PD1 or an anti-PDL1 antibody and (ii) an anti-IL17A or anti-IL-17R antibody and/or recombinant IL-7, for the treatment of cancer in a patient having a low serum level of soluble MAdCAM.

In the above combined therapy, the two or more agents can be administered together or separately, starting by blocking IL-17R or IL-17 and then inhibiting PD1 or PDL-1/PDL-2.

Other aspects of the present disclosure, not claimed as such in the present patent but still not inconsistent with the claimed subject-matter, relate to methods and tools for identifying agents capable of normalizing MAdCAM-1 expression levels in endothelial cells of the lamina propria.

Such a method comprises (i) *in vitro* contacting cells expressing MAdCAM-1, or engineered to express a reporter gene under the control of the MAdCAM-1 promoter, with compounds to be tested and (ii) assessing which of said compounds induce the expression of MAdCAM-1 or of the reporter gene expressed under the control of the MAdCAM-1 promoter.

Examples of cells that can be used to perform this method include Human umbilical vein endothelial cells (HUVECs), transformed sinusoidal endothelial cells (TSECs), bEnd.3 cells and derivatives thereof (in particular, engineered cells derived from these cell lines by stably integrating a reporter gene under the control of the MAdCAM-1 regulatory elements).

According to another method, not claimed as such in the present patent but still not inconsistent with the claimed subject-matter, the compounds to be tested are administered to gut humanized avatar mice, and the expression of MAdCAM-1 in endothelial cells of said mice is measured.

The two methods can also be combined.

In such a combined screening method, the *in vitro* results are completed by a step of validation in avatar mice, wherein avatar mice are gut humanized mice that were pre-treated with 8-10 days broad spectrum ATB and then recolonized by a human FMT product by oral gavage, wherein:
(i) the FMT product has been identified as inducing the expression of MAdCAM-1 in cells expressing MAdCAM-1, or engineered to express a reporter gene under the control of the MAdCAM-1 promoter, or
(ii) the FMT product has been identified as not inducing the expression of MAdCAM-1 in such cells and has been enriched with another agent that has been identified as inducing the expression of MAdCAM-1 in these cells, or
(iii) the FMT has a taxonomic composition associated with eubiosis or high soluble MADCAM-1 (in our data base or as a result of any former experiments).

Another method, not claimed as such in the present patent but still not inconsistent with the claimed subject-matter, can also comprise:
(i) by 8 days post-gavage, sacrificing some of the gut humanized avatar mice to run a MAdCAM-1 and Foxp3-based ileal PCR (and optionally, IHC stainings) in FMT recipients and comparing them to controls (continuous ATB);
(ii) in case of a significant upregulation of MAdCAM-1 in the gut-humanized avatar mice, inoculating a subcutaneous sarcoma in at least 3 mice of each group and treating them with anti-PD1 Abs to ensure that this FMT can mediate therapeutic efficacy compared with ATB-treated mice;
(iii) if gut-humanized avatar mice respond to the treatment with anti-PD1 Abs, deducing that the FMT product or the non-FMT agent that has been used to enrich it is capable of normalizing MAdCAM-1 expression levels in endothelial cells of the lamina propria.

A screening platform for performing the above method is also described and not claimed as such in the present patent but still not inconsistent with the claimed subject-matter. Such a platform comprises cells expressing a reporter gene under the control of the MAdCAM-1 promoter (e.g., HUVECs, TSECs or bEnd.3 cells, or cells derived therefrom) and a robot configured for picking agents in a bank of products, delivering these to cultivated cells and assessing expression of the reporter gene (for example by measuring fluorescence if the reporter gene expresses a fluorescent protein).

As mentioned above, a low level of soluble MAdCAM in the serum of a cancer patient is a marker of gut dysbiosis in this patient, likely to induce resistance to I-O therapy, so that this patient needs a compensatory preparation before the I-O therapy, and can advantageously benefit from a treatment comprising the steps of:
(i) administering an MCI selected from those described above, and
(ii) administering an I-O therapy selected from those described above.

Measuring the level of soluble MAdCAM in the serum of a cancer patient before beginning or continuing an I-O therapy (for example, by the above-described method) helps determining if the patient is in need of a combined treatment (MCI + I-O).

Patients for whom this measure is particularly useful are those who have taken broad spectrum antibiotics during a period ranging from 60 days before and 42 days after the first administration of an ICI, especially an anti-PD1/PDL-1 Ab, because they are more likely to have a severe gut dysbiosis.

Advantageously, a new measure of the serum level of soluble MAdCAM is done before step (ii) above to check whether the MCI has reduced the cancer-associated dybiosis.

When performing the above combined treatment (MCI + I-O), not claimed as such in the present patent but still not inconsistent with the claimed subject-matter, an individual who has received broad spectrum antibiotics less than 60 days ago can advantageously receive a fecal microbial transplantation, possibly enriched with pasteurized *Akkermansia* spp and/or *Akkermansia muciniphila;* and an individual who has not received broad spectrum antibiotics less than 60 days ago can advantageously receive live *Akkermansia* spp and/or *Akkermansia muciniphila.*

Other characteristics of the invention will also become apparent in the course of the description which follows of the biological assays which have been performed in the framework of the invention and which provide it with the required experimental support, without limiting its scope.

### EXAMPLES

### Example 1: Antibiotics disrupt ileal MAdCAM-1/α4β7 axis, compromising tumor immunosurveillance during PD-1 blockade

### Materials and Methods

### Medical centers and regulatory approvals for translational research.

*Fecal material.* For feces collection, ancillary studies were conducted at Institut Gustave Roussy/France according to the ethical guidelines and approval of the local CCPPRB. The study name was "Oncobiotics", B2M ethics protocol number PP: 15-013. Written informed consent in accordance with the Declaration of Helsinki was obtained from all patients. For collection of endoscopic and blood samples a clinical study "Einfluss von Antibiotika auf das Darm-Chemokinnetzwerk bei Patienten mit soliden Tumoren" was conducted at University Clinics Heidelberg/Germany according to the ethical guidelines and approval of the Regierungspräsisium Karlsruhe. Eligible patients had stage IIIA-IV non-small-cell lung cancer (NSCLC) with squamous or non-squamous histology and had documented recurrence or progression after at least one prior line of treatment. Participant sites were Gustave Roussy Cancer Campus (Villejuif, France). NSCLC patients received anti-PD-1 mAb, nivolumab, as part of the treatment modalities approved by the European Medical Agency (EMA). Tumor response was assessed using the Response Evaluation Criteria in Solid Tumors version 1.1 (RECIST1.1). Computer tomography (CT) scans were performed at baseline and every 8 to 12 weeks for the first year and then every 12 to 15 weeks until disease progression. Feces were collected according to International Human Microbiome Standards (IHMS) guidelines (SOP 03 V1) before the first injection (T0). In brief, a collection kit including an anaerobic generator (Biomerieux) was given to patients. Samples were collected by patients at home, and frozen 4 to 24h later at -80°C at Gustave Roussy Cancer Campus in plastic tubes (Plastic vessel by 1000-Sarstedt) with or without BHI + 2 % glycerol.

*Collection of endoscopic samples and blood samples.* Eligible patients underwent ileo-colonoscopy according to clinical standard protocols for non-study related indications (Table 1) between July 2018 and November 2019. When feasible, endoscopic biopsy of the mucous membrane of the terminal ileum, cecum, and right and left colon was performed on each patient. Tissue samples were either snap frozen in liquid nitrogen and stored at -80°C or immersed into 2% PFA for histology. In addition, two blood samples (10 ml EDTA tubes) were collected before ile-colonoscopy. All included patients responded a questionnaire to assess dietary history and clinical baseline data was retrieved from the local clinical information system.

**Cell culture,** reagents **and tumor cell lines.** MCA-205 fibrosarcoma cells, MC38 and RET melanoma cells (i.e. melanomas generated by transgene-enforced expression of the Ret proto-oncogene under the control of the metallothionein-1 promoter driving spontaneous melanomagenesis, kindly provided by Prof. Viktor Umansky) (all syngeneic for C57BL/6 mice) were cultured at 37°C in the presence of 5% CO2 in RPMI 1640 containing 10% FCS, 2 mM L-glutamine, 100 Ul/ml penicillin/streptomycin, 1 mM sodium pyruvate and MEM non-essential amino acids (henceforth referred to as complete RPMI 1640). Luciferase-transfected TC-1 cell lines (syngeneic for C57bl6 mice, kindly provided by Pr. Eric Deutsch, Institut Gustave Roussy, France) were cultured at 37°C in the presence of 5% CO2 in complete RPMI 1640 and 1mM Hepes buffer. Cell lines were regularly tested for mycoplasma contamination and were not used after more than 10 passages.

**Mice**. All animal experiments were carried out in compliance with French and European laws and regulations. The local institutional animal ethics board and French Ministère de la Recherche approved all mouse experiments (permission numbers: 2016-049-4646, 2017_049_99741, 2019_036_21124). Experiments were performed in accordance with Government and institutional guidelines and regulations. Female C57BL/6 were purchased from Harlan (France). Mice were used between 7 and 12 weeks of age. MAdCAM-1-KO and ITGB7-KO mice were a kind gift from Angela Schippers, University hospital Aachen, Aachen, Germany. MAdCAM-1-KO and ITGB7-KO mice and control littermates were backcrossed on the C57BL/6 background and were obtained from an in-house-breed at the local animal care facility at the University Hospital Aachen. CCR5-KO mice were a kind gift from Christophe Combadière, Hopital Salpetrière, Paris, France. CCR9-KO mice were a kind gift of Reinhold Förster, University hospital Hannover, Hannover, Germany. CCR5-KO and CCR9-KO mice were both maintained on a C57BL/6 background. All mouse experiments were performed at the animal facility in Gustave Roussy Cancer Campus where animals were housed in specific pathogen-free conditions.

**Antibiotic treatments.** If not indicated otherwise, mice were treated with a broad spectrum antibiotic solution (ATB) containing ampicillin (1 mg/ml), streptomycin (5 mg/ml), and colistin (1 mg/ml) (Sigma-Aldrich) added to the sterile drinking water of mice. In experiments where single antibiotics were used the concentrations were ampicillin (1 mg/ml), streptomycin (5 mg/ml), colistin (1 mg/ml), erythromycin (1 mg/ml) or ciprofloxacin (0.1 mg/ml) respectively. Solutions and bottles were changed 2 times weekly. Antibiotic activity, in the experiments where the antibiotic mix was used, was confirmed by cultivating fecal pellets resuspended in BHI+15% glycerol at 0.1 g/ml on COS (Columbia Agar with 5% Sheep Blood) plates for 48h at 37°C in aerobic and anaerobic conditions weekly. Duration of ATB treatment was slightly different (7-14 days before aPD-1 treatment) based on the experimental settings and is indicated in results for each experiment. In brief, to compromise the efficacy of anti-PD-1 mAb, mice were treated with ATB for one to two weeks before tumor implantation and ATB was continued throughout the experiment or discontinued on the day of anti PD-1 treatment initiation as indicated in the individual experiment. In the context of fecal microbial transplantation experiments, mice received 3 days of ATB before undergoing fecal microbial transplantation the next day by oral gavage using animal feeding needles. In the TC-1 model, ATB treatment was started 3 days before tumor injection and discontinued one day before the first bacterial gavage.

**Subcutaneous model of MCA-205 sarcoma, MC38 and RET melanoma.** Syngeneic C57BL/6 mice were respectively implanted with 0.8 × 10⁶ MCA-205 sarcoma, 1.0 × 10⁶ MC38 or 0.5 x 106 RET melanoma cells subcutaneously and treated intraperitoneally (i.p.) when tumors reached 20 to 40 mm² in size with anti-PD-1 mAb (250µg/mouse; clone RMP1-14,) or isotype control (clone 2A3). Mice were injected 4 times at 3-day intervals with anti-PD-1 mAb. Tumor length and width was routinely monitored every 3 times per week by means of a caliper. In experiments using anti-a4b7 mAb (DATK32, 200µg per mouse) or anti-MadCAM mAb (MECA-367, 200µg per mouse), mAbs (or their isotype controls, clone 2A3 in both cases) were injected i.p. every 3 days starting from day 0 until the final anti-PD-1 injection. All antibodies were purchased from BioXcell, NH, USA.

**Orthotopic luciferase engineered-TC-1.** C57BL/6 mice were anesthetized with isoflurane. Under sterile conditions a lateral incision was made on the chest wall of each mouse and 6x10⁵ TC-1-Luc cells in 10µl Matrigel (Corning) were injected into the lung. The skin incision was closed with a surgical skin clip. Tumor growth was monitored twice weekly on an IVIS Imaging System 50 Series (Caliper Life Sciences/Xenogen). Starting on day 3 mice were injected with anti-PD-1 mAb or recommended isotype according to the same dose and schedule as above.

**FMT experiments.** Fecal microbiota transfer (FMT) was performed by thawing fecal material. Mice were placed in a new cage. Two hundred µL of the suspension was then transferred by oral gavage into each germ-free or ATB pre-treated (3 days) recipient. In addition, another 100µL was applied on the fur of each animal. Two weeks after FMT, tumor cells were injected subcutaneously or orthotopically and mice were treated with anti-PD-1 or isotype controls as mentioned above. Oral bacterial gavage was performed on the same days as aPD-1 treatment as described below.

**Oral bacterial gavage with commensal species.** *Akkermansia muciniphila* CSUR P2261, and *A. indistinctus* CSUR P723 were provided by the Institut hospitalo-universitaire Méditerranée Infection, Marseille, France. *Enterococcus hirae* 13144 isolates were originally isolated from spleens or mesenteric lymph nodes of SPF mice treated with CTX at Gustave Roussy Cancer Campus. *A. muciniphila* was grown on COS plates in an anaerobic atmosphere created using 3 anaerobic generators (Biomerieux) at 37°C for at least 72h. *E. hirae* 13144 were grown on 5% sheep blood enriched Columbia agar for 24h at 37°C in aerobic conditions. Colonization of ATB pre-treated or GF C57BL/6 mice was performed by oral gavage with 100 µl of suspension containing 1 × 10⁸ bacteria. For bacterial gavage: suspensions of 10⁹ CFU/mL were obtained using a fluorescence spectrophotometer (Eppendorf) at an optical density of 600 nm in PBS. Five bacterial gavages were performed for each mouse, the first 24 h before the first injection of antiPD-1 mAb and subsequently four times on the same day as anti-PD-1 mAb injection. The efficacy of colonization with *E. hirae* 13144 was confirmed by culturing the feces 48h postgavage. Fecal pellets were harvested and resuspended in BHI+15% glycerol at 0.1 g/ml. Serial dilutions of feces were plated onto 5% sheep blood enriched Columbia agar and incubated for 48h at 37°C in aerobic and anaerobic conditions. After 48h, single colonies were isolated and Gram staining was performed. The identification of specific bacteria was accomplished using a Matrix-Assisted Laser Desorption/ lonisation Time of Flight (MALDI-TOF) mass spectrometer (Andromas, Beckman Coulter, France).

**Flow cytometry analyses.** Tumors, mesenteric lymph nodes (mLN), draining lymph nodes (tdLN) and spleens were harvested at different time points as indicated in the individual experiments. Excised tumors were cut into small pieces and digested in RPMI medium containing LiberaseTM at 25 µg/mL (Roche) and DNase1 at 150 UI/mL (Roche) for 30 minutes at 37°C and then crushed and filtered twice using 100 and 70 µm cell strainers (Becton & Dickinson). Lymph nodes and spleen were crushed in RPMI medium and subsequently filtered twice through a 100 µm cell strainer. Four million tumor cells, lymph node cells or splenocytes were pre-incubated with purified anti-mouse CD16/CD32 (clone 93; eBioscience) for 30 minutes at 4°C, before membrane staining. For intracellular staining, the Foxp3 staining kit (eBioscience) was used. Dead cells were excluded using the Live/Dead Fixable Yellow dead cell stain kit (Life Technologies) or LIVE/DEAD^{™} Fixable Aqua Dead Cell Stain Kit. Anti-mouse antibodies for CD3 (145-2C11), CD4 (GK1.5 or RM4-5), CD8 (53-6.7), CD25 (3C7), CD44 (IM7), CD45 (30-F11), CD62L (MEL-14), CD127 (A7R34), Foxp3 (150D), RORγt (B2D), CXCR3 (FAB1685P), CXCR5 (J252D4), CCR2 (SA203G11), CCR4 (2G12), CCR5 (HM-CCR5), CCR6 (29-2L17), CCR7 (4B12), CCR9 (CW-1.2), beta7 (FIB504), a4b7/LPAM-1 (DATK32 and REA457), MadCAM-1 (MECA367) all purchased from Miltenyi, BioLegend and eBioscience) were used to stain cells. Stained samples were acquired on CytoFLEX S 13 colours (Beckman Coulter) or a BD Facs CANTO II (BD) and analyses were performed with Kaluza software 1.5 (Beckmann Coulter). T central memory (TCM) gating: after gating on CD3+ alive, CD4+ were selected, then TCM were identified as being either CD62L+. Effector memory T (TEM) cells were selected as being CD62L and CD44+. Th17 gating depended on the mousemodel used. The Kaede fluorochrome does not retain its photoconverted state upon fixation with PFA. Th17 gating: after gating on CD3+ alive, CD4+ were selected, then Th17 were identified as RORγt+. For unfixed cells Th17 werde identified as CXCR3- and CCR6+. Treg gating: after gating on CD3+ alive, CD4+ were selected, for fixed cells Treg were identified as FoxP3+ CD25+. For unfixed cells Treg were identified as CD127- CD25+. For definition of cut off values Fluorescence minus one (FMO) controls were used as appropriate.

**Immunohistochemistry.** 3µm-thick sections of formalin-fixed, paraffin embedded murine ileum and colon were prepared as "swiss rolls" and were mounted on poly-L-lysine-coated slides, deparaffinized and hydrated through graded alcohols to water. Antigen retrieval was carried out by heating sections for 30 minutes in buffer at 98°C, (respectively with 0.01 M sodium citrate buffer, pH 6.0, for the staining of CD3 and with 1 mM EDTA, pH 8.0 for the staining of FoxP3). Endogenous peroxidase activity was inhibited with 3% hydrogen peroxidase (DAKO) for 10 minutes and then saturated for 20 minutes with PowerVision IHC/ISH Super Blocking Solution (Leica Biosystems, #PV6122). Without washing, the rabbit anti-human CD3 polyclonal Ab (ready-to-use, DAKO, # IS503) and rabbit anti- mouse Foxp3 polyclonal Ab (2µg/ml, Invitrogen, #PA1-46126) primary antibodies were applied and incubated for 1 hour, followed by the secondary Ab, PowerVision poly-horse radish peroxidase-conjugated anti-rabbit antibody (Leica Biosystems # PV6119). Peroxidase was detected by means of the Di Amino Benzidine (DAB)-peroxidase substrate kit (DAKO), and the sections were counterstained with Mayer's hematoxylin. The immunohistochemistry staining of CD4 was performed on an automated immunostainer (The BenchMark ULTRA, Ventana, IGR). Heat-induced antigen retrieval in EDTA buffer (pH 8.0) for 32 minutes at 95°C was performed. The primary monoclonal anti-mouse CD4 antibody (1,246µg/mL, #ab183685, Abcam) was diluted 1:500 in antibody diluent (Zytomed), and the slides were incubated for 1hour at 37°C. The biotin-free peroxydase system of detection technique with DAB as chromogen was applied (kit ultraView Universal DAB Detection kit, Ventana). The slides were also counterstained by means of a hematoxylin kit (Ventana).

**Kaede experiments.** The Kaede mice were a kind gift from Michio Tomura, Kyoto University, Kyoto, Japan and were backcrossed and maintained on the C57BL/6 background. Kaede transgenic mice were anesthetized with 2 to 2.5% isoflurane and administered buprenorphine (0.01 mg/kg) i.p. for analgesia. For photoconversion of ilea abdominal skin and peritoneum were cut at the midline to access the intraperitoneal terminal ileum. For photoconversion of the colon, ileum or ileum including the mesenteric lymph nodes, the cecal pole was identified and the cecal pole including the terminal ileum, the mesenteric lymph nodes and the proximal colon was gently mobilized through the midabdominal incision onto a sterile plastic coated surgical drape. Non target structures were covered with aluminium foil. The ventral and dorsal parts of the target structures were exposed to violet light emitted from a 395nm wave length emitting Diode (Winzwon) light for 30 seconds each. After illumination the tissue was moistened with sterile isotonic sodium chloride and gently repositioned into the peritoneal cavity. The peritoneal membrane was closed by continuous stitch with a 5-0 monofilic nylon suture (Ethicon). The skin was closed with two 9mm wound clips (EZ Clip Kit).

**Tracing migration of leukocytes from mesenteric and tumor draining lymph nodes using CFSE.** C57BI6 mice were anesthetized with 2 to 2.5% isoflurane and administered buprenorphine (0.01 mg/kg) i.p. for analgesia. Abdominal skin and peritoneum were cut at the midline to access the mesenteric lymph nodes. The mesenteric lymph nodes were gently mobilized through the midabdominal incision onto a sterile plastic coated surgical drape. Ileum draining mesenteric lymph nodes were visually identified according to their vasculature. The two most prominent mesenteric lymph nodes were injected with 100µM CFSE diluted in 5µl PBS using a 30G insulin syringe. After reposition of the mesenteric lymph node the peritoneal membrane was closed by continuous stitch with a 5-0 monofilic nylon suture (Ethicon). In the case of tumor draining lymph nodes the abdominal skin was incised in the midline sparing the peritoneum. The tumor draining lymph nodes were visualized by gentle separation of the abdominal skin and the peritoneum using scissors. The tumor draining lymph nodes were injected with 100µM CFSE diluted in 5µl PBS using a 30G insulin syringe. The skin was closed with two 9mm wound clips (EZ Clip Kit).

**RNA extraction and rtPCR.** Lysis and extraction protocols were identical for human and mouse samples. Tumor or intestinal samples were snap frozen in liquid nitrogen in RLT Plus buffer containing 0.1% beta mercaptoethanol. On the day of extraction samples were thawed at 4°C and homogenized on a microtube homogenizer (Benchmark Scientific) in RNA free glass bead tubes (Dutscher). Total RNA extraction and genomic DNA removal were performed with the RNeasy Mini kit (Qiagen), following the manufacturer's recommendations. A maximum of 1 µg of RNA, measured by using a NanoDrop^{™} Spectrophotometer (Thermo Fischer Scientific), was reverse transcribed into cDNA with a mix composed of SuperScript III Reverse Transcriptase (Life Technologies), RNaseOUT^{™} Recombinant Ribonuclease Inhibitor (Life Technologies), Random primers (Promega) and Deoxynucleoside Triphosphate Set, PCR grade (Roche Diagnostics).

**Single cell RNA sequencing by Rhapsody.** After isolation of CFSE+ CD4+ T cell by flow cytomatry, cells were washed in cold PBS 10.000 cells and loaded onto a BD Rhapsody^{™} cartridge and processed according to manufacturer's instructions for targeted single-cell RNA-seq using the predesigned Immune Response Panel (Mouse). The library was clustered at 1.75pM on a NextSeq500 system (Illumina) to generate ~40.000 paired end (2*75bp) reads per cell using High Output v2 chemistry. Sequenced single-cell data was demultiplexed using bcl2fastq2 v2.20.

**Quantitative gene expression assay.** Expression of B2M, FoxP3, IFNγ, IL-10, IL-17, MadCAM, Ppia, RORc, TNFα (all from Life Technologies) was analyzed with the TaqMan^{®} Gene Expression Assay using the Universal Master Mix II on a StepOnePlus^{™} Real-Time PCR System (Life Technologies). Amplifications were carried out using the following ramping profile: 1 cycle at 95 °C for 10 min, followed by 45 cycles of 95 °C for 30 s, 60 °C for 1 min. Quantitative RT-PCR data were normalized to the expression levels of the housekeeping genes β2M or Ppia, as indicated in each figure, by means of the 2-ΔCt method multiplied by 10⁶.

**Tissue lysis and chemokine analysis.** Intestinal and tumoral samples were snap frozen in liquid nitrogen in a non-denaturing cell lysis buffer containing 50 mM Tris HCL pH 7.4, 150 mM NaCL, 300 mM Sucrose, 10 mM EDTA and 0.1% Triton 100X. For subsequent lysis samples were thawed at 4°C and lysed on a tube homogenizer (Precellys) in ceramic beads lysis tubes (Precellys). Tissue homogenate was centrifuged at 4000g for 5 min. The supernatant was used for subsequent analysis. Chemokine concentrations in the tissue lysate were determined according to manufacturer recommendation using CCL2, CCL3, CCL4, CCL5, CCL25 and MadCAM Duoset ELISA kits (RnD) or using Legendplex Mouse proinflammatory chemokine panel (Biolegend) with cytometric analysis performed on a CytoFLEX S (Beckmann coulter).

**Statistics. Mice.** Data analyses were performed either with the statistical environment R (http://www.R-project.org/) or Prism 6 (GraphPad, San Diego, CA, USA). Tumor size differences were calculated using non-parametric t-test. All reported tests are two-tailed and were considered significant at p<0.05. **Patients.** *MADCAM-1* as *a predictor of response to PD1 blockade in NSCLC patients.* The prognostic role of sMadCAM1 level was studied using restricted cubic splines (RCS) with 4 knots. The number of knots was chosen according to the Akaike Information Criterion. We tested for non-linearity in the biomarker effect. When linearity hypothesis was not rejected, the linear coding was used to estimate the effect of the biomarker.

### Results

### 1.1. ATB downregulate MAdCAM-1 ileal expression in mice and patients

We reported that gut sterilization of mice with a broad-spectrum ATB cocktail (Ampicillin, Colistin and Streptomycin) blunted the anticancer efficacy of PD-1 blockade (Routy et al., 2018a). We first analyzed ATB-induced fluctuations of the expression of chemokines and integrin ligands in the intestines of MCA205 tumor-bearing mice treated with anti-PD-1 mAb. ATB induced a significant loss of expression of most ileal chemokines and *Madcam1* gene products (**Figure 5A****, left and right panels,** **Figure 1A** **left panel**) while failing to affect colonic and tumoral chemokine and *Madcam1* gene patterns (**Figure 1A** **right panel,** **Figure 5A** **right panel**). The decrease in ileal *Madcam1* gene expression was not only monitored at the transcription but also at the protein level, as measured in immunohistochemistry of ileal tissues (**Figure 1B**), by flow cytometry of ileal CD45⁻ LP cells **(****Figure 1C**), and in immunoenzymatic assays in ileal lysates (**Figure 1D**). Of note, the integrity of the intestinal architecture and vasculature were preserved under administration of ATB, as shown by the stabilization of CD31⁺ vessel numbers (**Figure 5B**). The decrease in *Madcam1* gene expression started by day 3 of ATB administration and was not restored by day 12 post-ATB cessation during the spontaneous recolonization (RECO) when ATB were taken for 7 to 14 days (**Figure 1D**). Apart from broad spectrum ATB, other ATB regimen downregulated *Madcam1* gene expression such as streptomycin, not only in ileal LP but also in mesenteric lymph nodes (mLN) where *Madcam1* gene expression was higher than in LP (**Figure 5C****, left and right panels**) as well as in Peyers' patches (PP) (**Figure 5D**). In contrast to ampicilline or erythromycin, vancomycine failed to downregulate mLN *Madcam1* expression, even increasing *Madcam1* transcription in ileal LP (**Figure 5C****, right and left panel** respectively). Of note, *Madcam1* (but not *Vcam1*) gene expression levels were 10 times lower in tumor draining LN than in mLN (**Figure 5E**). Intrigued by the potent inhibitory effects of ATB on GALT *Madcam1,* we undertook cultivation under aerobic and anaerobic conditions of ileal contents of animals treated with various ATB regimen. Mass spectrometry identification of the ileal bacteria colonies revealed several species (spp.) belonging to the *Enterocloster* gen.nov. clade of bacteria (such as *Enterocloster clostridioformis* comb. nov, *Enterocloster boltae* comb. nov (Haas and Blanchard, 2020)) that prevailed 4 days after ATB cessation but not in the other experimental conditions (**Figure 5F**). Those *Enterocloster spp.* were previously identified in stools of kidney and lung cancer patients resistant to PD1 blockade (Derosa et al., 2020), (Derosa et al. 2022). While vancomycin (which kills *Enterocloster spp.*) upregulated ileal *Madcam1* gene transcription (**Figure 5C****, left panel**), oral supplementation with *Enterocloster clostridioformis* annihilated this effect, and further affected the addressin expression (**Figure 1E**). Similarly, oral gavage with *Enterocloster clostridioformis* after ATB stop during the spontaneous recolonization phase aggravated the loss of *Madcam1* expression (**Figure 1F**). In contrast, oral gavages with immunogenic commensals such as *Akkermansia muciniphila* (Routy et al., 2018a), (Derosa et al. 2022) or *Enterococcus hirae* (Daillère et al., 2016; Goubet et al., 2021) could further increase basal *Madcam1* expression in ileal tissues from eubiotic mice reared in specific pathogen free (SPF) conditions (**Figure 1G**).

Fecal microbial transfer (FMT) of stools from melanoma patients who benefited from PD1 blockade could circumvent primary resistance to ICI in one third of metastatic melanoma recipients (Baruch et al., 2021; Davar et al., 2021). Indeed, distinct donor FMT failed to transfer clinical benefit to recipients (**Table 1**). Hence, we tested whether random FMT from lung or kidney cancer patients into avatar mice (as previously reported, (Routy et al., 2018a, 2018b) treated by ATB could downregulate ileal *Madcam1* gene expression in recipient mice reared in SPF conditions. Three out of 6 FMT did so (**Figure 1H****, left panel**), corresponding to distinct taxonomic composition, defined by the overrepresentation of *Enterocloster spp.* including *E. clostridioformis* (or the ATB-associated *Hungatella hathewayi* (Derosa et al., 2020), sharing the same clade as *Enterocloster spp* (Haas and Blanchard, 2020) in shot gun metagenomics-based analyses (**Figure 1H****, right panel**).

Oral supplementation of FMT recipients with various commensals, including the pro-inflammatory *Atopobium parvulum, L. reuteri* but not *H. hathewayi* could further influence *Madcam1* ileal gene expression (**Figure 5G**).

ATB-induced *Madcam1* gene downregulation in the ileum paralleled and correlated with that of regulatory cytokines and transcription factors (such as *Il17a*, *Il22, Foxp3, RORc*) (**Figure 5A** **left panel,** **Figure 5H-I**). These PCR results were supported by flow cytometric analyses showing that ATB induced a significant loss of mucosal CD25⁺FoxP3⁺ CD4⁺ T cells (Treg) and RORγt⁺ CD4⁺ (TH17) T cell populations in the ileal LP (**Figure 11**). In fact, ATB phenocopied the ileal immunomodulatory effects of Madcam1 gene deficiency or antibody neutralization of MADCAM1 in the ileal LP (**Figure 1J**).

As shown in mice, we confirmed the coordinated inhibitory effects of ATB on the intestinal expression of *Madcam1, Foxp3* and *Rorc* in 16 patients who took ATB and underwent intestinal endoscopy and biopsies for various indications (**Figure 1K****,** **Figure 5J****, Table 2**).

**Table 2. Description of patients who took ATB before endoscopy**

| **Category** | **Age** | **Gender** | **Primary diagnosis** | **Secondary diagnosis** | **Antibiotic characteristics** | **Macroscopic appearance of the mucosa** | **Ileum** | **Cecum** | **Colon** |
|---|---|---|---|---|---|---|---|---|---|
| No ATB | 20 | Female | Irritable Bowel Syndrome | none | - | normal | - | + | + |
| No ATB | 32 | Female | Colorectal cancer screening | none | - | normal | + | + | + |
| No ATB | 64 | Female | Polypectomy | none | - | colon adenoma | + | + | + |
| No ATB | 78 | Female | Colorectal cancer screening | COPD, diabetes mellitus, cardiovascular disease | - | normal | - | + | + |
| No ATB | 79 | Female | Sigmoid diverticula | arterial hypertension | - | normal | + | + | + |
| No ATB | 37 | Male | Polypectomy | none | - | normal | + | + | + |
| No ATB | 48 | Male | Colorectal cancer screening | none | - | normal | + | + | - |
| No ATB | 71 | Male | Elective polypectomy | arterial hypertension | - | normal | + | + | + |
| No ATB | 71 | Male | Polypectomy | none | - | colon adenoma | + | - | + |
| ATB | 36 | Female | Diarrhea | none | Cefazolin plus metronidazole; for 6 days, stopped 2 weeks prior | normal | + | + | + |
| ATB | 46 | Female | Colorectal carcinoma with hepatic metastases | none | Norfloxacin; *unknown duration* | colon carcinoma | - | + | + |
| ATB | 64 | Female | Cholangiocarcinoma | Esophageal varices | Ceftriaxone plus metronidazole; since 7 days | slightly edematous colon | + | + | + |
| ATB | 68 | Female | Cancer of unknown primary with hepatic and pulmonary metastases | none | Tazobactam; for 4 days | normal | - | + | + |
| ATB | 81 | Female | Control after endoscopic mucosa resection | Coronary artery disease, psoriasis | Cefuroxime plus metronidazole; 2 months prior | normal | + | + | + |
| ATB | 67 | Male | Colorectal cancer screening | Kidney transplantation | Teicoplanin plus tazobactam; *unknown duration* | normal | - | + | + |
| ATB | 76 | Male | Colorectal carcinoma with hepatic metastases | Psoriasis | Ceftriaxone; for 3 days | colon carcinoma | + | + | + |

Altogether, broad-spectrum ATB regimen induced a sustained downregulation of the ileal mucosal addressin MADCAM1 in mice and humans, that markedly correlated with reduced ileal *Foxp3, Il17a and RORc* expression.

### 1.2. ATB-induced exodus of enterotropic α4β7⁺ CD4⁺ T cells from the gut to tumor draining lymph nodes

We hypothesized that the loss of MADCAM-1 molecules could affect the homing or the retention of enterotropic T cells expressing its α4β7 receptor in the GALT. To track the migration of ileal T cells in tumor-bearing hosts, we utilized two complementary experimental strategies. First, we used a transgenic Kaede mouse model that allows to irreversibly label cells without disrupting their cellular integrity. This model makes use of a reporter mouse ubiquitously expressing the coral derived, photoconvertible, fluorescent protein Kaede (Tomura et al., 2008), which can be photoconverted from green to red after exposure to ultraviolet light with a wavelength of 350-400nm. Kaede mice represent a powerful tool for tracking migratory intestinal T_{H}17 cells to inflamed peripheral organs (Krebs et al., 2016; Magnuson et al., 2015; Morton et al., 2014). To track the fate of GALT cells in MCA205 tumor bearers, ileum, caecum and mesenteric LN were photoconverted at day 10 (D10) after subcutaneous tumor implantation and mice were killed 24 hours later for detection of photoconverted cells in various organs by flow cytometry (**Figure 2A****,** **Figure 6A**). We achieved a photoconversion rate of approximatively 60% in the mesenteric LN, five minutes after illumination (**Figure 6A**). At 24 hours, only 22.8±2.6% of intestine-photoconverted (PC) leukocytes remained in the mLN but a substantial percentage of GALT-egressing photoconverted (PC) cells could be detected at distant sites, in spleen (5.1±0.5%) and tumor draining lymph nodes (tdLN) (4.0±0.3%) (**Figure 2A****,** **Figure 6A-B**). Using a second approach, we directly traced carboxyfluorescein succinimidyl ester (CFSE) labeled cells after direct CFSE labeling of mLN by a surgical procedure (Singh et al., 2016) (**Figure 2B****,** **Figure 6C****, left panel**). Up to 1.0±0.2% splenocytes, 0.8±0.1% tdLN cells and 0.2±0.02% of tumor residing leukocytes were replaced with mLN-derived leukocytes within 24 hours after CFSE injection (**Figure 6C****, right panel).** Ileal illumination was less proficient than the GALT targeted one in mobilizing the exodus of gut leukocytes to tdLN or sc tumor (**Figure 6D**). However, both the local and GALT illumination induced the selective mobilization of the α4β7⁺ fraction of the photoconverted (PC) (gut derived)-CD4⁺T cells to the spleen, tdLN and sc MCA205 at 24 hours while the other PC cell subsets were not enriched compared with tissue resident (non PC) cells (**Figure 2A-B and** **Figure 6E**). Again, the vast majority of α4β7⁺ CD4⁺T cells within the tumor microenvironment (TME) were CFSE⁺ after CFSE mLN labeling **(****Figure 2B****,** **Figure 6F**). Confirming the molecular involvement of the α4β7 integrin in the CD4⁺ T cell gut exodus, we showed that photoconversion or CFSE labeling of cells in tdLN (but not contralateral LN) was significantly increased in tumor bearing mice treated with neutralizing anti-MAdCAM-1 Ab (**Figure 2C****, left, middle and right panels**).

To decipher the phenotype of the α4β7⁺ CD4⁺ T cells emigrating from the mesenteric LN, we performed a bulk-RNA sequencing of α4β7^{high} CD4⁺ T cells compared with α4β7⁻ CD4⁺ T cells purified from mLN of tumor bearers. The transcriptional profile of α4β7^{high} CD4⁺ T highlighted-not only their *Itga4* (encoding the α4β7 integrin) gut hallmark - but also a marked increase in gene products associated with Treg functions (such as *Icos, Ctla4, Cd74, Mki67, P2rx7* (Daniel et al., 2010) and T_{H}17 (*Il17re,Tnfsf11, Il22*) polarization, while the *Tnfrsf9* T-cell costimulatory gene product (4-1BB) was significantly downregulated in this subset (**Figure 2D**).

Next, we addressed how ATB-induced dysbiosis affected the exodus of enterotropic α4β7⁺ CD4⁺ T cells to the tumor draining LN using both imaging approaches combined to intracellular flow cytometry (**Figure 2E-F**). Recolonization facilitated the homing of IL-17A secreting α4β7⁺ Foxp3⁺CD4⁺ T cells (gut derived Tr17) but not of α4β7⁻ Foxp3⁺CD4⁺ T cells (tdLN resident- Tr17) nor IL-17A⁺ α4β7⁺ Foxp3⁻CD4⁺ T cells to tdLN (**Figure 2E****,** **Figure 6G**). ATB phenocopied the *madcam1* gene deficiency (**Figure 2F**). Actually, ATB bearly increased the bona fide Treg pool of the tdLN constituted by and large by the locally expanded (extraintestinal, CFSE negative) cells (**Figure 6H****, left panel**). The α4β7⁺ CFSE⁺ gut emigrating fraction of Tr17 represented 0.2±0.1% of the total CD4⁺ in the reached tdLN per 24 hours during the recolonization post-ATB (**Figure 6H****, middle panel**). Of note, the enterotropic Tr17 that emigrated during the recolonization produced not only IL-17 but also IL-22 (**Figure 6H****, right panel**).

Neutralizing anti-MADCAM-1 antibodies also promoted the emigration of CD25^{high} α4β7⁺ CD4⁺ T (**Figure 2G**). Recolonization post-ATB course was accompanied by the emergence of *Enterocloster spp* (Figure 5F) that we attempted to mimick by enforced oral gavage with *E. clostridioformis.* Indeed, this bacterium facilitated the gut exit of PC (but not non PC) TH17 CD25^{high} (and not CD25⁻) α4β7⁺ Tr17-like CD4⁺T cells towards tdLN (**Figure 2H**).

Because photoconversion does not allow precise intracellular staining and high dimensional phenotyping, we dwelled into a deeper characterization of the gut emigrating cells performing Rhapsody-based single cell RNA sequencing of CFSE⁺ CD4⁺ T cells reaching the tdLN at 24hrs post-CFSE injection of the mLN at day 4 after a 14 days ATB course (or no ATB) supplemented or not with *E. clostridioformis* (**Figure 7A-B**). We profiled a total of 451,246 individual CFSE⁺ CD4⁺ tdLN T cells harvested partitioned in each of the four groups respectively (**Figure 7A-B**), and characterized the gene signatures associated with ATB induced- dysbiosis. Unsupervised clustering of the CFSE⁺ CD4⁺ T cells partitioned the data into 4 cellular clusters (**Figure 2H****, left panel**), which we visualized using t-stochastic neighborhood embedding and labeled post hoc by the expression of reported marker genes (Cano-Gamez et al., 2020). Each cluster was associated with a distinct phenotype. A small cluster featured the prototypic pan-tissue effector Treg expression pattern defined by *Foxp3, Nrp1, Il2ra, Tnfsf4, Ctla4,* and intestine-specific gene expression imprinting (*Tnfsf18, Rgs1*) with a downregulation of *Tcf7,* and *Cd52* (Sefik et al., 2015) (**Figure 2H****, middle panel**). This Treg subset overexpressed the negative immune regulator *Pik3ip1* involved in tumor immunosuppression (Chen et al., 2019) and downregulated Fosb, member of the transcription factor AP1 involved in CD95L -mediated apoptosis of CD4⁺ T cells (Baumann et al., 2003) during the recolonization phase compared with the steady state (**Figure 7C**), also contrasting with all the other emigrating cells by the overexpression of genes associated with the Tr17 blueprint (Sefik et al., 2015) (*Iksf2* (also called helios), and *Lrrc32* (also called TGFβ activator GARP) during the recolonization phase or the oral gavage with *E. clostridioformis* (**Figure 7D** **right and left panel**). In contrast, another cluster with proliferative hallmarks (*Pcna, Myc, Mapk1, Fyn, Hif1a*) was dominated by *Irf8,* as well as *Pou2af1* (also called OBF1, which acts as a transcriptional co-activator of the transcription factors OCT1 or OCT2) and *Fas*, both promoting the TH17 program by suppressing IL-2 or IFNγ expression respectively (Yosef et al., 2013) (**Figure 2H****, right panel**). This proliferative TH17 subset also shared prototypic markers with pan-tissue Treg (*Il2rb, Ctla4, Icos),* intestinal Treg (such as *Bcl2a1a, Bcl6*) and skin Treg (*Lgals3*)*.* During ATB stop-induced recolonization and supplementation with *E. clostridioformis, caspase 1* gene that was described as a T cell-intrinsic gene, independent of inflammasome, indispensable for optimal priming of pathogen-specific Th17 responses (Gao et al., 2020) was upregulated, in conjunction with TNFRSF and cytokine/chemokine transduction and NFkb activation pathways *(Nfkb1, Cxcr3, Cxcr5, Eomes, Tnfrsf8, Tnfrsf9, Tnf, Fasl, Fas, Tigit, Icam1, Runx3, Jak2*) (**Figure 7E****, Table 3**). Two remaining and dominant cell clusters shared the expression of the prototypic *Il7r* and *Pik3ip1* gene hallmarks of gut inflammatory activation and/or suppression (Belarif et al., 2019; Chen et al., 2019). They slightly differ for other fingerprints such as *Egr1, Foxo1, Stat6, Ikbkb* transcription factors versus *Btla, Cnot2* and *Dusp2* during recolonization (**Figure 7F-G**), yet all converging towards tumor immunosuppressive properties (Dan Lu et al., 2020; Kim et al., 2019; Li et al., 2012).

Hence, we demonstrated the exit of enterotropic α4β7⁺ CD4⁺, most specifically Tᵣ17 cells exhibiting ilmmunosuppressive fingerprints, potentially harmful on the tumor immunosurveillance, from the GALT to tumor draining lymph nodes by two independent tracing methodologies, in conditions where the MAdCAM-1/α4β7 axis was compromised, either by anti-MAdCAM-1 mAb or by ATB-induced recolonization.

**Table 3. Single cell RNA sequencing of tdLN-derived CFSE+ T cells.**

| **Water: Proliferating T cells** | | | | **RECO: Proliferating T cells** | | | | **RECO + *E.clostridioformis:* Proliferating T cells** | | | |
|---|---|---|---|---|---|---|---|---|---|---|---|
| genes | avglog2FC | pvalue (adi) | UP or DOWN | genes | avglog2FC | pvalue (adi) | UP or DOWN | genes | avglog2FC | pvalue (adi) | UP or DOWN |
| **Tnfrsf9** | 5,6 | 6,6E-83 | UP | **X2810417H13Rik** | 5,2 | 1,4E-19 | UP | **Tnfrsf9** | 5,8 | 3,3E-255 | UP |
| **X2810417H13Rik** | 5,3 | 6,8E-23 | UP | **Tnfrsf9** | 5,0 | 1,0E-57 | UP | **Pou2af1** | 3,9 | 4,5E-35 | UP |
| **Eomes** | 5,0 | 2,7E-19 | UP | **Ybx3** | 4,0 | 8,6E-89 | UP | **Nkg7** | 3,7 | 5,9E-57 | UP |
| **Cxcr3** | 4,3 | 1,6E-35 | UP | **Cxcr3** | 3,9 | 1,2E-43 | UP | **Bcl2a1a** | 3,2 | 4,4E-34 | UP |
| **Cd160** | 3,6 | 7,0E-21 | UP | **Eomes** | 3,9 | 2,1E-12 | UP | **Ybx3** | 2,9 | 1,2E-29 | UP |
| **Tnfrsf4** | 3,6 | 1,5E-26 | UP | **Pou2af1** | 3,9 | 7,1E-33 | UP | **Tnfrsf4** | 2,8 | 8,8E-23 | UP |
| **Ybx3** | 3,4 | 1,9E-30 | UP | **Il21** | 3,7 | 3,9E-41 | UP | **Tmem97** | 2,7 | 2,9E-15 | UP |
| **Irf8** | 3,1 | 1,7E-16 | UP | **Irf8** | 3,7 | 1,7E-18 | UP | **Casp1** | 2,4 | 7,0E-10 | UP |
| **Tmem97** | 3,0 | 6,6E-12 | UP | **Tmem97** | 3,7 | 1,1E-31 | UP | **Il12rb** | 2,1 | 1,2E-27 | UP |
| **Pou2af1** | 2,9 | 7,6E-13 | UP | **Casp1** | 3,6 | 8,4E-28 | UP | **Icos** | 2,0 | 9,4E-08 | UP |
| **Il2rb** | 2,9 | 1,0E-19 | UP | **Tigit** | 3,2 | 2,4E-30 | UP | **Jak2** | 1,9 | 3,5E-12 | UP |
| **Bcl2a1a** | 2,8 | 1,8E-14 | UP | **Top2a** | 3,1 | 9,6E-17 | UP | **Fasl** | 1,9 | 5,1E-06 | UP |
| **Bcl6** | 2,7 | 5,9E-09 | UP | **Nrp1** | 3,1 | 2,4E-14 | UP | **Nfkb1** | 1,9 | 1,6E-09 | UP |
| **Ctla4** | 2,6 | 5,8E-11 | UP | **Cd200** | 2,9 | 1,8E-21 | UP | **Cd274** | 1,7 | 1,5E-04 | UP |
| **Casp1** | 2,2 | 1,0E-11 | UP | **Tnfsf8** | 2,8 | 1,2E-06 | UP | **Lap3** | 1,7 | 1,9E-05 | UP |
| **Tnfrsf1b** | 2,1 | 2,4E-07 | UP | **Cd22** | 2,7 | 3,9E-41 | UP | **Cst7** | 1,5 | 1,3E-04 | UP |
| **Lgals3** | 2,1 | 6,9E-28 | UP | **Cxcr5** | 2,7 | 6,7E-36 | UP | **Runx3** | 1,5 | 9,3E-03 | UP |
| **Lap3** | 2,0 | 7,7E-02 | UP | **Fasl** | 2,6 | 5,8E-07 | UP | **Tnfrsf1b** | 1,4 | 6,7E-03 | UP |
| **Nt5e** | 1,9 | 2,0E-04 | UP | **Tnfrsf4** | 2,6 | 5,3E-14 | UP | **Icam1** | 1,3 | 1,4E-01 | UP |
| **Icos** | 1,8 | 1,6E-03 | UP | **Ctla4** | 2,5 | 9,6E-07 | UP | **Cd5** | 1,3 | 5,0E-11 | UP |
| **Fas** | 1,6 | 1,1E-01 | UP | **Bcl2a1a** | 2,5 | 1,8E-09 | UP | **Tnfrsf18** | 1,3 | 2,1E-05 | UP |
| **Lgals1** | 1,6 | 1,6E-01 | UP | **Tnf** | 2,4 | 6,0E-08 | UP | **Cd28** | 1,3 | 1,1E-07 | UP |
| **Atf6b** | 1,5 | 5,0E-01 | UP | **Cd160** | 2,4 | 1,2E-08 | UP | **Lta** | 1,3 | 9,3E-02 | UP |
| **Cd28** | 1,4 | 9,4E-05 | UP | **Il2rb** | 2,2 | 5,5E-11 | UP | **Stat6** | 1,2 | 3,3E-03 | UP |
| **Tnfrsf18** | 1,3 | 1,2E-04 | UP | **Tyms** | 2,1 | 6,6E-06 | UP | **Hif1a** | 1,1 | 4,0E-05 | UP |
| **Tnfsf8** | 1,2 | 1,0E+00 | UP | **Nfkb1** | 2,0 | 5,6E-06 | UP | **Lamp3** | 1,1 | 3,1E-03 | UP |
| **Cd274** | 1,2 | 2,2E-02 | UP | **Fas** | 1,9 | 1,5E-05 | UP | **Pdia4** | 1,0 | 9,9E-01 | UP |
| **Cd5** | 1,2 | 6,2E-02 | UP | **Lap3** | 1,9 | 1,2E-09 | UP | **Birc3** | 1,0 | 3,6E-02 | UP |
| **Nfkb1** | 1,2 | 1,3E-03 | UP | **Cst7** | 1,7 | 2,0E-03 | UP | **Cblb** | 1,0 | 4,6E-01 | UP |
| **Xbp1** | 1,2 | 1,0E+00 | UP | **Icam1** | 1,7 | 1,5E-03 | UP | **Stat5a** | 1,0 | 2,5E-04 | UP |
| **Icam1** | 1,2 | 4,3E-02 | UP | **Gimap7** | 1,6 | 4,6E-01 | UP | **Bax** | 0,9 | 3,0E-05 | UP |
| **Cst7** | 1,1 | 3,1E-01 | UP | **Lamp3** | 1,6 | 1,0E+00 | UP | **Gapdh** | 0,9 | 2,0E-04 | UP |
| **Hif1a** | 1,1 | 5,0E-03 | UP | **Hif1a** | 1,4 | 6,3E-04 | UP | **Ccnd2** | 0,9 | 1,0E+00 | UP |
| **Gapdh** | 1,0 | 8,2E-05 | UP | **Tnfrsf18** | 1,4 | 4,1E-03 | UP | **Xbp1** | 0,9 | 7,0E-05 | UP |
| **Cd44** | 1,0 | 1,2E-04 | UP | **Kdelr1** | 1,3 | 4,3E-04 | UP | **Dusp2** | 0,8 | 1,8E-06 | UP |
| **Hprt** | 1,0 | 5,5E-03 | UP | **Jak2** | 1,3 | 3,3E-03 | UP | **Mbp** | 0,8 | 8,1E-02 | UP |
| **Stat5a** | 0,9 | 1,3E-02 | UP | **Tfrc** | 1,3 | 2,3E-02 | UP | **Myc** | 0,8 | 1,0E+00 | UP |
| **Stat6** | 0,9 | 7,9E-04 | UP | **Icos** | 1,2 | 6,0E-01 | UP | **Ctla4** | 0,8 | 3,3E-02 | UP |
| **Dusp2** | 0,9 | 5,0E-04 | UP | **Stat6** | 1,2 | 8,9E-02 | UP | **Btla** | 0,7 | 1,0E+00 | UP |
| **Zap70** | 0,8 | 7,6E-03 | UP | **Cd6** | 1,2 | 8,4E-01 | UP | **Cd44** | 0,7 | 2,3E-01 | UP |
| **Fyn** | 0,8 | 7,8E-02 | UP | **Stat5a** | 1,1 | 2,7E-03 | UP | **Zap70** | 0,7 | 6,5E-02 | UP |
| **Tfrc** | 0,7 | 1,4E-01 | UP | **Ada** | 1,0 | 2,8E-05 | UP | **Cd69** | 0,7 | 9,9E-04 | UP |
| **Tnfrsf13b** | 0,6 | 6,9E-01 | UP | **Pdia4** | 1,0 | 3,7E-01 | UP | **Fyn** | 0,7 | 3,2E-01 | UP |
| **Myd88** | 0,6 | 1,1E-01 | UP | **Bax** | 1,0 | 1,0E+00 | UP | **Mapk8** | 0,7 | 5,9E-02 | UP |
| **Ptprc** | 0,6 | 1,0E+00 | UP | **Fyn** | 1,0 | 5,5E-01 | UP | **Cd6** | 0,6 | 1,8E-01 | UP |
| **Il3ra** | 0,6 | 1,0E+00 | UP | **Gapdh** | 1,0 | 9,2E-01 | UP | **SIc25a37** | 0,6 | 8,7E-01 | UP |
| **Mbp** | 0,6 | 1,0E+00 | UP | **Dpp4** | 0,9 | 1,0E+00 | UP | **Ptprc** | 0,6 | 2,8E-06 | UP |
| **Lta** | 0,4 | 7,6E-01 | UP | **Mcm2** | 0,9 | 3,6E-02 | UP | **Hprt** | 0,6 | 4,2E-01 | UP |
| **Cnot2** | 0,4 | 1,0E+00 | UP | **Cd5** | 0,8 | 1,0E+00 | UP | **Pcna** | 0,5 | 3,0E-02 | UP |
| **H2.K1** | -0,3 | 3,2E-01 | DOWN | **Vps28** | 0,8 | 7,1E-01 | UP | **Cd27** | 0,4 | 1,0E+00 | UP |
| **Btg1** | -0,4 | 1,0E+00 | DOWN | **Btla** | 0,8 | 1,0E+00 | UP | **Stat4** | 0,3 | 1,0E+00 | UP |
| **Cd3d** | -0,5 | 1,0E+00 | DOWN | **Hprt** | 0,7 | 3,5E-01 | UP | **Fas** | 0,3 | 1,0E+00 | UP |
| **Thy1** | -0,5 | 1,0E+00 | DOWN | **Xbp1** | 0,7 | 7,3E-02 | UP | **Trac** | 0,3 | 1,0E+00 | UP |
| **Trbc2** | -0,6 | 1,0E+00 | DOWN | **Ccnd2** | 0,6 | 1,0E+00 | UP | **Btg1** | -0,3 | 1,0E+00 | DOWN |
| **Ccr7** | -0,6 | 1,0E+00 | DOWN | **Mapk1** | 0,6 | 2,9E-01 | UP | **Lat** | -0,3 | 9,0E-01 | DOWN |
| **Fth1** | -0,6 | 7,7E-02 | DOWN | **Zap70** | 0,6 | 8,9E-01 | UP | **Cd52** | -0,3 | 7,0E-02 | DOWN |
| **Sell** | -0,9 | 1,0E+00 | DOWN | **Psen1** | 0,5 | 1,0E+00 | UP | **Fth1** | -0,4 | 7,6E-03 | DOWN |
| **Lef1** | -1,7 | 2,0E-01 | DOWN | **Cd28** | 0,5 | 1,0E+00 | UP | **Ccr7** | -0,5 | 9,3E-01 | DOWN |
| **Arl4c** | -2,3 | 4,7E-02 | DOWN | **Nt5e** | 0,5 | 6,7E-01 | UP | **Tcf7** | -0,5 | 3,6E-02 | DOWN |
| **Il7r** | -3,5 | 7,4E-02 | DOWN | **Rpn2** | 0,5 | 1,0E+00 | UP | **Thy1** | -0,5 | 7,2E-03 | DOWN |
| | | | | **Mapk8** | 0,5 | 1,0E+00 | UP | **Lef1** | -1,0 | 1,7E-02 | DOWN |
| | | | | **Ptprc** | 0,3 | 1,0E+00 | UP | **Selplg** | -1,1 | 5,6E-01 | DOWN |
| | | | | **Dusp2** | 0,3 | 1,0E+00 | UP | **Sell** | -1,1 | 6,4E-04 | DOWN |
| | | | | **H2.K1** | -0,3 | 6,6E-01 | DOWN | **Trib2** | -1,3 | 1,0E+00 | DOWN |
| | | | | **Cd44** | -0,4 | 1,0E+00 | DOWN | **Trat1** | -1,5 | 1,0E+00 | DOWN |
| | | | | **Btg1** | -0,4 | 1,0E+00 | DOWN | **Il7r** | -1,5 | 2,9E-04 | DOWN |
| | | | | **Sell** | -0,7 | 1,0E+00 | DOWN | **Arl4c** | -1,5 | 1,1E-03 | DOWN |
| | | | | **Lef1** | -1,3 | 1,0E+00 | DOWN | **Cd1d1** | -1,5 | 1,0E+00 | DOWN |
| | | | | **Il7r** | -2,7 | 8,9E-02 | DOWN | **Pik3ip1** | -1,8 | 8,7E-03 | DOWN |
| | | | | **Arl4c** | -2,9 | 7,3E-02 | DOWN | | | | |

### 1.3. The anticancer efficacy of PD1 blockade relies on the MAdCAM-1/ α4β7 axis

Given the reported immunosuppressive role of Tr17 cells during cancer immuno-surveillance (Blatner et al., 2012; Rizzo et al., 2018; Voigt et al., 2017) and the capacity of a healthy gut to retain these T cell subsets, we anticipated that genetic defects in MADCAM-1 addressin or β7 integrins or host neutralization of MADCAM-1 or α4β7 heterodimers by specific antibodies could interfere in the immuno-stimulatory capacity of therapeutic anti-PD-1 mAbs.

First, loss of ileal *Madcam1* expression correlated with increased tumor size in MCA205 tumor bearing animals regardless of PD1 blockade (**Figure 8A**). Next, we observed a markedly reduced anti-cancer efficacy of PD-1 inhibition in *Itgb7*^{*-*/*-*} or *Madcam1*^{*-*/-} mice compared with wild type animals bearing transplantable MCA205 fibrosarcoma syngeneic from C57BL/6 mice (**Figure 3A**). Similarly, MCA205 fibrosarcoma, the orthotopic TC1 lung cancer and the 4T1 breast cancer syngeneic from BALB/c mice did grow in vivo despite PD1 blockade in the presence of neutralizing anti-MAdCAM-1 or anti-α4β7 heterodimeric antibodies while responding to the ICI when the MADCAM1/ α4β7 axis was unaffected (**Figure 3B-D**). In *Madcam1*^{*-*/*-*} mice, there was a constitutive increase of the recirculation of α4β7⁺ CD4⁺ T cells in the spleen (**Figure 3E****, left panel**) and the tumor where they represented up to 3% of TILs (**Figure 3E****, right panel**), as already described (Denning et al., 2005)). Flow cytometric analyses revealed that blockade of the MAdCAM-1 addressin/ α4β7 integrin axis using a neutralizing anti-MADCAM-1 Ab during spontaneous tumor progression reshaped the TME and led to about a 3 fold increase in the intratumoral accumulation of RORγt⁺ Treg (Foxp3⁺CD25⁺) Tᵣ17 cells (**Figure 3F-G**). Up to 76±1.5% among α4β7⁺ CD4⁺ TIL were RORγt⁺ Treg with 2- 3 fold less (17±0.9%) contained within α4β7⁻ CD4⁺ TIL (**Figure 8B**) in MCA205 TME without any manoeuvers. In subcutaneous tumors, Treg represented 12.1±1.0% of α4β7⁺ CD4⁺ TIL and among these Treg, 30.4±4.5% were RORγt⁺. (**Table 4**)

**Table 4. Deconvolution of enterotropic Tr17 subset proprotions invading MCA205 and 4T1 tumors.**

| **Group** | **Water** | | **ACS + 12d (RECO)** | | | **ACS + 12d (RECO) + αPD1** | | | |
|---|---|---|---|---|---|---|---|---|---|
| | | | | | vs Water | | | vs Water | vs ACS + 12d |
| **Population** | N* | Mean±SEM (%) | N* | Mean+SEM (%) | *p-value*** | N* | Mean+SEM (%) | *p-value*** | *p-value*** |
| **α4β7⁺ in CD4** | 45 | 17.0 ± 1.4 | 22 | 25.2 + 2.8 | ***0,0051*** | 15 | 21.2 + 1.9 | *0,0727* | *0,4963* |
| **CD25⁺FoxP3⁺ in α4β7⁺ CD4** | 45 | 12.1 +1.0 | 21 | 11.5 ± 4.1 | *0,8962* | 15 | 18.7 +3.4 | *0,2781* | *0,2781* |
| **RORγt⁺ in α4β7⁺ CD25⁺FoxP3⁺ CD4** | 34 | 30.4 ± 4.5 | 21 | 42.5 ± 5.8 | *0,1803* | 15 | 56.7 ± 8.3 | ***0,0171*** | *0,2078* |
| **IL-17A⁺ in RORγt⁺ α4β7⁺ CD25⁺FoxP3⁺ CD4** | 12 | 39.7 ± 7.2 | 12 | 61.9 + 8.4 | ***0,0478*** | 9 | 70.0 ± 8.6 | ***0,0288*** | *0,5461* |

Recolonization post- 4 days ATB stop phenocopied *madcam-1* gene deficiency inducing a 3-5 fold increase in the proportion of RORγt⁺ Treg (Tr17) in tumor beds (**Figure 3H**). Of note, this Tr17 intratumoral homing was transient and no longer observed by twelve days post-ATB stop, except when anti-PD1 Abs were administered (**Figure 3H-I,** **Figure 8C**). Indeed, anti-PD1 Abs facilitated the priming and/or expansion of Tr17 in the mesenteric LN in MCA205 tumor bearing mice (**Figure 8D**). Anti-PD1 Ab contributed to the maintenance and/or amplification of the intratumoral accumulation of Tr17 defined as Rorγt⁺ Treg or IL-17⁺IL-22⁺ Treg in MCA205 (**Figure 3I****,** **Figure 8E**) as well as 4T1 tumor bearers, (**Figure 8F**, left and right panels). In this context, intratumoral Treg represented 18.7±3.4% of α4β7⁺ CD4⁺ TIL and among these Treg, 56.7±8.3% were RORγt⁺. (**Table 4**)

The Tr17 recruitment promoted by PD1 inhibition was further increased when *E. clostridioformis* was supplemented by oral gavage after ATB stop, while in similar conditions, oral *Lactobacillus reuteri* failed to do so (**Figure 8G****, right panel and left panel**). Not surprisingly, blockade of the MAdCAM-1/α4β7 axis during the anti-PD-1 mAb therapy dramatically impaired the infiltration of effector CCR5⁺ CD8⁺ TILs (**Figure 3J**).

Given that IL-17 and IL-22 have direct and indirect pro-angiogenic and pro-tumorigenic effects (Lim and Savan, 2014; Voigt et al., 2017), neutralizing either one or both cytokines may circumvent the harmful effects of the recolonization post -ATB during PD1 inhibition. We undertook treating mice inoculated with a tumorigenic dose of WT or IL-22Rα1^{-/-} deficient mammary 4T1 tumor cells by ip injections of anti-PD1 Abs in conjunction with anti-IL-17A neutralizing antibodies (**Figure 3K****, left panel**). As expected, IL-22Rα1^{-/-} deficient mammary 4T1 grew slowler than their WT counterparts. Interestingly, anti-IL-17A antibodies could circumvent the deleterious effects of ATB, to a similar extent for both WT or IL-22Rα1^{-/-} deficient 4T1 (**Figure 3K**), suggesting that tumor cell reactivity to IL-22 is not an essential mechanism underlying the immunosuppressive effects of ATB.

Therefore, we conclude that PD1 blockade facilitates the amplification and homing to tumors of TH17/Tr17 in the GALT in the setting of intestinal dysbiosis.

Re-establishing the fitness of the α4β7/MAdCAM-1 axis by external maneuvers may revert resistance to PD-1 blockade in tumor bearers treated with ATB or suffering from overt gut dysbiosis. Restoring gut eubiosis before starting PD1 blockade may represent a reasonable option. Short term ATB followed by fecal microbial transfer from a patient relapsing despite αPD-1 Abs caused ileal *Madcam1* downregulation (Figure 1G) and inefficiency of PD-1 blockade, unless hosts were compensated with oral *Akkermansia muciniphila* ((Routy et al., 2017), Figure 1F, **Figure 9A**). The compensatory effect of exogenous *A. muciniphila* depended upon MAdCAM-1 function since neutralizing antibodies prevented the beneficial effect of this commensal drug (**Figure 9B**). *A. muciniphila* could prevent the gut exodus of α4β7⁺ and CCR9⁺ single or double positive CD4⁺T cells and their recirculation to the TME recirculation to the tdLN (**Figure 9A****, right panels**) as well as of α4β7+ CD4+ IL-17+ IL-22+ and Tr17 (**Figure 9C-D**) in a manner depending on MADCAM-1 (**Figure 9D**).

Hence, restoring intestinal eubiosis or neutralizing IL-17A represent potential options to counteract ATB-induced resistance to PD1 blockade.

### 1.4. Soluble MAdCAM-1 is a predictive biomarker of resistance to PD1 blockade in advanced NSCLC patients

Because intestinal tissue biopsies are difficult to obtain, friendly-using blood assays have been developed such as a sMAdCAM-1 ELISA to study the follow up of patients suffering from IBD and treated with anti-heterodimeric α4β7 antibodies (such as vedolizumab) (Holmer et al., 2020). However, soluble MAdCAM-1 levels have not also been monitored in mice. We found robust correlations between ileal *madcam1* gene expression and serum soluble MAdCAM-1 (**Figure 4A**). Next, we analyzed the clinical significance of the serum soluble MAdCAM-1 at diagnosis in two independent cohorts of advanced non small cell lung cancer (NSCLC) patients treated with anti-PD1/L-1 antibodies with or without chemotherapy in France (N=186) and France and Canada (N=115) (**Table 5**). First, NSCLC patients harbored reduced circulating serum sMAdCAM-1 concentrations in both cohorts compared with healthy volunteers, even more so in those who took ATB (**Figure 4B****, left and right panels**). Indeed, patients who took ATB presented sMAdCAM-1 serum levels in the same range as those ATB- free individuals belonging to the lower tertile of sMAdCAM-1 expression (**Figure 4B**). Patients exhibiting high sMAdCAM-1 serum levels above the median of the whole cohort had a prolonged progression-free and overall survival compared with the other half, in both cohorts (**Figure 4G**). There was a linear relationship between the augmentation of sMAdCAM-1 absolute serum concentrations and the reduction of death risk with a 8-10% risk reduction every +10 U/ml increase of the biomarker for each cohort (**Figure 10A****, right and left panels**). ATB-treated patients exhibited the worst PFS in the MAdCAM-1^{low} subgroup (**Figure 4F**). Moreover, multivariate Cox regression analysis taking into consideration ECOG-PS, age, gender, PD-L1 expression, therapy lines in the model concluded that sMAdCAM-1 is an independent predictor of clinical benefit to PD1 blockade in NSCLC patients (**Table 6**). We extended the clinical relevance of this biomarker beyond PD1 inhibition, as a biomarker predicting early NSCLC incidence in high risk heavy smokers experiencing a cardiovascular event (HRHSCV) (PREVALUNG study, **Table 7**). Indeed, sMAdCAM-1 serum levels were significantly lower in those 13 HRHSCV individuals deemed to develop a lung cancer nodule 6-12 months prior to the diagnosis of NSCLC compared with the 99 paired controls who did not (**Figure 10B**). Soluble MAdCAM-1 levels were anticorrelated with circulating α4β7⁺ Rorγt⁺ CD4⁺ T cells (**Figure 10C**) in the whole cohort.

Lastly, to directly show the relationship between soluble MAdCAM-1 serum levels and gut dysbiosis in advanced NSCLC patients, we performed a non supervised and supervised hierarchical clustering of the metagenomics species (MGS) defining the taxonomic composition of the intestinal microbiota according to the median of sMAdCAM-1 serum levels using shot gun metagenomics (MG) analyses in n=95 patients providing all the requested MG and serum pieces of information. First, there was a reduction in the MGS richness in MAdCAM-1 ^{low} patients (segregated based on the median of all 112 NSCLC patients) (**Figure 4C**). Moreover, this different alpha diversity was accompanied by a significantly different beta-diversity, meaning that the taxonomic gut composition was not similar in both subsets (**Figure 5F**). The non supervised MG analysis revealed two clusters that indeed, also segregated patients based on the MAdCAM-1 median (Fisher's exact method, p<0.05, **Figure 10D-E**).

Finally, selecting only the list of MGS found in mice (Figure 1G right panel) and clinically associated with dismal prognosis in a large series of NSCLC patients (Tsay et al., 2021; Zitvogel and Kroemer, 2021), (Derosa et al. 2022), we conclude and confirm that patients having higher prevalence of intestinal *E. clostridioformis* and *V. parvula* are those presenting low levels of sMAdCAM-1.

Altogether, these findings indicate that soluble MAdCAM-1 is a surrogate marker of intestinal dysbiosis predicting resistance to PD1 blockade in cancer patients.

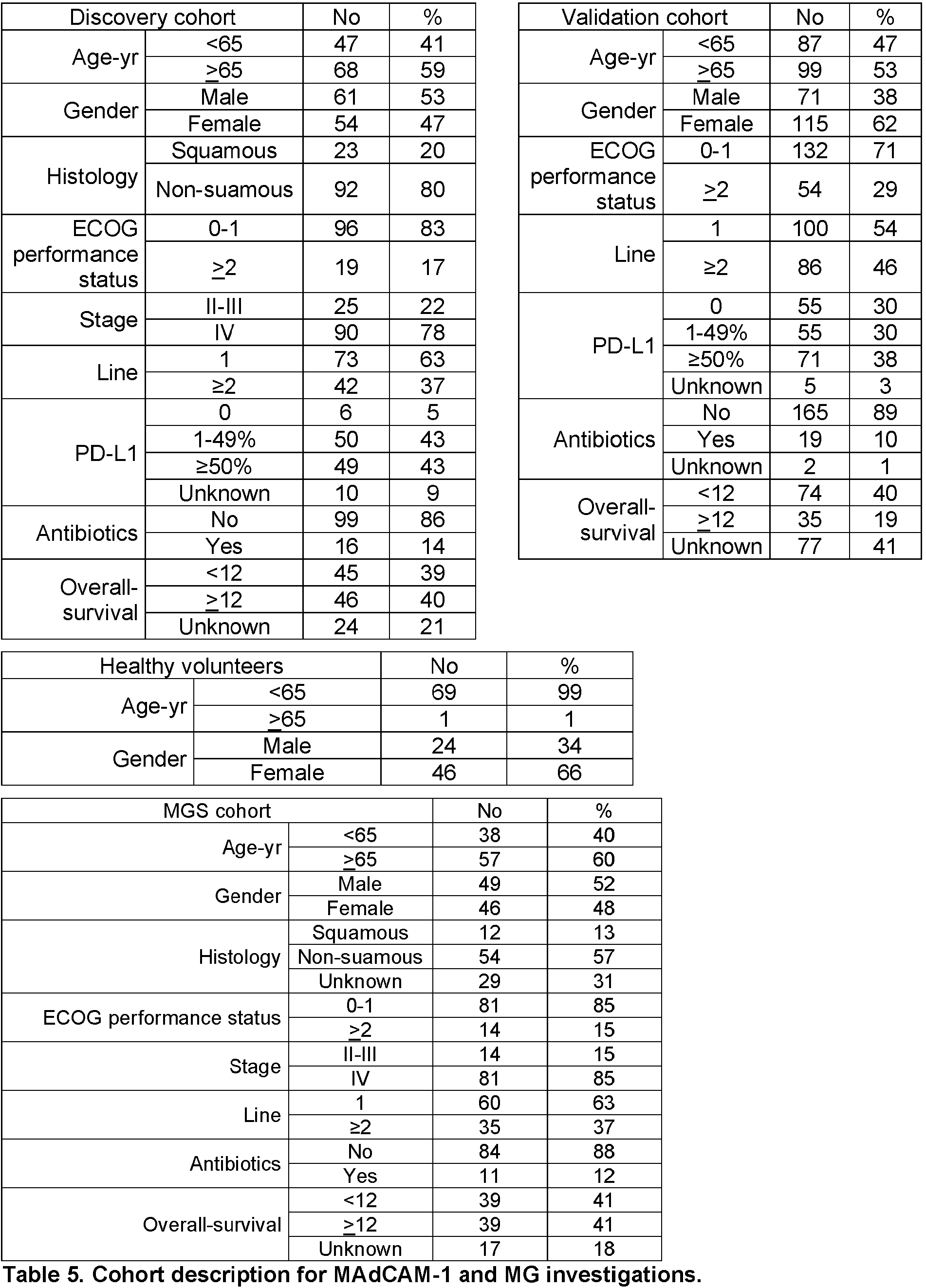

**Table 6. Multivariate analysis investigating the predictive value of sMAdCAM-1 in NSCLC patients.**

| **Overall survival**** | | HR | 95% CI | *p-value**** |
|---|---|---|---|---|
| **sMAdCAM-1** | HR for an increase of 10 units | 0,973 | 0.950-0.996 | ***0,023*** |
| | 0 | 1 | Reference | |
| **PD-L1** | 1-49% | 0,565 | 0.356-0.894 | ***0,015*** |
| | ≥ 50% | 0,448 | 0.279-0.718 | ***0,001*** |
| **Line** | 1 | 0,691 | 0.483-0.987 | ***0,042*** |
| **ECOG performance status** | 0-1 | 1 | Reference | |
| | 2 | 2,709 | 1.684-4.359 | ***<0.001*** |

**Table 7. Clinical characteristics of smokers suffering from cardiovascular disease and followed up for lung cancer incidence (Prevalung study).**

| | | No nodule | | Cancer | |
|---|---|---|---|---|---|
| | | No | % | No | % |
| Age-yr | <65 | 17 | 30 | 1 | 11 |
| | ≥65 | 39 | 70 | 8 | 89 |
| Gender | Male | 9 | 16 | 7 | 78 |
| | Female | 47 | 84 | 2 | 22 |
| Smoking status | Smoker | 12 | 21 | 5 | 56 |
| | Former | 44 | 79 | 4 | 44 |
| | Never | 0 | 0 | 0 | 0 |

### Discussion

Our findings unveil the pivotal role of the ileum in hosts challenged by a tumorigenic process whereby the GALT keeps in check the emigration of gut tropic immunosuppressive T_{H}22/T_{H}17, among which up to 20% are Foxp3⁺CD25⁺ T_{reg} cells. Both the gut and the TME are rich in cell attraction and homing molecules competing for circulating lymphocytes expressing their receptor or ligand pairs. Using tools enabling fate mapping from the GALT to tumor beds, we uncover that the fate decision for enterotropic T cells between gut residency and chemoattraction towards tumor deposits is -at least partly- controlled by the MAdCAM-1 addressin/ α4β7 integrins molecular interactions.

First, ATB downregulated MAdCAM-1 expression in LP ileal venules and HEV of the mucosae and the mLN, favoring the ileal exodus of the α4β7+ Th17/Tr17 cells towards extraintestinal lesions. Secondly, MAdCAM deficiency or neutralization phenocopied the effects of ATB. *Madcam-1* or *β7* integrin gene deficient mice or animals receiving neutralizing Abs targeting these molecules failed to respond to ICI, by accumulating gut derived- α4β7+ Tr17/TH17 cells in the TME. Third, maneuvers aimed at restoring MAdCAM-1 expression on ileal HEV (such as *A. muciniphila*) or blocking IL-17A bioactivity compensated for the inhibitory effects of ATB in tumor immunosurveillance. Fourthly, enforced oral gavage with Enterocloster spp (such as *E. clostridioformis*) exacerbated the exodus of enterotropic Tr17 to tdLN. Finally, PD1 blockade tended to aggravate the expansion and/or priming of α4β7+ Tr17 cells in mLN and homing back to tumors, compromising cancer immunosurveillance. All these lines of evidence support the epidemiological studies pointing to a harmful effect of ATB prior to ICI administration, as opposed to during or after ICI start (Derosa et al. 2022). Consequently, we show that serum soluble MADCAM-1 is a biological proxy for ileal *madcam1* gene expression and represents a reliable predictor of response to PD1 blockade, at least in NSCLC patients. Low sMADCAM-1 serum levels mirrored intestinal dysbiosis dominated by pro-TH17 bacteria (such as *Veilonella parvula*) or species from the Enterocloster genus associated with physiopathological disorders (Ghosh et al., 2020; Tsay et al., 2021; Zitvogel and Kroemer, 2021) (Derosa et al. 2022).

This demonstration follows prior evidence showing that gut derived-T_{H}17 also control extraintestinal autoimmune (Magnuson et al., 2015; Morton et al., 2014; Wu et al., 2010) (Krebs et al., 2016; Lee et al., 2011b) or inflammatory/ischemic lesions (Benakis et al., 2016; Liesz et al., 2009). RORγt⁺ Treg harbour an exacerbated immunosuppressive phenotype compared with their lineage related-Treg with overexpression of gene products (such as Ctla4, Icos, Havcr2 (Sefik Science 2015) that we also found upregulated in our ATB-induced gut dysbiosis setting. Moreover, we identified gut-specific Treg features in our Tr17 fingerprint previously described in landmark papers (Miragaia et al., 2019; Muñoz-Rojas and Mathis, 2021), TH17 related blueprints (Yosef et al., 2013) as well as immunosuppressive traits functionally relevant in tumor immunosurveillance (such as Dusp2/PCA1 (Dan Lu et al., 2020), PIK3ip1 (Uche et al., 2018).

This exemplification in cancer bearers is in line with previous studies performed in patients or macaques suffering from inflammatory bowel disease, reporting an increased recirculation of extraintestinal α4β7⁺ T_{reg} and central memory T cells or CCR6⁺ CD4⁺ T lymphocytes during treatment with the anti- α4β7 antibody vedolizumab (Calenda et al., 2018; D'Haens et al., 2018; Fischer et al., 2016). Our findings have important clinical implications. So far, vedolizumab was considered as a safer alternative to TNFα inhibition in cancer patients suffering from ICI-induced autoimmune colitis because of their gut restricted-mode of action (Sandborn et al., 2016). However, prospective studies should monitor microbiome composition, gut or soluble serum MAdCAM-1 expression, as well as the recirculation of CCR9 α4β7⁺ T_{reg} , Tr17 and Tₕ22 cell subsets to correlate these novel parameters with the clinical outcome and toxicity profiles in patients treated with ICI. Finally, the field of fecal microbial transplantation may evolve towards guiding selection of donor feces based on their capacity to normalize MADCAM-1 levels in the cancer bearing recipients.

These findings fuel the concept that cancer bearing patients can exacerbate their cancer-associated ileopathy (Yonekura et al 2022) by taking antibiotics that alter one of the most potent gut immune checkpoint MADCAM-1. We surmise that ATB-induced dysbiosis and most specifically recolonization post-ATB course is a major regulator of gut immune checkpoint MADCAM-1. Several molecular cues may link gut dysbiosis with the alteration of GALT MADCAM-1 such as perturbations of the bacteria-induced metabolism of biliary salts (Campbell et al., 2020; Song et al., 2020), and activation of the sympathetic nervous system for instance (Schiller et al., 2021; Yan et al., 2021), that deserve further investigations.

### Example 2: Soluble MAdCAM-1 is a predictive biomarker of resistance to PD1 blockade in several solid cancers

### 2.1. Soluble MADCAM-1 is a predictive biomarker of resistance to PD1 blockade in kidney cancer patients

In Example 1, we showed, in two independent cohorts of advanced non small cell lung cancer patients (NSCLC), that serum soluble MAdCAM-1 level is a marker of resistance or sensitivity to an immuno-oncology (I-O) therapy, wherein a reduced level of serum soluble MAdCAM-1 is a marker of resistance to the I-O therapy.

Here, we extend this finding to another cancer type: the kidney cancer treated with nivolumab in second line therapy (after failure of tyrosine kinase inhibitors) (Table 8).

**Table 8. Patient's clinical characteristics**

| | | **Overall (n=212)** |
|---|---|---|
| Age (year) | Median (range) | 64.0 (22.0; 87.0) |
| | <70 - no (%) | 151 (71.2) |
| | ≥70 - no (%) | 61 (28.8) |
| Sex - no (%) | Male | 174 (82.1) |
| | Female | 38 (17.9) |
| ECOG performance status - no (%) | 0-1 | 115 (54.5) |
| | 2 or more | 96 (45.4) |
| | Not reported | 1 |
| BMI classification (kg/m²) - no. (%) | <25 | 91 (44.2) |
| | [25;30[ | 78 (37.9) |
| | ≥ 30 | 37 (18.0) |
| | Not reported | 6 |
| Tumor histology - no. (%) | Clear Cell | 212 (100) |
| | Favorable | 39 (18.5) |
| IMDC score- no. (%) | Intermediate | 123 (58.3) |
| | Poor | 49 (23.2) |
| | Not reported | 1 |
| Brain metastasis- no. (%) | Yes | 28 (14.4) |
| | No | 166 (85.6) |
| | Not reported | 18 |

Overall, 212 patients from the NIVOREIN cohorts were enrolled to receive anti-PD1 mAb (Nivolumab, BMS). 31 were antibiotics users (between day -60 and day +42 after treatment initiation) and 176 did not take antibiotics. The ELISA monitoring of serum soluble MAdCAM-1 at baseline was performed, revealing a **median (min,max)** of **88.8 ng/ml (19.1, 174.7**) (and a mean±SD of 88.4 ±27.7 ng/ml) for these 212 metastatic clear cell renal cell carcinoma (RCC) patients. As shown for NSCLC patients, antibiotic uptake (ATB) tended to decrease the concentrations of sMAdCAM-1 (from a median of 88.7 ng/ml in those patients who refrained from taking ATB down to 76.3 ng/ml in those who took ATB). Of note, this is underestimated since the vast majority of these ATB+patients (23/31) took ATB after initiation of anti-PD1 Ab (which is less clinically significant).

The overall survival (OS) was analyzed according to the median value of sMAdCAM-1 baseline levels right before anti-PD1 Ab initiation, for the whole cohort of 212 patients. The Kaplan Meier OS curves of RCC patients show that those presenting a sMAdCAM-1 serum level <88.8 ng/ml died in 72/106 cases, while the mortality rate was 36/106 in those who had a sMAdCAM-1 serum concentration ≥88.8 ng/ml (p<10e-4) (Figure 11). This prognosis parameter was analyzed in multivariate analysis encompassing all clinical factors impacting the survival of stage IV RCC such as age, IMDC score, lines of therapies and metastases location and hypoalbuminemia. The multivariate analyses revealed that sMAdCAM-1 <88.8 ng/ml outperformed the IMDC score with a Hazard ratio: 2.40 (1.52-3.80, p=0.0002) for sMAdCAM-1 versus HR= 2.29 (1.11-4.76, p=0.08) for IMDC severe (Table 9).

**Table 9. Multivariate Cox model of the prognostic significance of serum sMAdCAM-1 <88.8ng/ml for overall survival of stage IV second line aPD1 Ab-treated RCC patients**

| | Factor label | Events/N | Hazard Ratio (95% CI) | P-value |
|---|---|---|---|---|
| **Antibiotics (ATB)** | | | | |
| | ATB non-users | 86/171 | 1 | 0.3956 |
| | ATB users | 17/28 | 1.27 (0.73-2.23) | |

| **MAdCAM-1** | | | | |
|---|---|---|---|---|
| | ≥88.8 | 33/96 | 1 | 0.0002 |
| | <88.8 | 70/103 | 2.40 (1.52-3.80) | |

| **Gender** | | | | |
|---|---|---|---|---|
| | Male | 86/163 | 1 | 0.5075 |
| | Female | 17/36 | 0.83 (0.47-1.45) | |

| **Age (years)** | | | | |
|---|---|---|---|---|
| | <70 | 69/144 | 1 | 0.0116 |
| | ≥70 | 34/55 | 1.73 (1.13-2.66) | |

| **IMDC prognostic risk** | | | | |
|---|---|---|---|---|
| | Favorable | nov-35 | 1 | 0.0828 |
| | Intermediate | 59/117 | 1.75 (0.91-3.37) | |
| | Poor | 33/47 | 2.29 (1.11-4.76) | |

| **Previous lines >2** | | | | |
|---|---|---|---|---|
| | No | 85/163 | 1 | 0.3519 |
| | Yes | 18/36 | 0.77 (0.45-1.33) | |

| **Brain metastasis** | | | | |
|---|---|---|---|---|
| | No | 88/178 | 1 | <.0001 |
| | Yes | 15/21 | 3.49 (1.93-6.28) | |

| **Bone metastasis** | | | | |
|---|---|---|---|---|
| | No | 63/136 | 1 | 0.0240 |
| | Yes | 40/63 | 1.64 (1.07-2.52) | |

| **Liver metastasis** | | | | |
|---|---|---|---|---|
| | No | 80/148 | 1 | 0.2467 |
| | Yes | 23/51 | 0.75 (0.46-1.22) | |

| **Hypoalbuminemia** | | | | |
|---|---|---|---|---|
| | No | 57/130 | 1 | 0.0571 |
| | Yes | 46/69 | 1.52 (0.99-2.35) | |

The progression-free survival (PFS) was analyzed according to the median value of sMAdCAM-1 baseline levels right before anti-PD1 Ab initiation, for the whole cohort of 212 patients. The Kaplan Meier PFS curves of RCC show that patients presenting a sMAdCAM-1 serum level <88.8 ng/ml progressed in 99/106 cases, while progression rate was 86/106 in those who had a sMAdCAM-1 serum concentration ≥88.8 ng/ml (p<10e-4) (Figure 12). This prognosis parameter was analyzed in multivariate analysis encompassing all clinical factors impacting the survival of stage IV RCC such as age, IMDC score, lines of therapies and metastases location and hypoalbuminemia. The multivariate analyses revealed that sMAdCAM-1 <88.8 ng/ml was as good as the IMDC score (Table 10, HR= 1.55, p=0.0071).

**Table 10. Multivariate analysis of the prognostic significance of serum sMAdCAM-1 <88.8ng/ml for progression-free survival of stage IV second line aPD1 Ab-treated RCC patients**

| | Factor label | Events/N | Hazard Ratio (95% CI) | P-value |
|---|---|---|---|---|
| **Antibiotics (ATB)** | | | | |
| | ATB non-users | 153/171 | 1 | 0.8438 |
| | ATB users | 26/28 | 0.96 (0.61-1.49) | |

| **MAdCAM-1** | | | | |
|---|---|---|---|---|
| | ≥88.8 | 83/96 | 1 | 0.0071 |
| | <88.8 | 96/103 | 1.55 (1.13-2.13) | |

| **Gender** | | | | |
|---|---|---|---|---|
| | Male | 146/163 | 1 | 0.1833 |
| | Female | 33/36 | 1.31 (0.88-1.94) | |

| **Age (years)** | | | | |
|---|---|---|---|---|
| | <70 | 128/144 | 1 | 0.6814 |
| | ≥70 | 51/55 | 1.08 (0.76-1.53) | |

| **IMDC prognostic risk** | | | | |
|---|---|---|---|---|
| | Favorable | 30/35 | 1 | 0.0088 |
| | Intermediate | 104/117 | 1.40 (0.91-2.14) | |
| | Poor | 45/47 | 2.23 (1.32-3.76) | |

| **Previous lines >2** | | | | |
|---|---|---|---|---|
| | No | 145/163 | 1 | 0.8519 |
| | Yes | 34/36 | 1.04 (0.70-1.55) | |

| **Brain metastasis** | | | | |
|---|---|---|---|---|
| | No | 159/178 | 1 | 0.0008 |
| | Yes | 20/21 | 2.33 (1.42-3.81) | |

| **Bone metastasis** | | | | |
|---|---|---|---|---|
| | No | 122/136 | 1 | 0.3622 |
| | Yes | 57/63 | 1.16 (0.84-1.62) | |

| **Liver metastasis** | | | | |
|---|---|---|---|---|
| | No | 133/148 | 1 | 0.7620 |
| | Yes | 46/51 | 1.06 (0.74-1.50) | |

| **Hypoalbuminemia** | | | | |
|---|---|---|---|---|
| | No | 116/130 | 1 | 0.6116 |
| | Yes | 63/69 | 1.09 (0.78-1.54) | |

As for objective response rates (ORR), NIVOREN trial (N=729 RCC patients) recorded an objective response rate of 20.8% (1.2% of complete responses and 19.6 % of partial responses, 79.2% of progressive diseases (PD)). Interestingly, the subgroup of 212 RCC patients for whom sMAdCAM-1 serum levels were available, was subdivided according to ATB uptake Yes/no and to sMAdCAM-1 (≥88.8 versus <88.8 ng/ml). First, subgroup analysis of best objective response according to sMAdCAM-1 revealed that the percentages of PD was 36.7% vs 62.6% in patients with sMAdCAM-1 ≥88.8 versus <88.8 ng/ml respectively (p=0.0003) (Table 10). Next, when we considered only patients who did not take ATB, the same subgroup analysis of best objective response according to sMAdCAM-1 revealed that the percentages of PD was 38.6% vs 60.5% in patients with sMAdCAM-1 ≥88.8 versus <88.8 ng/ml respectively (p=0.0044). Thirdly, when we considered RCC patients who took ATB, this subgroup analysis of best response rate according to sMAdCAM-1 revealed that the percentages of PD was 36.4% vs 76.9% in patients with sMAdCAM-1 ≥88.8 versus <88.8 ng/ml respectively (p=0.0446). In the ATB-treated subgroup, the percentage of complete responders was 18.2% versus 7.7% in patients with sMAdCAM-1 ≥88.8 versus <88.8 ng/ml respectively (Table 11 below).

**Table 11. Overall response rate**

| | | **Overall (n=212)** | **High sMadCAM1 (n=106)** | **Low sMadCAM1 (n=106)** | **P-value** |
|---|---|---|---|---|---|
| **Best response** - no (%) | | | | | |
| | Partial response | 14 (7.1) | 9 (9.2) | 5 (5.1 | |
| | Stable disease | 85 (43.1) | 53 (54.1) | 32 (32.3) | 0.0003 |
| | Progressive disease | 98 (49.7) | 36 (36.7) | 62 (62.6) | |
| | Not reported | 15 | 8 | 7 | |

| **Antibiotic users** - no (%) | | | | | |
|---|---|---|---|---|---|
| | Partial response | 3 (12.5) | 2 (18.2) | 1 (7.7) | |
| | Stable disease | 7 (29.2) | 5 (45.5) | 2 (15.4) | 0.0446 |
| | Progressive disease | 14 (58.3) | 4 (36.4) | 10 (76.9) | |
| | Not reported | 7 | 3 | 4 | |

| **Antibiotic non-users** - no (%) | | | | | |
|---|---|---|---|---|---|
| | Partial response | 9 (5.3) | 5 (6.0) | 4 (4.7) | |
| | Stable disease | 76 (45.0) | 46 (55.4) | 30 (34.9) | 0.0044 |
| | Progressive disease | 84 (49.7) | 32 (38.6) | 52 (60.5) | |
| | Not reported | 7 | 4 | 3 | |

Altogether, these findings demonstrate that high levels of serum soluble MAdCAM-1 are associated with increased response to PD1 blockade in second line immunotherapy of RCC, and more so in patients who took antibiotics. The threshold of 88 ng/ml is valid for second line but is not representative of first line patients. For routine usage, the median of a cohort of 1L or 2L therapy must be considered.

### 2.2. Soluble MADCAM-1 is a predictive biomarker of resistance to PD1 blockade in bladder cancer patients

Finally, we sought to triplicate the predictive value of sMAdCAM-1 in a subcohort of a Phase 3a trial enrolling pre-treated metastatic bladder cancer patients to receive a fixed dose of 1500 mg of Durvalumab iv (anti-PD-L1 Abs)/4 weeks. In this STRONG study, 79 patients only were analyzed, with an overall median OS of 5.79 months, and a median PFS of 2.79 months. We selected the extreme of the STRONG cohort, 30 Elite patients (OS>6 months, PFS>5 months, PR+CR only) and rapid progressors (n=49, with OS<6 months and PFS<5 months and PD at the first CT scan). In this cohort, the MAdCAM-1 median (min,max) was158.8 ng/ml (53.41, 244.36). In Figure 13A, we show that the median ±SEM of sMAdCAM-1 was dramatically decreased in progressors and OS was significantly better in the first half of patients presenting a baseline level of sMAdCAM-1 > 158.8 ng/ml compared with the other half (Figure 13B, p=0.03). PFS tended to be superior in patients with high MAdCAM-1 levels (Figure 13C, ns).

### 2.3. Conclusion

Altogether, these findings demonstrate that high levels of serum soluble MAdCAM-1 are associated with increased response to PD1/PDL-1 blockade in second line immunotherapy of RCC, in second line metastatic bladder tumors and in 1L and 2L NSCLC treated with pembrolizumab regardless of ATB uptake.

### Abbreviations:

α4β7: alpha4beta7, Abs: antibodies, ACS: Ampicillin, Colistin, Streptomycin, ATB: antibiotics, ATRA: all-*trans* retinoic acid, CCL: Chemokine ligand, CCR: Chemokine receptor, CD: Cluster of differentiation, cDC1: classical dendritic cell type 1, CFSE: Carboxyfluorescein succinimidyl ester, cLN: contralateral lymph node, CSF-1: colony stimulating factor 1, CTL: cytotoxic T lymphocyte, FMT: fecal mucosal transfer, FoxP3: forkhead box P3, GALT: Gut-associated lymphoid tissue, HEV: high endothelial venule, IBD: inflammatory bowel disease, ICI: Immune checkpoint inhibitors, IFNγ: Interferon γ, LP: lamina propria, mABs: monoclonal antibodies, MAdCAM-1: Mucosal Addressin Cell Adhesion Molecule-1, mLN: mesenteric lymph node, PP: Peyer's patch, RA: retinoic acid, RORC: RAR-related orphan receptor C, SLO: secondary lymphoid organ, tdLN : tumor draining lymph node, T_{H}17: T-helper 17-CD4-T-cell, TME: tumor microenvironment, TNFα: Tumor necrosis factor alpha, T_{reg}: regulatory T-cell, Tr17: RORγt+ T_{reg} -CD4-T-cell.

### REFERENCES

Baruch, E.N., Youngster, I., Ben-Betzalel, G., Ortenberg, R., Lahat, A., Katz, L., Adler, K., Dick-Necula, D., Raskin, S., Bloch, N., et al. (2021). Fecal microbiota transplant promotes response in immunotherapy-refractory melanoma patients. Science 371, 602-609.
Baumann, S., Hess, J., Eichhorst, S.T., Krueger, A., Angel, P., Krammer, P.H., and Kirchhoff, S. (2003). An unexpected role for FosB in activation-induced cell death of T cells. Oncogene 22, 1333-1339.
Belarif, L., Danger, R., Kermarrec, L., Nerrière-Daguin, V., Pengam, S., Durand, T., Mary, C., Kerdreux, E., Gauttier, V., Kucik, A., et al. (2019). IL-7 receptor influences anti-TNF responsiveness and T cell gut homing in inflammatory bowel disease. J Clin Invest 129, 1910-1925.
Benakis, C., Brea, D., Caballero, S., Faraco, G., Moore, J., Murphy, M., Sita, G., Racchumi, G., Ling, L., Pamer, E.G., et al. (2016). Commensal microbiota affects ischemic stroke outcome by regulating intestinal γδ T cells. Nat. Med. 22, 516-523.
Blatner, N.R., Mulcahy, M.F., Dennis, K.L., Scholtens, D., Bentrem, D.J., Phillips, J.D., Ham, S., Sandall, B.P., Khan, M.W., Mahvi, D.M., et al. (2012). Expression of RORγt Marks a Pathogenic Regulatory T Cell Subset in Human Colon Cancer. Science Translational Medicine 4, 164ra159-164ra159.
Brahmer, J., Reckamp, K.L., Baas, P., Crinò, L., Eberhardt, W.E.E., Poddubskaya, E., Antonia, S., Pluzanski, A., Vokes, E.E., Holgado, E., et al. (2015). Nivolumab versus Docetaxel in Advanced Squamous-Cell Non-Small-Cell Lung Cancer. N. Engl. J. Med. 373, 123-135.
Briskin, M., Winsor-Hines, D., Shyjan, A., Cochran, N., Bloom, S., Wilson, J., McEvoy, L.M., Butcher, E.C., Kassam, N., Mackay, C.R., et al. (1997). Human mucosal addressin cell adhesion molecule-1 is preferentially expressed in intestinal tract and associated lymphoid tissue. Am. J. Pathol. 151, 97-110.
Calenda, G., Keawvichit, R., Arrode-Brusés, G., Pattanapanyasat, K., Frank, I., Byrareddy, S.N., Arthos, J., Cicala, C., Grasperge, B., Blanchard, J.L., et al. (2018). Integrin α4β7 Blockade Preferentially Impacts CCR6+ Lymphocyte Subsets in Blood and Mucosal Tissues of Naive Rhesus Macaques. J. Immunol. 200, 810-820.
Campbell, C., McKenney, P.T., Konstantinovsky, D., Isaeva, O.I., Schizas, M., Verter, J., Mai, C., Jin, W.-B., Guo, C.-J., Violante, S., et al. (2020). Bacterial metabolism of bile acids promotes generation of peripheral regulatory T cells. Nature 581, 475-479.
Cano-Gamez, E., Soskic, B., Roumeliotis, T.I., So, E., Smyth, D.J., Baldrighi, M., Willé, D., Nakic, N., Esparza-Gordillo, J., Larminie, C.G.C., et al. (2020). Single-cell transcriptomics identifies an effectorness gradient shaping the response of CD4+ T cells to cytokines. Nat Commun 11, 1801.
Chen, D.S., and Mellman, I. (2017). Elements of cancer immunity and the cancer-immune set point. Nature 541, 321-330.
Chen, Y., Wang, J., Wang, X., Li, X., Song, J., Fang, J., Liu, X., Liu, T., Wang, D., Li, Q., et al. (2019). Pik3ip1 Is a Negative Immune Regulator that Inhibits Antitumor T-Cell Immunity. Clin Cancer Res 25, 6180-6194.
Cochaud, S., Giustiniani, J., Thomas, C., Laprevotte, E., Garbar, C., Savoye, A.-M., Curé, H., Mascaux, C., Alberici, G., Bonnefoy, N., et al. (2013). IL-17A is produced by breast cancer TILs and promotes chemoresistance and proliferation through ERK1/2. Sci Rep 3, 3456.
Daillère, R., Vétizou, M., Waldschmitt, N., Yamazaki, T., Isnard, C., Poirier-Colame, V., Duong, C.P.M., Flament, C., Lepage, P., Roberti, M.P., et al. (2016). Enterococcus hirae and Barnesiella intestinihominis Facilitate Cyclophosphamide-Induced Therapeutic Immunomodulatory Effects. Immunity 45, 931-943.
Dan Lu, Liu, L., Sun, Y., Song, J., Yin, Q., Zhang, G., Qi, F., Hu, Z., Yang, Z., Zhou, Z., et al. (2020). The phosphatase PAC1 acts as a T cell suppressor and attenuates host antitumor immunity. Nat Immunol 21, 287-297.
Daniel, C., Wennhold, K., Kim, H.-J., and von Boehmer, H. (2010). Enhancement of antigen-specific Treg vaccination in vivo. Proc Natl Acad Sci U S A 107, 16246-16251.
Davar, D., Dzutsev, A.K., McCulloch, J.A., Rodrigues, R.R., Chauvin, J.-M., Morrison, R.M., Deblasio, R.N., Menna, C., Ding, Q., Pagliano, O., et al. (2021). Fecal microbiota transplant overcomes resistance to anti-PD-1 therapy in melanoma patients. Science 371, 595-602.
Denning, T.L., Kim, G., and Kronenberg, M. (2005). Cutting edge: CD4+CD25+ regulatory T cells impaired for intestinal homing can prevent colitis. J. Immunol. 174, 7487-7491.
Derosa, L., Routy, B., Fidelle, M., lebba, V., Alla, L., Pasolli, E., Segata, N., Desnoyer, A., Pietrantonio, F., Ferrere, G., et al. (2020). Gut Bacteria Composition Drives Primary Resistance to Cancer Immunotherapy in Renal Cell Carcinoma Patients. Eur Urol 78, 195-206.
Derosa, L., Routy, B., Desilets, A., Daillère, R., Terrisse, S., Kroemer, G., and Zitvogel, L. (2021). Microbiota-Centered Interventions: The Next Breakthrough in Immuno-Oncology? Cancer Discov 11, 2396-2412.
Derosa L, Routy B, Thomas AM, lebba V, Zalcman G, Friard S, Mazieres J, Audigier-Valette C, Moro-Sibilot D, Goldwasser F et al. (2022). Intestinal Akkermansia muciniphila predicts clinical response to PD-1 blockade in patients with advanced non-small-cell lung cancer. Nat Med 28(2), 315-324.
D'Haens, G., Vermeire, S., Vogelsang, H., Allez, M., Desreumaux, P., Van Gossum, A., Sandborn, W.J., Baumgart, D.C., Ransohoff, R.M., Comer, G.M., et al. (2018). Effect of PF-00547659 on Central Nervous System Immune Surveillance and Circulating β7+ T Cells in Crohn's Disease: Report of the TOSCA Study. J Crohns Colitis 12, 188-196.
Dogan, A. et al (1997). American Journal of Pathology, pages 1361-1369.
Elkrief, A., Derosa, L., Kroemer, G., Zitvogel, L., and Routy, B. (2019a). The negative impact of antibiotics on outcomes in cancer patients treated with immunotherapy: a new independent prognostic factor? Ann. Oncol.
Elkrief, A., El Raichani, L., Richard, C., Messaoudene, M., Belkaid, W., Malo, J., Belanger, K., Miller, W., Jamal, R., Letarte, N., et al. (2019b). Antibiotics are associated with decreased progression-free survival of advanced melanoma patients treated with immune checkpoint inhibitors. Oncoimmunology 8, e1568812.
Fischer, A., Zundler, S., Atreya, R., Rath, T., Voskens, C., Hirschmann, S., López-Posadas, R., Watson, A., Becker, C., Schuler, G., et al. (2016). Differential effects of α4β7 and GPR15 on homing of effector and regulatory T cells from patients with UC to the inflamed gut in vivo. Gut 65, 1642-1664.
Fridman, W.H., Pagès, F., Sautès-Fridman, C., and Galon, J. (2012). The immune contexture in human tumours: impact on clinical outcome. Nat. Rev. Cancer 12, 298-306.
Gao, J., Shi, L.Z., Zhao, H., Chen, J., Xiong, L., He, Q., Chen, T., Roszik, J., Bernatchez, C., Woodman, S.E., et al. (2016). Loss of IFN-γ Pathway Genes in Tumor Cells as a Mechanism of Resistance to Anti-CTLA-4 Therapy. Cell 167, 397-404.e9.
Gao, Y., Deason, K., Jain, A., Irizarry-Caro, R.A., Dozmorov, I., Coughlin, L.A., Rauch, I., Evers, B.M., Koh, A.Y., Wakeland, E.K., et al. (2020). Transcriptional profiling identifies caspase-1 as a T cell-intrinsic regulator of Th17 differentiation. J Exp Med 217, e20190476.
Ghosh, T.S., Das, M., Jeffery, I.B., and O'Toole, P.W. (2020). Adjusting for age improves identification of gut microbiome alterations in multiple diseases. Elife 9, e50240.
Gorfu, G., Rivera-Nieves, J., and Ley, K. (2009). Role of beta7 integrins in intestinal lymphocyte homing and retention. Curr. Mol. Med. 9, 836-850.
Goubet, A.-G., Wheeler, R., Fluckiger, A., Qu, B., Lemaitre, F., Iribarren, K., Mondragón, L., Tidjani Alou, M., Pizzato, E., Durand, S., et al. (2021). Multifaceted modes of action of the anticancer probiotic Enterococcus hirae. Cell Death Differ 28, 2276-2295.
Guéry, L., and Hugues, S. (2015). Th17 Cell Plasticity and Functions in Cancer Immunity. Biomed Res Int 2015, 314620.
Haas, K.N., and Blanchard, J.L. (2020). Reclassification of the Clostridium clostridioforme and Clostridium sphenoides clades as Enterocloster gen. nov. and Lacrimispora gen. nov., including reclassification of 15 taxa. Int J Syst Evol Microbiol 70, 23-34.
Hassan-Zahraee, M., Banerjee, A., Cheng, J.B., Zhang, W., Ahmad, A., Page, K., von Schack, D., Zhang, B., Martin, S.W., Nayak, S., et al. (2018). Anti-MAdCAM Antibody Increases ß7+ T Cells and CCR9 Gene Expression in the Peripheral Blood of Patients With Crohn's Disease. J Crohns Colitis 12, 77-86.
Holmer, A.K., Battat, R., Dulai, P.S., Vande Casteele, N., Nguyen, N., Jain, A., Miralles, A., Neill, J., Le, H., Singh, S., et al. (2020). Biomarkers are associated with clinical and endoscopic outcomes with vedolizumab treatment in Crohn's disease. Therap Adv Gastroenterol 13, 1756284820971214.
Kim, J.-H., Hwang, J., Jung, J.H., Lee, H.-J., Lee, D.Y., and Kim, S.-H. (2019). Molecular networks of FOXP family: dual biologic functions, interplay with other molecules and clinical implications in cancer progression. Molecular Cancer 18, 180.
Krebs, C.F., Paust, H.-J., Krohn, S., Koyro, T., Brix, S.R., Riedel, J.-H., Bartsch, P., Wiech, T., Meyer-Schwesinger, C., Huang, J., et al. (2016). Autoimmune Renal Disease Is Exacerbated by S1P-Receptor-1-Dependent Intestinal Th17 Cell Migration to the Kidney. Immunity 45, 1078-1092.
Langowski, J.L., Zhang, X., Wu, L., Mattson, J.D., Chen, T., Smith, K., Basham, B., McClanahan, T., Kastelein, R.A., and Oft, M. (2006). IL-23 promotes tumour incidence and growth. Nature 442, 461-465.
Lee, Y.K., Menezes, J.S., Umesaki, Y., and Mazmanian, S.K. (2011a). Proinflammatory T-cell responses to gut microbiota promote experimental autoimmune encephalomyelitis. Proc Natl Acad Sci U S A 108 Suppl 1, 4615-4622.
Lee, Y.K., Menezes, J.S., Umesaki, Y., and Mazmanian, S.K. (2011b). Proinflammatory T-cell responses to gut microbiota promote experimental autoimmune encephalomyelitis. Proc. Natl. Acad. Sci. U.S.A. 108 Suppl 1, 4615-4622.
Leung et al. (2003). Immunology and Cell Biology, vol. 81, no. 4, pages 320-327.
Leung et al. (2004). Immunology and Cell Biology, vol. 82, pages 400-409.
Li, L., Patsoukis, N., Petkova, V., and Boussiotis, V.A. (2012). Runx1 and Runx3 are involved in the generation and function of highly suppressive IL-17-producing T regulatory cells. PLoS One 7, e45115.
Liesz, A., Suri-Payer, E., Veltkamp, C., Doerr, H., Sommer, C., Rivest, S., Giese, T., and Veltkamp, R. (2009). Regulatory T cells are key cerebroprotective immunomodulators in acute experimental stroke. Nat. Med. 15, 192-199.
Lim, C., and Savan, R. (2014). The role of the IL-22/IL-22R1 axis in cancer. Cytokine Growth Factor Rev 25, 257-271.
Littman, D.R., and Rudensky, A.Y. (2010). Th17 and regulatory T cells in mediating and restraining inflammation. Cell 140, 845-858.
Magnuson, A.M., Thurber, G.M., Kohler, R.H., Weissleder, R., Mathis, D., and Benoist, C. (2015). Population dynamics of islet-infiltrating cells in autoimmune diabetes. Proc. Natl. Acad. Sci. U.S.A. 112, 1511-1516.
Miragaia, R.J., Gomes, T., Chomka, A., Jardine, L., Riedel, A., Hegazy, A.N., Whibley, N., Tucci, A., Chen, X., Lindeman, I., et al. (2019). Single-Cell Transcriptomics of Regulatory T Cells Reveals Trajectories of Tissue Adaptation. Immunity 50, 493-504.e7493.
Mohiuddin, J.J., Chu, B., Facciabene, A., Poirier, K., Wang, X., Doucette, A., Zheng, C., Xu, W., Anstadt, E.J., Amaravadi, R.K., et al. (2021). Association of Antibiotic Exposure With Survival and Toxicity in Patients With Melanoma Receiving Immunotherapy. J Natl Cancer Inst 113, 162-170.
Morton, A.M., Sefik, E., Upadhyay, R., Weissleder, R., Benoist, C., and Mathis, D. (2014). Endoscopic photoconversion reveals unexpectedly broad leukocyte trafficking to and from the gut. Proc. Natl. Acad. Sci. U.S.A. 111, 6696-6701.
Muñoz-Rojas, A.R., and Mathis, D. (2021). Tissue regulatory T cells: regulatory chameleons. Nat Rev Immunol 21, 597-611.
Neubert, N.J., Schmittnaegel, M., Bordry, N., Nassiri, S., Wald, N., Martignier, C., Tillé, L., Homicsko, K., Damsky, W., Maby-El Hajjami, H., et al. (2018). T cell-induced CSF1 promotes melanoma resistance to PD1 blockade. Sci Transl Med 10.
Ogawa, H., Binion, D.G., Heidemann, J., Theriot, M., Fisher, P.J., Johnson, N.A., Otterson, M.F., and Rafiee, P. (2005). Mechanisms of MAdCAM-1 gene expression in human intestinal microvascular endothelial cells. Am. J. Physiol., Cell Physiol. 288, C272-281.
Pandiyan, P., Bhaskaran, N., Zou, M., Schneider, E., Jayaraman, S., and Huehn, J. (2019). Microbiome Dependent Regulation of Tregs and Th17 Cells in Mucosa. Front Immunol 10, 426.
Porter, RJ et al (2021). Journal of Crohn's and Colitis, vol. 15, no. 12, 1 pages 2131-2141.
Reinisch, W., Sandborn, W.J., Danese, S., Hébuterne, X., Klopocka, M., Tarabar, D., Vaňásek, T., Greguš, M., Hellstern, P.A., Kim, J.S., et al. (2021). Long-term Safety and Efficacy of the Anti-MAdCAM-1 Monoclonal Antibody Ontamalimab [SHP647] for the Treatment of Ulcerative Colitis: The Open-label Study TURANDOT II. J Crohns Colitis *15*, 938-949.
Riaz, N., Havel, J.J., Kendall, S.M., Makarov, V., Walsh, L.A., Desrichard, A., Weinhold, N., and Chan, T.A. (2016). Recurrent SERPINB3 and SERPINB4 mutations in patients who respond to anti-CTLA4 immunotherapy. Nat. Genet. 48, 1327-1329.
Rizvi, N.A., Hellmann, M.D., Snyder, A., Kvistborg, P., Makarov, V., Havel, J.J., Lee, W., Yuan, J., Wong, P., Ho, T.S., et al. (2015). Mutational landscape determines sensitivity to PD-1 blockade in non-small cell lung cancer. Science 348, 124-128.
Rizzo, A., Di Giovangiulio, M., Stolfi, C., Franzè, E., Fehling, H.-J., Carsetti, R., Giorda, E., Colantoni, A., Ortenzi, A., Rugge, M., et al. (2018). RORγt-Expressing Tregs Drive the Growth of Colitis-Associated Colorectal Cancer by Controlling IL6 in Dendritic Cells. Cancer Immunol Res 6, 1082-1092.
Robert, C., Thomas, L., Bondarenko, I., O'Day, S., Weber, J., Garbe, C., Lebbe, C., Baurain, J.-F., Testori, A., Grob, J.-J., et al. (2011). Ipilimumab plus dacarbazine for previously untreated metastatic melanoma. N. Engl. J. Med. 364, 2517-2526.
Routy, B., Le Chatelier, E., Derosa, L., Duong, C.P.M., Alou, M.T., Daillère, R., Fluckiger, A., Messaoudene, M., Rauber, C., Roberti, M.P., et al. (2017). Gut microbiome influences efficacy of PD-1-based immunotherapy against epithelial tumors. Science.
Routy, B., Le Chatelier, E., Derosa, L., Duong, C.P.M., Alou, M.T., Daillère, R., Fluckiger, A., Messaoudene, M., Rauber, C., Roberti, M.P., et al. (2018a). Gut microbiome influences efficacy of PD-1-based immunotherapy against epithelial tumors. Science 359, 91-97.
Routy, B., Gopalakrishnan, V., Daillère, R., Zitvogel, L., Wargo, J.A., and Kroemer, G. (2018b). The gut microbiota influences anticancer immunosurveillance and general health. Nat Rev Clin Oncol 15, 382-396.
Sandborn, W.J., Panés, J., Zhang, H., Yu, D., Niezychowski, W., and Su, C. (2016). Correlation Between Concentrations of Fecal Calprotectin and Outcomes of Patients With Ulcerative Colitis in a Phase 2 Trial. Gastroenterology 150, 96-102.
Schiller, M., Azulay-Debby, H., Boshnak, N., Elyahu, Y., Korin, B., Ben-Shaanan, T.L., Koren, T., Krot, M., Hakim, F., and Rolls, A. (2021). Optogenetic activation of local colonic sympathetic innervations attenuates colitis by limiting immune cell extravasation. Immunity 54, 1022-1036.e8.
Sefik, E., Geva-Zatorsky, N., Oh, S., Konnikova, L., Zemmour, D., McGuire, A.M., Burzyn, D., Ortiz-Lopez, A., Lobera, M., Yang, J., et al. (2015). Individual intestinal symbionts induce a distinct population of RORγ+ regulatory T cells. Science 349, 993-997.
Singh, V., Roth, S., Llovera, G., Sadler, R., Garzetti, D., Stecher, B., Dichgans, M., and Liesz, A. (2016). Microbiota Dysbiosis Controls the Neuroinflammatory Response after Stroke. J. Neurosci. 36, 7428-7440.
Smyth, M.J., Ngiow, S.F., Ribas, A., and Teng, M.W.L. (2016). Combination cancer immunotherapies tailored to the tumour microenvironment. Nat Rev Clin Oncol 13, 143-158.
Song, X., Sun, X., Oh, S.F., Wu, M., Zhang, Y., Zheng, W., Geva-Zatorsky, N., Jupp, R., Mathis, D., Benoist, C., et al. (2020). Microbial bile acid metabolites modulate gut RORγ+ regulatory T cell homeostasis. Nature 577, 410-415.
Spranger, S., Bao, R., and Gajewski, T.F. (2015). Melanoma-intrinsic β-catenin signalling prevents anti-tumour immunity. Nature 523, 231-235.
Tian, Yuan et al (2019). Aging, pages 8623-8641
Tinsley, N., Zhou, C., Tan, G., Rack, S., Lorigan, P., Blackhall, F., Krebs, M., Carter, L., Thistlethwaite, F., Graham, D., et al. (2019). Cumulative Antibiotic Use Significantly Decreases Efficacy of Checkpoint Inhibitors in Patients with Advanced Cancer. Oncologist.
Tomura, M., Yoshida, N., Tanaka, J., Karasawa, S., Miwa, Y., Miyawaki, A., and Kanagawa, O. (2008). Monitoring cellular movement in vivo with photoconvertible fluorescence protein "Kaede" transgenic mice. Proc. Natl. Acad. Sci. U.S.A. 105, 10871-10876.
Tsay, J.-C.J., Wu, B.G., Sulaiman, I., Gershner, K., Schluger, R., Li, Y., Yie, T.-A., Meyn, P., Olsen, E., Perez, L., et al. (2021). Lower Airway Dysbiosis Affects Lung Cancer Progression. Cancer Discov 11, 293-307.
Uche, U.U., Piccirillo, A.R., Kataoka, S., Grebinoski, S.J., D'Cruz, L.M., and Kane, L.P. (2018). PIK3IP1/TrIP restricts activation of T cells through inhibition of PI3K/Akt. J Exp Med 215, 3165-3179.
Vétizou, M., Pitt, J.M., Daillère, R., Lepage, P., Waldschmitt, N., Flament, C., Rusakiewicz, S., Routy, B., Roberti, M.P., Duong, C.P.M., et al. (2015). Anticancer immunotherapy by CTLA-4 blockade relies on the gut microbiota. Science 350, 1079-1084.
Voigt, C., May, P., Gottschlich, A., Markota, A., Wenk, D., Gerlach, I., Voigt, S., Stathopoulos, G.T., Arendt, K.A.M., Heise, C., et al. (2017). Cancer cells induce interleukin-22 production from memory CD4+ T cells via interleukin-1 to promote tumor growth. PNAS 114, 12994-12999.
Wu, H.-J., Ivanov, I.I., Darce, J., Hattori, K., Shima, T., Umesaki, Y., Littman, D.R., Benoist, C., and Mathis, D. (2010). Gut-residing segmented filamentous bacteria drive autoimmune arthritis via T helper 17 cells. Immunity 32, 815-827.
Yan, Y., Ramanan, D., Rozenberg, M., McGovern, K., Rastelli, D., Vijaykumar, B., Yaghi, O., Voisin, T., Mosaheb, M., Chiu, I., et al. (2021). Interleukin-6 produced by enteric neurons regulates the number and phenotype of microbe-responsive regulatory T cells in the gut. Immunity 54, 499-513.e5.
Yonekura S, Terrisse S, Alves Costa Silva C, Lafarge A, lebba V, Ferrere G, Goubet AG, Fahrner JE, Lahmar I, Ueda K, et al. (2022). Cancer Induces a Stress Ileopathy Depending on β-Adrenergic Receptors and Promoting Dysbiosis that Contributes to Carcinogenesis. Cancer Discov 12(4):1128-1151.
Yosef, N., Shalek, A.K., Gaublomme, J.T., Jin, H., Lee, Y., Awasthi, A., Wu, C., Karwacz, K., Xiao, S., Jorgolli, M., et al. (2013). Dynamic regulatory network controlling TH17 cell differentiation. Nature 496, 461-468.
Young, M.R.I. (2016). Th17 Cells in Protection from Tumor or Promotion of Tumor Progression. J Clin Cell Immunol 7, 431.
Zitvogel, L., and Kroemer, G. (2021). Lower Airway Dysbiosis Exacerbates Lung Cancer. Cancer Discov 11, 224-226.

## Claims

1. Use of serum soluble MAdCAM-1 level, as a marker of resistance or sensitivity to an immuno-oncology (I-O) therapy, wherein a reduced level of serum soluble MAdCAM-1 is a marker of resistance to the I-O therapy.

2. Use of serum soluble MAdCAM-1 level, as a marker of cancer-associated dysbiosis or antibiotics-associated dysbiosis, wherein reduced level of serum soluble MAdCAM-1 is a marker of cancer- or antibiotics-associated dysbiosis.

3. A method for *in vitro* diagnosing cancer-associated dysbiosis or antibiotics-associated intestinal dysbiosis in an individual having a cancer, comprising measuring serum soluble MAdCAM-1, wherein a reduced level of serum soluble MAdCAM-1 indicates that the individual has a cancer-associated dysbiosis or antibiotics-associated intestinal dysbiosis.

4. The use of claim 1 or claim 2, or the method of claim 3, wherein the level of serum soluble MAdCAM-1 is considered as reduced if it is inferior to the median of the levels of soluble MAdCAM-1 in a representative cohort.

5. A theranostic method for determining if an individual having a cancer needs a compensatory microbiota-centered intervention (MCI) before administration of an immune-oncology (I-O) therapy, comprising assessing, using the method according to any one of claims 3 and 4, whether the individual has a cancer- or antibiotics-associated dysbiosis, wherein if the individual has a cancer- or antibiotics-associated dysbiosis, he/she needs a MCI before administration of the treatment with an I-O therapy.

6. The theranostic method of claim 5, wherein said treatment with an I-O therapy is selected from the group consisting of anti-PD1 antibodies (Ab), anti PDL-1 Ab, anti-CTLA4 Ab, anti-Lag3 Ab, anti-Tim3 Ab, anti-TIGIT Ab, anti-OX40 Ab, anti-41BB Ab, anti-VISTA Ab, bispecific antibodies targeting PD1 and Lag3, CAR-T cells, adoptive TIL transfer and any combination thereof, alone or combined to another antineoplastic agent, especially any combination of an immune checkpoint inhibitor (ICI) with taxane, cis-platin and/or oxaliplatinum.

7. The theranostic method of any one of claims 5 and 6, wherein the MCI is selected from the group consisting of:
- oral vancomycin antibiotics,
- phages killing bacteria of the *Enterocloster* gen. nov. clade,
- rare-cutting endonucleases such as Crispr Cas9 engineered to kill bacteria of the *Enterocloster* gen. nov. clade,
- *Akkermansia* spp and/or *Akkermansia muciniphila,* possibly mixed with other beneficial bacteria,
- retinoic acid
- fecal microbial transplantation, and
- mixtures thereof.

8. An agent for MCI, for use in the treatment of cancer in combination with an ICI in an individual having a reduced level of serum soluble MAdCAM-1, wherein said agent is selected from the group consisting of:
- oral vancomycin antibiotics,
- phages killing bacteria of the *Enterocloster* gen. nov. clade,
- rare-cutting endonucleases such as Crispr Cas9 engineered to kill bacteria of the *Enterocloster* gen. nov. clade,
- *Akkermansia* spp and/or *Akkermansia muciniphila,* possibly mixed with other beneficial bacteria,
- fecal microbial compositions, and
- mixtures thereof.

9. A method for following-up the success of a MCI, in an individual previously diagnosed with cancer- or antibiotics-associated dysbiosis, comprising measuring serum soluble MAdCAM-1, wherein a normalized level of serum soluble MAdCAM-1 indicates that the MCI successfully restored gut eubiosis.

10. Use of serum soluble MAdCAM-1 level, as a biomarker predicting early NSCLC incidence in high risk heavy smokers experiencing a cardiovascular event (HRHSCV), wherein reduced level of serum soluble MAdCAM-1 is a marker predicting the onset of NSCLC.

11. A combined therapy comprising (i) an anti-PD1 or an anti-PDL1 blocker antibody and (ii) an anti-IL17A or anti-IL17R blocker antibody and/or a recombinant IL-7, for use in the treatment of cancer in a patient having a low serum level of soluble MAdCAM.

## Patentansprüche

1. Verwendung des Spiegels von im Serum löslichem MAdCAM-1 als Marker für Resistenz oder Empfindlichkeit gegenüber einer immunonkologischen (I-O) Therapie, wobei ein reduzierter Spiegel des im Serum löslichen MAdCAM-1 ein Marker für Resistenz gegenüber der I-O-Therapie ist.

2. Verwendung des Spiegels von im Serum löslichem MAdCAM-1 als Marker für krebsassoziierte Dysbiose oder antibiotikaassoziierte Dysbiose, wobei ein reduzierter Spiegel des im Serum löslichen MAdCAM-1 ein Marker für krebs- oder antibiotikaassoziierte Dysbiose ist.

3. Verfahren zur In-vitro-Diagnose von krebsassoziierter Dysbiose oder antibiotikaassoziierter Darmdysbiose bei einer Person mit Krebs, das die Messung von im Serum löslichem MAdCAM-1 umfasst, wobei ein reduzierter Spiegel von im Serum löslichem MAdCAM-1 darauf hinweist, dass die Person an einer krebsassoziierten Dysbiose oder antibiotikaassoziierten Darmdysbiose leidet.

4. Verwendung nach Anspruch 1 oder Anspruch 2 oder Verfahren nach Anspruch 3, wobei der Spiegel von im Serum löslichem MAdCAM-1 als reduziert angesehen wird, wenn er unter dem Median der Konzentrationen von löslichem MAdCAM-1 in einer repräsentativen Kohorte liegt.

5. Theranostisches Verfahren zur Bestimmung, ob eine Person mit Krebs eine kompensatorische mikrobiotazentrierte Intervention (MCI) vor der Verabreichung einer immunonkologischen (I-O) Therapie benötigt, umfassend die Beurteilung unter Verwendung des Verfahrens nach einem der Ansprüche 3 und 4, ob die Person eine krebs- oder antibiotikaassoziierte Dysbiose hat, wobei, falls die Person eine krebs- oder antibiotikaassoziierte Dysbiose hat, er/sie eine MCI vor der Verabreichung der Behandlung mit einer I-O-Therapie benötigt.

6. Theranostisches Verfahren nach Anspruch 5, wobei die Behandlung mit einer I-O-Therapie aus der Gruppe ausgewählt wird, bestehend aus Anti-PD1-Antikörpern (Ab), Anti-PDL-1-Ab, Anti-CTLA4-Ab, Anti-Lag3-Ab, Anti-Tim3-Ab, Anti-TIGIT-Ab, Anti-OX40-Ab, Anti-41BB-Ab, Anti-VISTA-Ab, bispezifischen Antikörpern, die PD1 und Lag3 anvisieren, CAR-T-Zellen, adoptiven TIL-Transfer und jede Kombination davon, allein oder in Kombination mit einem anderen antineoplastischen Wirkstoff, insbesondere jeder Kombination eines Immun-Checkpoint-Inhibitors (ICI) mit Taxan, Cisplatin und/oder Oxaliplatin.

7. Theranostisches Verfahren nach einem der Ansprüche 5 und 6, wobei die MCI aus der Gruppe ausgewählt wird, die aus Folgendem besteht:
- oralen Vancomycin-Antibiotika,
- Phagen, die Bakterien der Klade *Enterocloster* gen. nov. abtöten,
- selten schneidenden Endonukleasen wie Crispr Cas9, die entwickelt wurden, um Bakterien der Klade *Enterocloster* gen. nov. abzutöten,
- *Akkermansia* spp und/oder *Akkermansia muciniphila,* möglicherweise gemischt mit anderen nützlichen Bakterien,
- Retinsäure
- mikrobieller Stuhltransplantation, und
- Mischungen davon.

8. Wirkstoff für MCI zur Verwendung bei der Behandlung von Krebs in Kombination mit einem ICI bei einer Person mit einem reduzierten Spiegel an im Serum löslichem MAdCAM-1, wobei der Wirkstoff aus der Gruppe ausgewählt wird, die aus Folgendem besteht:
- oralen Vancomycin-Antibiotika,
- Phagen, die Bakterien der Klade *Enterocloster* gen. nov. abtöten,
- selten schneidenden Endonukleasen wie Crispr Cas9, die entwickelt wurden, um Bakterien der Klade *Enterocloster* gen. nov. abzutöten,
- *Akkermansia* spp und/oder *Akkermansia muciniphila,* möglicherweise gemischt mit anderen nützlichen Bakterien,
- mikrobiellen Stuhlzusammensetzungen, und
- Mischungen davon.

9. Verfahren zur Nachverfolgung des Erfolgs einer MCI bei einer Person, bei der zuvor eine krebs- oder antibiotikaassoziierte Dysbiose diagnostiziert wurde, umfassend die Messung von im Serum löslichem MAdCAM-1, wobei ein normalisierter Spiegel von im Serum löslichem MAdCAM-1 darauf hinweist, dass die MCI die Darmeubiose erfolgreich wiederhergestellt hat.

10. Verwendung des Spiegels von im Serum löslichem MAdCAM-1 als Biomarker zur Vorhersage einer frühen Inzidenz von NSCLC bei stark rauchenden Hochrisikopatienten, die ein kardiovaskuläres Ereignis (HRHSCV) erlitten haben, wobei ein reduzierter Spiegel des im Serum löslichen MAdCAM-1 ein Marker ist, der das Auftreten von NSCLC vorhersagt.

11. Kombinierte Therapie, umfassend (i) einen Anti-PD1- oder einen Anti-PDL1-Blocker-Antikörper und (ii) einen Anti-IL17A- oder Anti-IL17R-Blocker-Antikörper und/oder ein rekombinantes IL-7 zur Verwendung bei der Behandlung von Krebs bei Patienten mit niedrigem Serumspiegel an löslichem MAdCAM.

## Revendications

1. Utilisation du taux de MAdCAM-1 soluble sérique comme marqueur de résistance ou de sensibilité à une thérapie d'immuno-oncologie (I-O), dans laquelle un taux réduit de MAdCAM-1 soluble sérique est un marqueur de résistance à la thérapie I-O.

2. Utilisation du taux de MAdCAM-1 soluble sérique comme marqueur de dysbiose associée au cancer ou de dysbiose associée aux antibiotiques, dans laquelle un taux réduit de MAdCAM-1 soluble sérique est un marqueur de dysbiose associée au cancer ou aux antibiotiques.

3. Procédé de diagnostic *in vitro* de dysbiose associée au cancer ou de dysbiose intestinale associée aux antibiotiques chez un individu atteint d'un cancer, comprenant la mesure de MAdCAM-1 soluble sérique, dans lequel un taux réduit de MAdCAM-1 soluble sérique indique que l'individu présente une dysbiose associée au cancer ou une dysbiose intestinale associée aux antibiotiques.

4. Utilisation selon la revendication 1 ou la revendication 2, ou procédé selon la revendication 3, dans laquelle ou lequel le taux de MAdCAM-1 soluble sérique est considéré comme réduit s'il est inférieur à la médiane des taux de MAdCAM-1 soluble dans une cohorte représentative.

5. Procédé théragnostique pour déterminer si un individu atteint d'un cancer nécessite une intervention centrée sur le microbiote (MCI) compensatoire avant administration d'une thérapie d'immuno-oncologie (I-O), comprenant le fait d'apprécier, en utilisant le procédé selon l'une quelconque des revendications 3 et 4, si l'individu présente une dysbiose associée au cancer ou aux antibiotiques, dans lequel si l'individu présente une dysbiose associée au cancer ou aux antibiotiques, il a besoin d'une MCI avant administration du traitement par thérapie I-O.

6. Procédé théragnostique selon la revendication 5, dans lequel ledit traitement par thérapie I-O est choisi dans le groupe consistant en des anticorps (Ac) anti-PD1, Ac anti-PDL-1, Ac anti-CTLA4, Ac anti-Lag3, Ac anti-Tim3, Ac anti-TIGIT, Ac anti-OX40, Ac anti-41BB, Ac anti-VISTA, anticorps bispécifiques ciblant PD1 et Lag3, cellules CAR-T, transfert adoptif de TIL et toute combinaison de ceux-ci, seuls ou combinés à un autre agent antinéoplasique, notamment toute combinaison d'un inhibiteur du point de contrôle immunitaire (ICI) avec taxane, cis-platine et/ou oxaliplatine.

7. Procédé théragnostique selon l'une quelconque des revendications 5 et 6, dans lequel la MCI est choisie dans le groupe consistant en :
- antibiotiques vancomycine oraux,
- phages tuant des bactéries du clade *Enterocloster* gen. nov.,
- endonucléases à coupures rares telles que Crispr Cas9, conçues pour tuer des bactéries du clade *Enterocloster* gen. nov.,
- *Akkermansia* spp et/ou *Akkermansia muciniphila,* éventuellement mélangées à d'autres bactéries bénéfiques,
- acide rétinoïque
- transplantation microbienne fécale, et
- leurs mélanges.

8. Agent pour MCI, pour utilisation dans le traitement du cancer en association avec un ICI chez un individu présentant un taux réduit de MAdCAM-1 soluble sérique, dans lequel ledit agent est choisi dans le groupe consistant en :
- antibiotiques vancomycine oraux,
- phages tuant des bactéries du clade *Enterocloster* gen. nov.,
- endonucléases à coupures rares telles que Crispr Cas9, conçues pour tuer des bactéries du clade *Enterocloster* gen*.* nov.,
- *Akkermansia* spp et/ou *Akkermansia muciniphila,* éventuellement mélangées à d'autres bactéries bénéfiques,
- compositions microbiennes fécales, et
- leurs mélanges.

9. Procédé de suivi du succès d'une MCI chez un individu chez qui a été précédemment diagnostiquée une dysbiose associée au cancer ou aux antibiotiques, comprenant la mesure de MAdCAM-1 soluble sérique, dans lequel un taux normalisé de MAdCAM-1 soluble sérique indique que la MCI a restauré avec succès l'eubiose intestinale.

10. Utilisation du taux de MAdCAM-1 soluble sérique comme biomarqueur prédisant une incidence précoce d'un CPNPC chez de gros fumeurs à haut risque ayant connu un événement cardiovasculaire (HRHSCV), dans laquelle un taux réduit de MAdCAM-1 soluble sérique est un marqueur prédisant l'apparition du CPNPC.

11. Thérapie combinée comprenant (i) un anticorps bloquant anti-PD1 ou anti-PDL1 et (ii) un anticorps bloquant anti-IL17A ou anti-IL17R et/ou une IL-7 recombinante, pour utilisation dans le traitement du cancer chez un patient présentant un faible taux sérique de MAdCAM soluble.
